# EUROPEAN PATENT APPLICATION

(11) **EP 2 070 928 A1**
(43) Date of publication of application: **17.06.2009**
(21) Application number: 08171301.8
(22) Date of filing: 11.12.2008
(51) Int. Cl.: C07D 471/04, A61K 31/437, A61P 35/00

(54) **7-azaindol-3-ylacrylamides active as kinase inhibitors**

(30) Priority: 12.12.2007 EP 07123050
(71) Applicant: NERVIANO MEDICAL SCIENCES S.r.l., 20014 Nerviano (MI) (IT)
(72) Inventor: Casuscelli, Francesco, 20020 Dairago(MI) (IT); Mongelli, Nicola, 20137 Milan (IT); Amici, Raffaella, 26845 Codogno (Lodi) (IT); Traquandi, Gabriella, 20151 Milan (IT); Menichincheri, Maria, 20124 Milan (IT); Ermoli, Antonella, 20090 Buccinasco (MI) (IT)

(57) **Abstract**

Compounds represented by Formula (I) 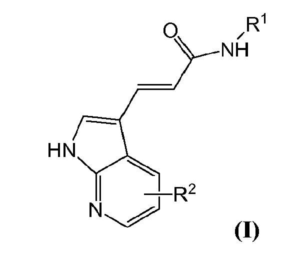 wherein **R¹** and **R²** are as defined in the specification, compositions thereof, and methods of use thereof.

## Description

### FIELD OF THE INVENTION

The invention relates to 7-azaindol-3-yl-acrylamides derivatives, to pharmaceutical compositions comprising them and to their use as therapeutic agents, particularly in the treatment of cancer and cell proliferation disorders.

### BACKGROUND OF THE INVENTION

Protein kinases (PKs) represent a large family of proteins which play a central role in the regulation of a wide variety of cellular processes, maintaining control over cellular function.

The malfunctioning of protein kinases is the hallmark of numerous diseases. A large share of the oncogenes and protooncogenes involved in human cancers code for PKs. The enhanced activities of PKs are also implicated in many non-malignant diseases. For a general reference to PKs malfunctioning or disregulated see, for instance, Current Opinion in Chemical Biology 1999, 3, 459-465.

Inhibition of such kinases has become an important therapeutic target. In particular the present invention relates to azaindolacrylamide compounds useful for treating diseases mediated by AKT and CDC7.

AKT (also known as protein kinase B (PKB) or Rac-PK-beta), and its gene family products, has been identified as a serine/threonine protein kinase (Proc. Natl. Acad. Sci. 2001, 98, 10983-10985; J. Cell. Sci. 2001, 114, 2903-2910; Circ. Res. 2000, 86, 15-23). Three isoforms of PKB are currently known, PKBα (AKT1 PKBβ,(AKT2), and PKBγ (AKT3) (Proc. Natl. Acad. Sci. 1992, 89, 9267-9271; J. Biol. Chem. 1999, 274, 9133-9136). PKB mediates many effects of IGF-1 and other growth factors on tumor growth and inhibition of apoptosis (Cell. Signal. 2002, 14, 381-395). PKB plays an important role in cell proliferation, apoptosis and response to insulin. For these reasons, modulation of PKBs is of interest in the treatment of tumorigenesis, abnormal cell proliferation and diabetes. The molecular structure of the PKBs comprises a regulatory site near the carboxy terminus of the polypeptide, a catalytic domain with an activation loop having a threonine, and an amino-terminal pleckstrin homology domain. The pleckstrin homology domain permits anchorage of the enzyme to the cell membrane through interaction with phospholipids, which triggers the activation of the PKBs. The role of pleckstrin homology domain requires phosphorylation of phosphatidylinositol at the D-3 position via phosphatidylinositol 3-kinase PI3K, an SH2 domain protein that associates with activated receptor tyrosyne kinases, particularly IGF-1R. In particular, phosphoinositol-3-kinase, when activated by receptor tyrosine kinase, catalyzes the synthesis of phosphoinositol-3,4-diphosphate and phosphatidylinositol-3,4,5-triphosphate. The pleckstrin homology domain binds 3-phosphoinositides, which are synthesized by PI3K upon stimulation by growth factors such as platelet derived growth factor (PDGF), nerve growth factor (NGF) and insulin-like growth factor (IGF-1) (Mol. Cell. Biol. 1997, 17, 1595-1606; Science 1997, 275, 628-630; Genev. Dev. 1999, 13, 2905-2927). Lipid binding to the pleckstrin homology domain promotes translocation of PKB to the plasma membrane. Further activation of PKB occurs by phosphorilation by another protein kinase, PDK1 at Thr309, and Thr305 for the PKB isoforms 1, 2 and 3, respectively. A third step of activation is catalyzed by a kinase that phosphorylates Ser473, Ser474 or Ser472 in the C-terminal tails of PKB/AKT-1, -2 and -3 respectively. The Ser473 kinase activity has been identified to be associated with plasma membrane and is not due to PKB and PDK1 kinase activity (Current Biology 2002, 12, 1251-1255; J. Biol. Chem. 2003, 278, 21615-21622). The process produces the fully activated form of PKB.

Activation of PKB can also occur by inhibiting the D-3 phosphoinositide specific phosphatase, PTEN, which is a membrane associated FYVE finger phosphatase commonly inactivated in many cancers, including prostate cancer (Eur. J. Biochem. 1999, 263, 605-611; Cancer Res. 1997, 57, 2124-2129).

The catalytic domain of PKB is responsible for the phosphorylation of serine or threonine in the target protein.

Once activated, PKB mediates several cellular functions, including proliferation, cell growth, and promotion of survival.

The antiapoptotic function of PKB is reported to be mediated by its ability to phosphorylate apoptosis regulatory molecules including BAD, caspase 9, IKK-, and the forkhead transcriptional factor FKHRL1. PKB signal is also implicated in the physiological regulation of organ size (Nat. Cell. Biol. 1999, 1, 500-506), glucose homeostasis (J. Biol. Chem. 1999, 274, 1865-1868), vasomotor tone (J. Clin. Invest. 1999, 106, 493-499), and angiogenesis (Nat. Med. 2000, 6, 1004-1010).

Manifestations of altered PKB regulation appear in both injury and disease, the most important role being in cancer. PKB kinase activity is constitutively activated in tumors with PTEN mutation, PI3-kinase mutation and overexpression, and receptor tyrosin kinase overexpression. PKB is also a mediator of normal cell functions in response to growth factor signalling. Expression of the AKT gene was found to be amplified in 15 % of human ovarian carcinoma cases (Proc. Natl. Acad. Sci. 1992 , 89, 9267-9271). AKT is also overexpressed in 12 % of pancreatic cancers (Proc. Natl. Acad. Sci 1996, 93, 3636-3641). In particular, AKT-2 is overexpressed in 12 % of ovarian carcinomas and in 50 % of indifferentiated tumors, suggesting that PKB may be associated with tumor aggressiveness (Int. J. Cancer 1995, 64, 280-285). PKB is also a mediator of normal cell functions (Nature 1999, 401, 33-34; Oncogene 2000, 19, 2324-2330; Neurosci. 2000, 20,2875-2886).

Elucidation of the role of PKB in the increase of growth and inhibition of apoptosis is complicated by the many protein substrates of PKB, including BAD, Forkhead (FOXO family), GSK3, Tuberin (TSC2), p27 Kip1, p70s6k, protein kinase C-, forkhead in rhabdomyosarcoma, Raf, cAMP-response element-binding protein, glycogen synthase kinase-3, m-TOR, and the androgen receptor (Proc. Natl. Acad. 2001, 98, 7200-7205; Nature 2001, 411, 355-365; Nat. Rev. Cancer 2002, 2, 489-501).

The various PKBs vary in their abundance in different mammalian cell types. For example, PKBβ are especially abundant in highly insulin-responsive tissues, including brown fat.

Modulation of PKB by small molecules can be achieved by identifying compounds that bind to and activate or inhibiting one or more PKBs.

The hereinbelow described and claimed azaindolacrylamide compounds are also useful for treating diseases mediated by Cdc7.

DNA replication is a fundamental process for cell proliferation. Mechanisms that control entry in the S-phase and proper execution of DNA synthesis are often altered in malignant cells and at the same time are attractive targets for the development of antitumoral agents. DNA replication is a two-step process: first, during the initiation reaction, proteins bound to origin DNA are activated by phosphorylation in order to assemble replication forks and, second, during elongation DNA polymerases, together with accessory factors, synthesize new DNA strands. Cdc7 kinase, a key cell cycle regulator, is an evolutionary conserved serine-threonine kinase, which plays a pivotal role in linking cell cycle regulation to genome duplication. In S-phase Cdc7 allows the firing of DNA replication origins by phosphorylating proteins that are recruited at origin sequences during the previous G1. Cdc7-dependent phosphorylation is therefore essential for converting a dormant pre-replicative complex into two active replication forks (Annu. Rev. Biochem. 2002, 71, 333-374). The Cdc7 kinase, like cyclin-dependent kinases, is activated by the binding of alternative regulatory subunits, Dbf4 and Drf1 (Mol. Cell Biol. 1999, 19, 5083-5095; EMBO J. 2002, 21, 3171-3181). Cdc7, Dbf4 and Drf1 mRNA are overexpressed in a number of tumor cell lines (Gene 1998, 211, 133-140; (Mol. Cell Biol. 1999, 19, 5083-5095; EMBO J. 2002, 21, 3171-3181).

Cdc7 kinase is a potential good target for the development of anticancer drugs. In fact Cdc7 function is essential for DNA replication and cell proliferation of human cells: microinjection of specific anti-Cdc7 antibodies prevents DNA replication (EMBO J. 1999, 18, 5703-5713) and genetic downregulation of Cdc7 kinase by siRNA block DNA synthesis leading to a p53 independent cell death in several cancer cell lines (Cancer Res. 2004, 64, 7110-7116). Furthermore Cdc7 inhibition abolishes the phosphorylation of relevant cellular substrate (Mcm2 protein) at specific phosphorylation sites (J. Biol. Chem 2006, 281, 10281-10290).

### SUMMARY OF THE INVENTION

It is an object of the invention to provide compounds which are useful, in therapy, as agents against a host of diseases caused by and/or associated to a disregulated protein kinase activity and, more particularly, AKT known also as protein kinase B (PKB) and Cdc7 activity.

Acrylamide derivatives as antiallergic agents, their synthesis and structure-activity relationships are described by Nishikawa, Yoshinori et al. in Chem. Pharm. Bull.(1989), 37(3),684-7.

The present inventors have now discovered that certain azaindolacrylamides are endowed with protein kinase inhibitory activity and are thus useful in therapy as antitumor agents and lack, in terms of both toxicity and side effects, the aforementioned drawbacks associated with currently available antitumor drugs.

More specifically, the azaindolacrylamides of the invention are useful in the treatment of a variety of disorders and cancers including, but not limited to: carcinoma such as bladder, breast, colon, kidney, liver, lung, including small cell lung cancer, esophagus, gall-bladder, ovary, pancreas, stomach, cervix, thyroid, prostate, and skin, including squamous cell carcinoma; hematopoietic tumors of myeloid or lymphoid lineage including leukaemia, acute lymphocitic leukaemia, acute lymphoblastic leukaemia, B-cell lymphoma, T-cell-lymphoma, Hodgkin's lymphoma, non-Hodgkin's lymphoma, hairy cell lymphoma and Burkett's lymphoma; hematopoietic tumors of myeloid lineage, including acute and chronic myelogenous leukemias, myelodysplastic syndrome and promyelocytic leukaemia; tumors of mesenchymal origin, including fibrosarcoma and rhabdomyosarcoma; tumors of the central and peripheral nervous system, including astrocytoma neuroblastoma, glioma and schwannomas; other tumors, including melanoma, seminoma, teratocarcinoma, osteosarcoma, xeroderma pigmentosum, keratoxanthoma, thyroid follicular cancer and Kaposi's sarcoma. The azaindolacrylamides of the invention also antagonize or inhibit activity toward AKT or Cdc7.

Due to the key role of AKT and Cdc7 in the regulation of cellular proliferation, these azaindolacrylamide derivatives are also useful in the treatment of a variety of cell proliferative disorders such as, for example, benign prostate hyperplasia, familial adenomatosis, polyposis, neurofibromatosis, psoriasis, vascular smooth cell proliferation associated with atherosclerosis, pulmonary fibrosis, arthritis, glomerulonephritis and post-surgical stenosis and restenosis.

The compounds of this invention, as modulators of apoptosis, may also be useful in the treatment of cancer, viral infections, prevention of AIDS development in HIV-infected individuals, autoimmune diseases and neurodegenerative disorders.

The present invention also provides a method for treating diseases caused by and/or associated with dysregulated protein kinase activity, particularly one or more isoforms of the serin/threonine kinase AKT, PLK family, protein kinase C in different isoforms, Met, PAK-4, PAK-5, ZC-1, STLK-2, DDR-2, Aurora 1, Aurora 2, Bub-1, Chk1, Chk2, HER2, raf1, MEK1, MAPK, EGF-R, PDGF-R, FGF-R, IGF-R, ALK , PI3K, weel kinase, Src, Abl, MAPK, ILK, MK-2, IKK-2, Nek, Cdk/cyclin kinase family, CK2, GSK3, SULU, PDK, RET, KIT, LCK, TRKA, more particulary one or more isoforms of the serin/threonine kinase AKT, which comprises administering to a mammal in need thereof an effective amount of a substituted 7-azaindol-3-yl-acrylamides derivatives and thus be effective in the treatment of diseases associated with these protein kinases.

Accordingly, in a first embodiment, the present invention provides a 7-azaindol-3-yl-acrylamide derivative represented by formula (I) 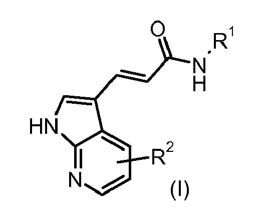 wherein
R¹ is a hydrogen or a straight or branched C₁-C₆ alkyl, C₃-C₆ cycloalkyl, heterocyclyl; aryl, arylalkyl, heteroarylalkyl, heterocyclylalkyl; or R¹ is the group of formula (II) 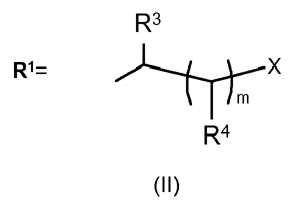 wherein each of said (C₁-C₆)alkyl, (C₃-C₆)cycloalkyl, heterocyclyl; aryl, arylalkyl, heteroarylalkyl, heterocyclylalkyl moieties can be unsubstituted or substituted by one or more substituents, each substituent being independently selected from the group consisting of alkyl, aryl, -OCF₃, -OC(O)alkyl, -OC(O)aryl, -CF₃, heteroaryl, aralkyl, alkylaryl, heteroaralkyl, alkylheteroaryl, hydroxy, hydroxyalkyl, alkoxy, aryloxy, aralkoxy, acyl, aryl, halo, haloalkyl, haloalkoxy, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, heterocyclyl, amino; - NH(alkyl),
-NH(cycloalkyl), and -N(alkyl)₂;
m = 0-1-2;
R³ and R⁴ are selected from the group consisting of hydrogen, (C₁-C₆)alkyl, (C₂-C₆)alkenyl, (C₂-C₆)alkynyl, (C₃-C₆)cycloalkyl, phenyl, arylalkyl, and heteroarylalkyl; wherein each of said (C₁-C₆)alkyl, (C₂-C₆)alkenyl, (C₂-C₆)alkynyl, (C₃-C₆)cycloalkyl, phenyl,arylalkyl and heteroarylalkyl moieties can be unsubstituted or substituted by one or more substituents, each substituent being independently selected from the group consisting of alkyl, aryl, -OCF₃, - OC(O)alkyl, -OC(O)aryl, -CF₃, heteroaryl, aralkyl, alkylaryl, heteroaralkyl, alkylheteroaryl, hydroxy, hydroxyalkyl, alkoxy, aryloxy, aralkoxy, acyl, aryl, halo, haloalkyl, haloalkoxy, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, heterocyclyl, amino; -NH(alkyl), -NH(cycloalkyl), and - N(alkyl)₂;
X can be hydrogen, hydroxyl, alkoxy, carbamoyl, cyano, azido or a group of formula (III) 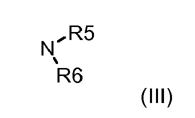 wherein R⁵ and R⁶ are, each independently, hydrogen or an optionally substituted group selected from straight or branched C₁-C₆ alkyl, C₃-C₆ cycloalkyl, and R⁵ and R⁶, taken together with the nitrogen atom to which they are bonded, may form an optionally substituted heterocyclyl group, optionally containing an additional heteroatom selected from N, O and S;
R² is selected from the group consisting of hydrogen, nitro, amino, arylamino, arylalkylamino, heteroarylamino, (C₃-C₈)alkylcarbonylamino, (C₄-C₇)cycloalkylcarbonylamino, arylcarbonylamino, heteroarylcarbonylamino, heterocyclylcarbonylamino, arylalkylcarbonylamino, and halogen; wherein each of said arylamino, heteroarylamino, (C₃-C₈)alkylcarbonylamino, (C₄-C₇)cycloalkylcarbonylamino, arylcarbonylamino, heteroarylcarbonylamino, heterocyclylcarbonylamino, and arylalkylcarbonylamino moieties can be unsubstituted or substituted by one or more substituents, each substituent being independently selected from the group consisting of alkyl, aryl, -OCF₃, -OC(O)alkyl, -OC(O)aryl, -CF₃, heteroaryl, aralkyl, alkylaryl, heteroaralkyl, alkylheteroaryl, hydroxy, hydroxyalkyl, alkoxy, aryloxy, aralkoxy, acyl, aryl, halo, haloalkyl, haloalkoxy, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, heterocyclyl, amino; -NH(alkyl), -NH (cycloalkyl), and -N(alkyl)₂, or a pharmaceutically acceptable salt thereof, with the proviso that the compound (E)-2-propenamide, N-[4-[4-(diphenylmethyl)-1-piperazinyl]butyl]-3-(1H-pyrrolo[2,3-b]pyridin-3-yl) is excluded.

A method of treating cell proliferative disorders caused by and/or associated with an altered AKT (PKB) or Cdc7 kinase activity by administering to a mammalian subject in need thereof an effective amount of a compound of formula I as defined above is also provided.

A further object of the present invention is a pharmaceutical composition comprising an amount of the compound of formula (I) as defined above, or a pharmaceutically acceptable salt thereof, and a pharmaceutically acceptable carrier.

A further object of the present invention is a compound of formula (I) as defined above for use as medicament.

A further object of the present invention is a compound of formula (I) as defined above for use in a method for treating a cell proliferative disorder or condition in a mammal.

Preferably, the cell proliferative disorder or condition is selected from the group consisting of bladder cancer, breast cancer, colon cancer, kidney cancer, liver cancer, lung cancer, including small cell lung cancer, esophagus cancer, gall-bladder cancer, ovarian cancer, pancreatic cancer, stomach cancer, cervical cancer, thyroid cancer, prostate cancer, and skin cancer, including squamous cell carcinoma, hematopoietic tumors of lymphoid lineage, including leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma, Burkett's lymphoma, hematopoietic tumors of myeloid lineage, including acute and chronic myelogenous leukemias, myelodysplastic syndrome and promyelocytic leukemia; tumors of mesenchymal origin, including fibrosarcoma and rhabdomyosarcoma; tumors of the central and peripheral nervous system, including astrocytoma, neuroblastoma, glioma and schwannomas; other tumors, including melanoma, seminoma, teratocarcinoma, osteosarcoma, xeroderma pigmentosum, keratoxanthoma, thyroid follicular cancer and Kaposi's sarcoma.

### DETAILED DESCRIPTION OF THE INVENTION

The compounds of formula (I) of the invention may have asymmetric carbon atoms and may therefore exist as individual optical isomers, as racemic admixtures or as any other admixture including a majority of one of the two optical isomers, which are all to be intended as comprised within the scope of the present invention.

Likewise, the use as an antitumor agent of all the possible isomers and their admixtures and of both the metabolites and the pharmaceutically acceptable bio-precursors (otherwise referred to as pro-drugs) of the compounds of formula (I) are also within the scope of the present invention.

The aryl group of the present invention can be unsubstituted or substituted on the ring with one or more substituents, each being independently selected from the group consisting of alkyl, aryl, -OCF₃, -OC(O)alkyl, -OC(O)aryl, -CF₃, heteroaryl, aralkyl, alkylaryl, heteroaralkyl, aminoalkyl, amino, alkylamino, dialkylamino, alkylheteroaryl, hydroxy, hydroxyalkyl, alkoxy, aryloxy, aralkoxy, acyl, aryl, halo, haloalkyl, haloalkoxy, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, heterocyclyl, -NH(alkyl), -NH(cycloalkyl), polyfluoroalkyl and -N(alkyl)₂. Non-limiting examples of suitable aryl groups include phenyl and naphthyl. The "aryl" group can also be substituted by linking two adjacent carbons on its aromatic ring via a combination of one or more carbon atoms and one or more oxygen atoms such as, for example, methylenedioxy, ethylenedioxy, and the like. Examples of aryl groups according to the invention are, for instance, phenyl, biphenyl, α- or β-naphthyl, dihydronaphthyl, thienyl, benzothienyl, furyl, benzofuranyl, pyrrolyl, imidazolyl, pyrazolyl, thiazolyl, isothiazolyl, oxazolyl, isoxazolyl, pyridyl, pyrazinyl, pyrimidinyl, pyridazinyl, indolyl, isoindolyl, purinyl, quinolyl, isoquinolyl, dihydroquinolinyl, quinoxalinyl, benzodioxolyl, indanyl, indenyl, triazolyl, and the like.

With the term heteroaryl the application contemplates any aromatic heterocyclic ring which may optionally comprise a condensed 5 or 6 membered heterocycle with from 1 to 3 heteroatoms selected among N, O or S.

Non limiting examples of heteroaryl groups according to the invention may thus include, for instance, pyridyl, pyrazinyl, pyrimidinyl, pyridazinyl, indolyl, imidazolyl, thiazolyl, isothiazolyl, pyrrolyl, phenyl-pyrrolyl, furyl, phenyl-furyl, oxazolyl, isoxazolyl, pyrazolyl, thienyl, benzothienyl, isoindolinyl, benzoimidazolyl, quinolinyl, isoquinolinyl, 1,2,3-triazolyl, 1-phenyl-1,2,3-triazolyl, and the like.

With the term heterocyclyl the application contemplates any saturated or partially saturated heterocyclic ring with from 1 to 3 heteroatoms selected among N, O or S, which may optionally comprise a condensed (C₃-C₆)cycloalkyl, aryl or 5 or 6 membered heterocycle.

Non limiting examples of heterocyclyl groups according to the invention may thus include, for instance, pyrane, pyrrolidine, pyrroline, imidazoline, imidazolidine, pyrazolidine, pyrazoline, thiazoline, thiazolidine, dihydrofuran, tetrahydrofuran, 1,3-dioxolane, piperidine, piperazine, morpholine and the like.

With the term straight or branched C₁-C₃ alkyl or C₁-C₃ alkoxy the application contemplates any of the groups such as methyl, ethyl, n-propyl, isopropyl, methoxy, ethoxy, n-propoxy and isopropoxy.

With the term halogen atom the invention comtemplates a fluorine, chlorine, bromine or iodine atom.

With the term C₃-C₆ cycloalkyl the invention contemplates any group such as cyclopropyl, cyclobutyl, cyclopentyl or cyclohexyl.

Preferred compounds of the present invention are the following:
1. (2E)-3-(1H-Pyrrolo[2,3-b]pyridin-3-yl)-acrylamide;
2. (2E)-N-Methyl-3-(1H-pyrrolo[2,3-b]pyridin-3-yl)-acrylamide;
3. (2E)-N-Benzyl-3-(1H-pyrrolo[2,3-b]pyridin-3-yl)-acrylamide;
4. (2E)-N-[(1S)-1-phenyl-2-pyrrolidin-1-ylethyl]-3-(1H-pyrrolo[2,3-b]pyridin-3-yl)acrylamide;
5. (2E)-N-[(1S)-2-(4-methylpiperazin-1-yl)-1-phenylethyl]-3-(1H-pyrrolo[2,3-b]pyridin-3-yl)acrylamide;
6. (2E)-N-(2-morpholin-4-ylethyl)-3-(1H-pyrrolo[2,3-b]pyridin-3-yl)acrylamide;
7. (2E)-N-(2-methoxyethyl)-3-(1H-pyrrolo[2,3-b]pyridin-3-yl)acrylamide;
8. (2E)-N,N-dimethyl-3-(1H-pyrrolo[2,3-b]pyridin-3-yl)acrylamide;
9. (2E)-N-(2-pyrrolidin-1-ylethyl)-3-(1H-pyrrolo[2,3-b]pyridin-3-yl)acrylamide;
10. (2E)-N-(1-phenylethyl)-3-(1H-pyrrolo[2,3-b]pyridin-3-yl)acrylamide;
11. (2E)-N-[3-(4-methylpiperazin-1-yl)propyl]-3-(1H-pyrrolo[2,3-b]pyridin-3-yl)acrylamide;
12. (2E)-N-[(1S)-2-morpholin-4-yl-1-phenylethyl]-3-(1H-pyrrolo[2,3-b]pyridin-3-yl)acrylamide;
13. (2E)-N-benzyl-3-(1H-pyrrolo[2,3-b]pyridin-3-yl)acrylamide;
14. (2E)-N-(4-fluorobenzyl)-3-(1H-pyrrolo[2,3-b]pyridin-3-yl)acrylamide;
15. (2E)-N-(2-phenylethyl)-3-(1H-pyrrolo[2,3-b]pyridin-3-yl)acrylamide;
16. (2E)-N-(4-methoxyphenyl)-3-(1H-pyrrolo[2,3-b]pyridin-3-yl)acrylamide;
17. (2E)-N-[4-(4-methylpiperazin-1-yl)benzyl]-3-(1H-pyrrolo[2,3-b]pyridin-3-yl)acrylamide;
18. (2E)-N-(2-morpholin-4-ylbenzyl)-3-(1H-pyrrolo[2,3-b]pyridin-3-yl)acrylamide;
19. (2E)-N-[(2R)-2-hydroxy-2-phenylethyl]-3-(1H-pyrrolo[2,3-b]pyridin-3-yl)acrylamide;
20. (2E)-N-[(2S)-2-hydroxy-2-phenylethyl]-3-(1H-pyrrolo[2,3-b]pyridin-3-yl)acrylamide;
21. (2E)-N-(1-benzylpyrrolidin-3-yl)-3-(1H-pyrrolo[2,3-b]pyridin-3-yl)acrylamide;
22. (2E)-N-(diphenylmethyl)-3-(1H-pyrrolo[2,3-b]pyridin-3-yl)acrylamide;
23. (2E)-N-(3-fluorobenzyl)-3-(1H-pyrrolo[2,3-b]pyridin-3-yl)acrylamide;
24. (2E)-N-(3-chlorobenzyl)-3-(1H-pyrrolo[2,3-b]pyridin-3-yl)acrylamide;
25. (2E)-N-(4-chlorobenzyl)-3-(1H-pyrrolo[2,3-b]pyridin-3-yl)acrylamide;
26. (2E)-N-(2-chlorobenzyl)-3-(1H-pyrrolo[2,3-b]pyridin-3-yl)acrylamide;
27. (2E)-N-(3,4-difluorobenzyl)-3-(1H-pyrrolo[2,3-b]pyridin-3-yl)acrylamide;
28. (2E)-N-[(3S)-2-oxoazepan-3-yl]-3-(1H-pyrrolo[2,3-b]pyridin-3-yl)acrylamide;
29. (2E)-N-(3-methoxybenzyl)-3-(1H-pyrrolo[2,3-b]pyridin-3-yl)acrylamide;
30. (2E)-N-[(1R)-1-phenyl-2-pyrrolidin-1-ylethyl]-3-(1H-pyrrolo[2,3-b]pyridin-3-yl)acrylamide;
31. (2E)-N-(2,5-difluorobenzyl)-3-(1H-pyrrolo[2,3-b]pyridin-3-yl)acrylamide;
32. (2E)-N-(1-methylpiperidin-4-yl)-3-(1H-pyrrolo[2,3-b]pyridin-3-yl)acrylamide;
33. (2E)-N-(1-benzylpiperidin-4-yl)-3-(1H-pyrrolo[2,3-b]pyridin-3-yl)acrylamide;
34. (2E)-N-[3-(dimethylamino)-2,2-dimethylpropyl]-3-(1H-pyrrolo[2,3-b]pyridin-3-yl)acrylamide;
35. (2E)-N-(2,6-difluorobenzyl)-3-(1H-pyrrolo[2,3-b]pyridin-3-yl)acrylamide;
36. (2E)-3-(1H-pyrrolo[2,3-b]pyridin-3-yl)-N-[3-(1H-pyrrol-1-yl)benzyl]acrylamide;
37. (2E)-N-[2-(diisopropylamino)ethyl]-3-(1H-pyrrolo[2,3-b]pyridin-3-yl)acrylamide;
38. (2E)-N-[3-(2-oxopyrrolidin-1-yl)propyl]-3-(1H-pyrrolo[2,3-b]pyridin-3-yl)acrylamide;
39. (2E)-N-[(1S)-1-benzyl-2-hydroxyethyl]-3-(1H-pyrrolo[2,3-b]pyridin-3-yl)acrylamide;
40. N-α-[(2E)-3-(1H-pyrrolo[2,3-b]pyridin-3-yl)prop-2-enoyl]-L-phenylalaninamide;
41. (2E)-N-[(1S)-2-cyclohexyl-1-(hydroxymethyl)ethyl]-3-(1H-pyrrolo[2,3-b]pyridin-3-yl)acrylamide;
42. (2E)-N-[(2E)-3-phenylprop-2-en-1-yl]-3-(1H-pyrrolo[2,3-b]pyridin-3-yl)acrylamide;
43. (2E)-N-[(1S)-1-benzyl-2-methoxyethyl]-3-(1H-pyrrolo[2,3-b]pyridin-3-yl)acrylamide;
44. N-α-[(2E)-3-(1H-pyrrolo[2,3-b]pyridin-3-yl)prop-2-enoyl]-L-tryptophanamide;
45. (2E)-N-[(1S)-2-hydroxy-1-(4-hydroxybenzyl)ethyl]-3-(1H-pyrrolo[2,3-b]pyridin-3-yl)acrylamide;
46. (2E)-N-[cyano(phenyl)methyl]-3-(1H-pyrrolo[2,3-b]pyridin-3-yl)acrylamide;
47. (2E)-N-(1-benzyl-2-pyrrolidin-1-ylethyl)-3-(1H-pyrrolo[2,3-b]pyridin-3-yl)acrylamide;
48. (2E)-N-[1-benzyl-2-(dimethylamino)ethyl]-3-(1H-pyrrolo[2,3-b]pyridin-3-yl)acrylamide;
49. (2E)-*N*-[1-benzyl-2-(4-methylpiperazin-1-yl)ethyl]-3-(1H-pyrrolo[2,3-*b*]pyridin-3-yl)acrylamide;
50. (2E)-N-(1-phenyl-3-pyrrolidin-1-ylpropyl)-3-(1H-pyrrolo[2,3-b]pyridin-3-yl)acrylamide;
51. 3-{(1E)-3-[(2S)-2-benzyl-4-methylpiperazin-1-yl]-3-oxoprop-1-en-1-yl}-1H-pyrrolo[2,3-b]pyridine;
52. (2E)-N-[(1S)-3-methyl-1-(pyrrolidin-1-ylmethyl)butyl]-3-(1H-pyrrolo[2,3-b]pyridin-3-yl)acrylamide;
53. (2E)-N-[(1S)-2,2-dimethyl-1-(pyrrolidin-1-ylmethyl)propyl]-3-(1H-pyrrolo[2,3-b]pyridin-3-yl)acrylamide;
54. (2E)-N-[(1S)-2-cyclohexyl-1-(pyrrolidin-1-ylmethyl)ethyl]-3-(1H-pyrrolo[2,3-b]pyridin-3-yl)acrylamide;
55. (2E)-N-[(1S)-2-methyl-1-(pyrrolidin-1-ylmethyl)propyl]-3-(1H-pyrrolo[2,3-b]pyridin-3-yl)acrylamide;
56. (2E)-N-[(1R)-2-phenoxy-1-(pyrrolidin-1-ylmethyl)ethyl]-3-(1H-pyrrolo[2,3-b]pyridin-3-yl)acrylamide
57. (2*E*)-*N*-[(1*S*)-2-amino-1-benzylethyl]-3-(1*H*-pyrrolo[2,3-*b*]pyridin-3-yl)acrylamide;
58. (3S)-4-phenyl-3-{[(2E)-3-(1H-pyrrolo[2,3-b]pyridin-3-yl)prop-2-enoyl]amino}butan-1-aminium chloride;
59. [(1S,2S)-2-{[(2E)-3-(1H-pyrrolo[2,3-b]pyridin-3-yl)prop-2-enoyl]amino}cyclohexyl]methanaminium chloride;
60. (2R)-3-phenyl-2-{[(2E)-3-(1H-pyrrolo[2,3-b]pyridin-3-yl)prop-2-enoyl]amino}propan-1-aminium chloride;
61. (2S)-1-phenyl-3-{[(2E)-3-(1H-pyrrolo[2,3-b]pyridin-3-yl)prop-2-enoyl]amino}propan-2-aminium chloride;
62. (2E)-N-[(1S)-2-amino-1-(3-fluorobenzyl)ethyl]-3-(1H-pyrrolo[2,3-b]pyridin-3-yl)acrylamide dihydrochloride;
63. (2E)-N-{(1S)-2-amino-1-[3-(aminomethyl)benzyl]ethyl}-3-(1H-pyrrolo[2,3-b]pyridin-3-yl)acrylamide trihydrochloride;
64. (2E)-N-[(1S)-2-amino-1-(2-fluorobenzyl)ethyl]-3-(1H-pyrrolo[2,3-b]pyridin-3-yl)acrylamide dihydrochloride;
65. (2E)-N-[(1S)-2-amino-1-(2-chlorobenzyl)ethyl]-3-(1H-pyrrolo[2,3-b]pyridin-3-yl)acrylamide dihydrochloride;
66. (2E)-N-[(1S)-2-amino-1-(4-chlorobenzyl)ethyl]-3-(1H-pyrrolo[2,3-b]pyridin-3-yl)acrylamide dihydrochloride;
67. (2E)-N-[(1S)-2-amino-1-(4-fluorobenzyl)ethyl]-3-(1H-pyrrolo[2,3-b]pyridin-3-yl)acrylamide dihydrochloride;
68. (2E)-N-[(1S)-2-amino-1-(1-naphthylmethyl)ethyl]-3-(1H-pyrrolo[2,3-b]pyridin-3-yl)acrylamide dihydrochloride;
69. (2E)-N-[(1S)-2-amino-1-(1-benzothien-3-ylmethyl)ethyl]-3-(1H-pyrrolo[2,3-b]pyridin-3-yl)acrylamide dihydrochloride;
70. (2E)-N-[(1S)-2-amino-1-(pyridin-3-ylmethyl)ethyl]-3-(1H-pyrrolo[2,3-b]pyridin-3-yl)acrylamide trihydrochloride;
71. (2E)-N-[(1S)-2-amino-1-(2-furylmethyl)ethyl]-3-(1H-pyrrolo[2,3-b]pyridin-3-yl)acrylamide dihydrochloride;
72. (2E)-N-[(2S)-2-amino-2-phenylethyl]-3-(1H-pyrrolo[2,3-b]pyridin-3-yl)acrylamide dihydrochloride;
73. (2E)-N-[(1S)-1-(aminomethyl)-2,2-diphenylethyl]-3-(1H-pyrrolo[2,3-b]pyridin-3-yl)acrylamide dihydrochloride;
74. (2E)-N-[(1S)-1-benzyl-2-pyrrolidin-1-ylethyl]-3-(1H-pyrrolo[2,3-b]pyridin-3-yl)acrylamide;
75. (2E)-N-[(1S)-1-(aminomethyl)-3-phenylpropyl]-3-(1H-pyrrolo[2,3-b]pyridin-3-yl)acrylamide dihydrochloride;
76. (2E)-N-[(1S)-2-amino-1-(2-naphthylmethyl)ethyl]-3-(1H-pyrrolo[2,3-b]pyridin-3-yl)acrylamide dihydrochloride;
77. (2E)-N-[(1S)-2-amino-1-(4-methoxybenzyl)ethyl]-3-(1H-pyrrolo[2,3-b]pyridin-3-yl)acrylamide dihydrochloride;
78. (2E)-N-[(2R)-2-amino-3-(1H-indol-3-yl)propyl]-3-(1H-pyrrolo[2,3-b]pyridin-3-yl)acrylamide;
79. (2E)-N-{(1S)-2-amino-1-[3-(trifluoromethyl)benzyl]ethyl}-3-(1H-pyrrolo[2,3-b]pyridin-3-yl)acrylamide dihydrochloride;
80. (2E)-N-[(1S)-2-amino-1-(3-methylbenzyl)ethyl]-3-(1H-pyrrolo[2,3-b]pyridin-3-yl)acrylamide;
81. (2E)-N-[(1S)-2-amino-1-(biphenyl-4-ylmethyl)ethyl]-3-(1H-pyrrolo[2,3-b]pyridin-3-yl)acrylamide dihydrochloride;
82. (2E)-N-[(1S)-2-amino-1-(3,4-dichlorobenzyl)ethyl]-3-(1H-pyrrolo[2,3-b]pyridin-3-yl)acrylamide;
83. (2E)-N-[(1S)-2-amino-1-(3,4-dichlorobenzyl)ethyl]-3-(1H-pyrrolo[2,3-b]pyridin-3-yl)acrylamide dihydrochloride;
84. (2E)-N-[(1S)-2-amino-1-(pyridin-4-ylmethyl)ethyl]-3-(1H-pyrrolo[2,3-b]pyridin-3-yl)acrylamide dihydrochloride;
85. (2E)-N-[(2S)-2-amino-3-(1H-indol-3-yl)propyl]-3-(1H-pyrrolo[2,3-b]pyridin-3-yl)acrylamide;
86. (2E)-N-[(1S)-2-amino-1-(1H-indol-3-ylmethyl)ethyl]-3-(1H-pyrrolo[2,3-b]pyridin-3-yl)acrylamide;
87. (E)-N-(S)-2-Amino-1-phenyl-ethyl)-3-(1H-pyrrolo[2,3-b]pyridin-3-yl)-acrylamide;
88. (2E)-N-[(1S)-2-amino-1-cyclohexylethyl]-3-(1H-pyrrolo[2,3-b]pyridin-3-yl)acrylamide;
89. (2E)-N-[(1R)-2-amino-1-(phenoxymethyl)ethyl]-3-(1H-pyrrolo[2,3-b]pyridin-3-yl)acrylamide;
90. (2E)-N-[(1S)-1-(aminomethyl)-3-methylbutyl]-3-(1H-pyrrolo[2,3-b]pyridin-3-yl)acrylamide;
91. (2E)-N-[(1S)-1-(aminomethyl)-2,2-dimethylpropyl]-3-(1H-pyrrolo[2,3-b]pyridin-3-yl)acrylamide;
92. (2E)-N-[(1S)-2-amino-1-(cyclohexylmethyl)ethyl]-3-(1H-pyrrolo[2,3-b]pyridin-3-yl)acrylamide;
93. (2E)-N-[(1S)-1-(aminomethyl)-2-methylpropyl]-3-(1H-pyrrolo[2,3-b]pyridin-3-yl)acrylamide;
94. (E)-N-((R)-2-Amino-1-{3-[5-(3,3-dimethyl-piperidin-1-yl)-2-hydroxy-benzoyl]-phenoxymethyl}-ethyl)-3-(1H-pyrrolo[2,3-b]pyridin-3-yl)-acrylamide;
95. (E)-3-(5-Phenylacetylamino-1H-pyrrolo[2,3-b]pyridin-3-yl)-acrylamide;
96. Cyclopropanecarboxylic acid [3-((E)-2-carbamoyl-vinyl)-1H-pyrrolo[2,3-b]pyridin-5-yl]-amide;
97. (2E)-3-(5-acetylamino-1H-pyrrolo[2,3-b]pyridin-3-yl)-acrylamide;
98. N-[3-((2E)-2-Carbamoyl-vinyl)-1H-pyrrolo[2,3-b]pyridin-5-yl]-benzamide;
99. Thiophene-2-carboxylic acid [3-((E)-2-carbamoyl-vinyl)-1H-pyrrolo[2,3-b]pyridin-5-yl]-amide;
100. (2E)-3-(5-Isobutyrylamino-1H-pyrrolo[2,3-b]pyridin-3-yl)-acrylamide;
101. N-[3-((E)-2-Carbamoyl-vinyl)-1H-pyrrolo[2,3-b]pyridin-5-yl]-3-methylbutyramide;
102. Cyclohexanecarboxylic acid [3-((2E)-2-carbamoyl-vinyl)-1H-pyrrolo[2,3-b]pyridin-5-yl]-amide;
103. N-[3-((E)-2-carbamoyl-vinyl)-1H-pyrrolo[2,3-b]pyridin-5-yl]-nicotinamide;
104. 1-Methyl-piperidine-4-carboxilic acid [3-((E)-2-carbamoyl-vinyl)-1H-pyrrolo[2,3-b]pyridin-5-yl]-amide;
105. N-[3-((E)-2-carbamoyl-vinyl)-1H-pyrrolo[2,3-b]pyridin-5-yl]-4-(4-methyl-piperazin-1-yl)-benzamide;
106. N-[3-((E)-2-carbamoyl-vinyl)-1H-pyrrolo[2,3-b]pyridin-5-yl]-4-morpholin-4-ylmethyl-benzamide;
107. N-[3-((E)-2-carbamoyl-vinyl)-1H-pyrrolo[2,3-b]pyridin-5-yl]-3-phenoxy-benzamide;
108. N- [3-((E)-2-Benzylcarbamoyl-vinyl)-1H-pyrrolo[2,3-b]pyridin-5-yl]-benzamide;
109. (E)-N-Benzyl-3-(5-phenylacetylamino-1H-pyrrolo[2,3-b]pyridin-3-yl]-acrylamide;
110. (E)-N-Benzyl-3-(5-isobutyrylamino-1H-pyrrolo[2,3-b]pyridin-3-yl]-acrylamide;
111. (2E)-N-[(1S)-2-amino-1-benzylethyl]-3-(5-nitro-1H-pyrrolo[2,3-b]pyridin-3-yl)acrylamide dihydrochloride;
112. N-{3-[(1E)-3-{[(1S)-2-amino-1-benzylethyl]amino}-3-oxoprop-1-en-1-yl]-1H-pyrrolo[2,3-b]pyridin-5-yl}benzamide dihydrochloride;
113. (E)-3-(5-Phenylamino-1H-pyrrolo[2,3-b]pyridin-3-yl)-acrylamide;

For a reference to any specific compound of formula (I) of the invention, optionally in the form of a pharmaceutically acceptable salt, see the experimental section and claims.

A further embodiment of the present invention is represented by the process for preparing the compounds of formula (I).

Therefore, the compounds of formula (I) wherein R¹ is a group (II) (wherein, R³, R⁴, m and X are as described above), R² is as described above (Ia) and the pharmaceutically acceptable salts thereof may be obtained by a process comprising:
a) reacting the compounds of formula (IV) wherein R² is as described above with a suitable amine derivative of formula (V) wherein R³, R⁴, m and X are as described above, so as to obtain a compound of formula (Ia):

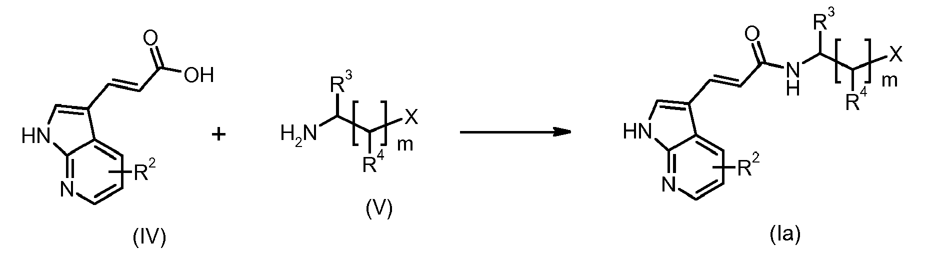

In further embodiments, the compounds of formula (I) wherein X is an amino group, m is = 1 and R² is hydrogen (Ib) may be prepared by a process comprising:
a) reacting the compounds of formula (IV) wherein R² is hydrogen (IVa), with the suitable amino derivatives of formula (V), wherein X is a tert-butoxycarbonyl amino group and m is = 1 (Va), thus obtaining the compounds of formula (VI), wherein R³ and R⁴ are as described above;
b) hydrolyzing in acidic medium the compounds of formula (VI), thus obtaining the compounds of formula (Ib).

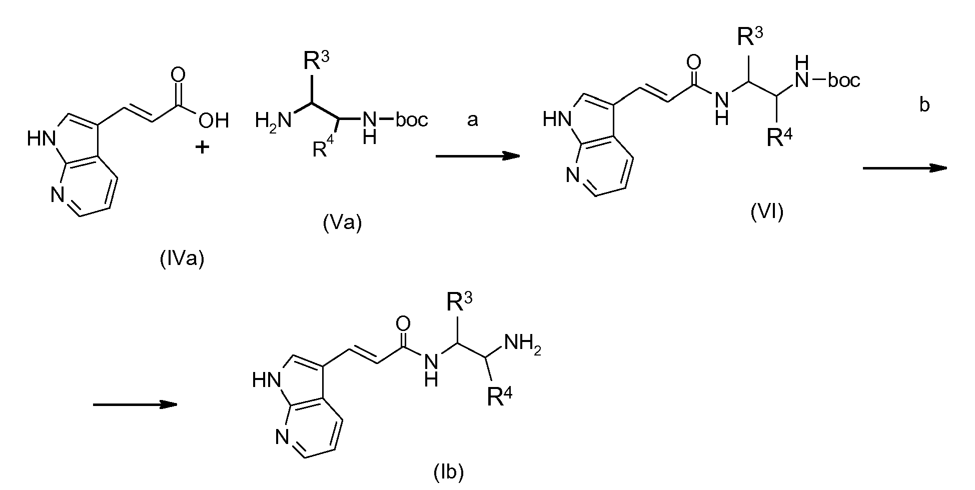

In other examples, the compounds of formula (I) wherein X is an amino group and R² is hydrogen (Ib) may be prepared by a process comprising:
a) reacting the compounds of formula (IVa), wherein R² is hydrogen, with the suitable amino derivatives of formula (V) wherein X is an azido group (Vb), thus obtaining the compounds of formula (VII) wherein R³ and R⁴ are as described above;
b) reducing the compounds of formula (VII), thus obtaining the compounds of formula (Ib):

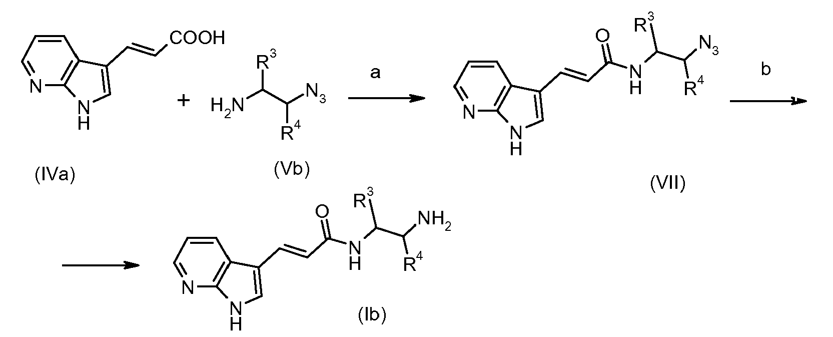

The compounds of formula (Va) and the pharmaceutically acceptable salts thereof may be obtained by a process comprising: 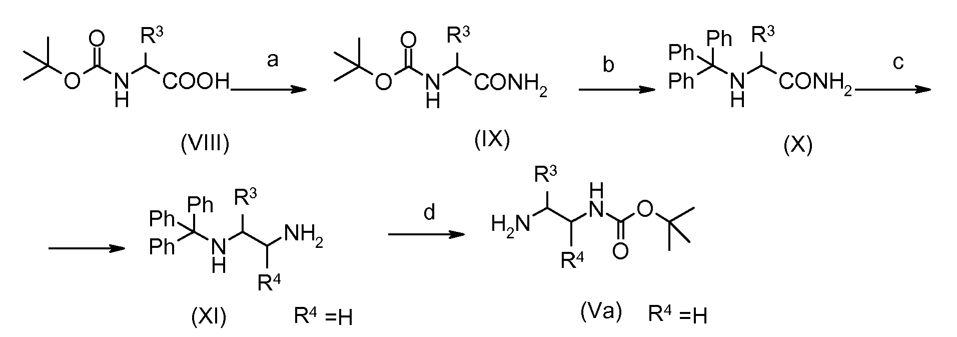
a) reacting the compounds of formula (VIII) wherein R³ is as above reported, with ammonia or a synthetic equivalent of it, thus obtaining the compounds of formula (IX);
b) sequentially removing the tertbutoxycarbonyl group in acidic medium and then introducing the triphenylmethyl protecting group, thus obtaining the compounds of formula (X) wherein R³ is as defined above;
c) reducing the carbamoyl group, thus obtaining the corresponding amino derivatives (XI) wherein R³ is as above reported and R⁴ is hydrogen;
d) sequentially introducing the tertbutoxycarbonyl group and removing the triphenylmethyl group, thus obtaining the compounds of formula (Va) where R³ is as above defined and R⁴ is hydrogen.

In other examples the compounds of formula (V) and the pharmaceutically acceptable salts thereof, wherein R³ and R⁴ are as described above and X is an azido group (Vb) may be prepared by a process comprising: 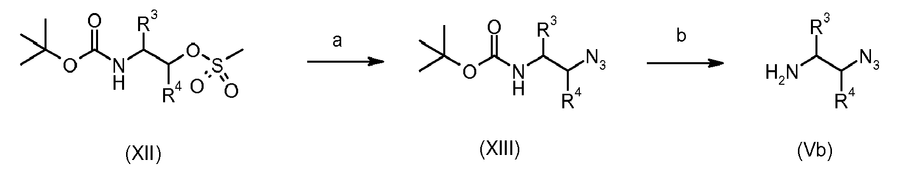
a) reacting the compounds of formula (XII) wherein R³ and R⁴ are as above reported with sodium azide, thus obtaining the compounds of formula (XIII);
b) hydrolyzing the compounds of formula (XIII) wherein R³ and R⁴ are as above reported in acidic medium, thus obtaining the compounds of formula (Vb).

The employed starting materials (XII) may be prepared from the corresponding commercially available aminoalcohols as described in Tetrahedron Asymmetry (1996), 7(7), 2145-2150*.*

The compounds of formula (V) wherein X is a group N(R⁵R⁶) (III) wherein R⁵ and R⁶ are as described above (Vc) can be prepared by a process comprising: 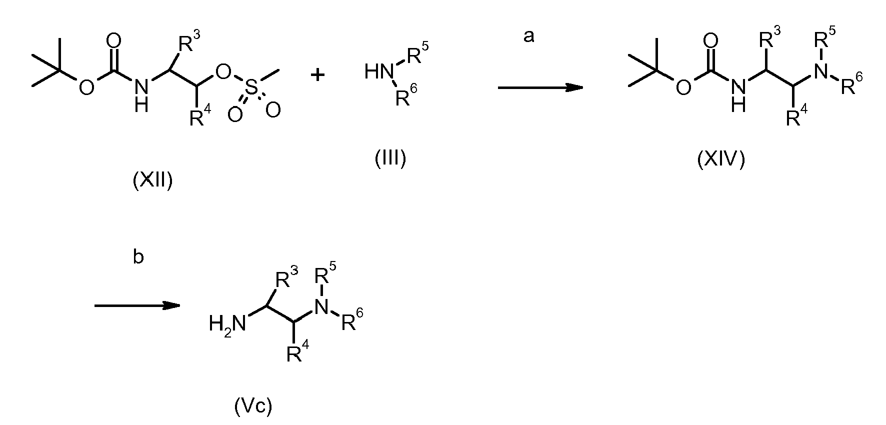
a) reacting the compounds of formula (XII) wherein R³ and R⁴ are as described above with the suitable amino derivatives (III) wherein R⁵ and R⁶ are as described above, thus obtaining the compounds of formula (XIV);
b) hydrolyzing the tert-butoxycarbonyl (BOC) protecting group in acidic medium, thus obtaining the compounds of formula (Vc).

Compounds of formula (Va), (Vb) and (Vc), if not described specifically in the experimental part, have a CAS registry number and their synthesis is described in the literature.

In another example the compound of formula (I) wherein R² is hydrogen, R³ is a [5-(3,3-dimethylpiperidin-1-yl)-2-methoxybenzoyl]-phenoxymethyl group, R⁴ is hydrogen, m = 1 and X is an amino group (Ic), may be prepared by a process comprising: 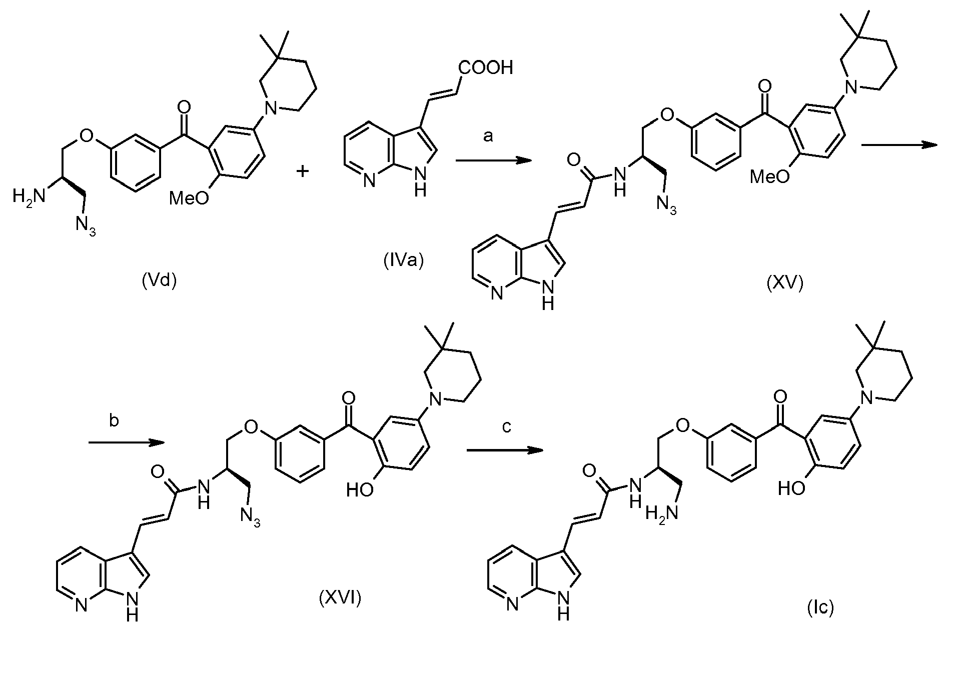
a) reacting the compound of formula (V) wherein R³ is a [5-(3,3-dimethylpiperidin-1-yl)-2-methoxybenzoyl]-phenoxymethyl group, R⁴ is hydrogen, m is = 1 and X is an azido group (Vd) with the compound of formula (IV) wherein R² is hydrogen (IVa), thus obtaining the compound of formula (XV);
b) transforming the methoxy group in the compounds of formula (XV) into the corresponding hydroxyl group, thus obtaining the compound of formula (XVI);
c) reducing the azido group in the compounds of formula (XVI) to the corresponding amino group, thus obtaining the compound of formula (Ic).

Intermediate (Vd) may be prepared as described in the experimental part (example 8).

The compounds of formula (I) wherein R² is alkylcarbonylamino, cycloalkylcarbonylamino, arylcarbonylamino, arylalkylcarbonylamino, heteroarylcarbonylamino, and R¹ is hydrogen (Id), may be prepared by a process comprising: 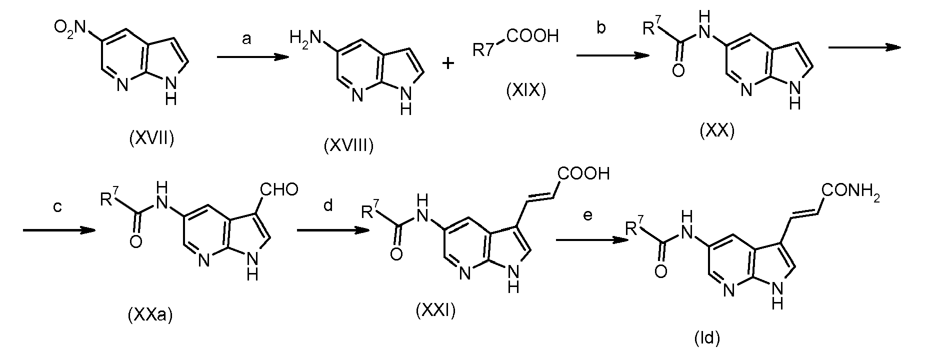
a) reducing the compound of formula (XVII), thus obtaining the compound of formula (XVIII);
b) reacting the compounds of formula (XVIII) with the compounds of formula (XIX) wherein R⁷ is alkyl, cycloalkyl, aryl, arylalkyl, heteroaryl, thus obtaining the compounds of formula (XX);
c) formylating the compounds of formula (XX), wherein R⁷ is alkyl, cycloalkyl, aryl, arylalkyl, heteroaryl, thus obtaining the compounds of formula (XXa) wherein R⁷ is alkyl, cycloalkyl, aryl, arylalkyl, , heteroaryl ;
d) heating the compounds of formula (XXa) with malonic acid, thus obtaining the compounds of formula (XXI) wherein R⁷ is alkyl, cycloalkyl, aryl, arylalkyl, heteroaryl;
e) condensing the compounds of formula (XXI) with ammonia, thus obtaining the compounds of formula (Id).

The compounds of formula (I) wherein R² is alkylcarbonylamino, cycloalkylcarbonylamino, arylcarbonylamino, arylalkylcarbonylamino, heteroarylcarbonylamino and R1 is hydrogen (Id), may be also prepared on solid phase by a process comprising: 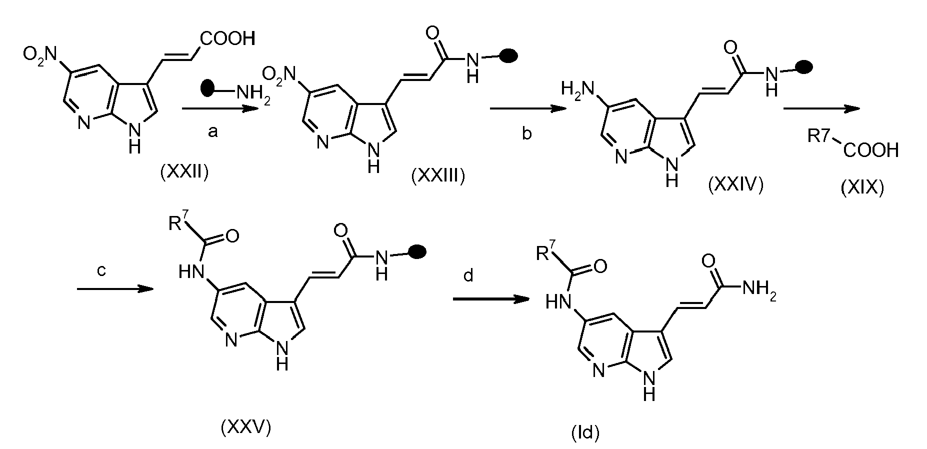
a) reacting the compound of formula (XXII) with a Rink-resin thus obtaining the compound of formula (XXIII);
b) reducing the compound of formula (XXIII), thus obtaining the compound of formula (XXIV);
c) condensing the compound of formula (XXIV) with the compounds of formula (XIX), wherein R⁷ is alkyl, cycloalkyl, aryl, arylalkyl, heteroaryl, thus obtaining the compounds of formula (XXV), wherein R⁷ is alkyl, cycloalkyl, aryl, arylalkyl, heteroaryl;
d) carrying out the cleavage from the resin of the compounds of formula (XXV), thus obtaining the compounds of formula (Id).

The compounds of formula (I) wherein R² is alkylcarbonylamino, arylcarbonylamino, arylalkylcarbonylamino, nitro and R¹ is arylalkyl or a group (II) wherein R³ is arylalkyl, m is 0 and X is an amino group (Ie), may be prepared by a process comprising: 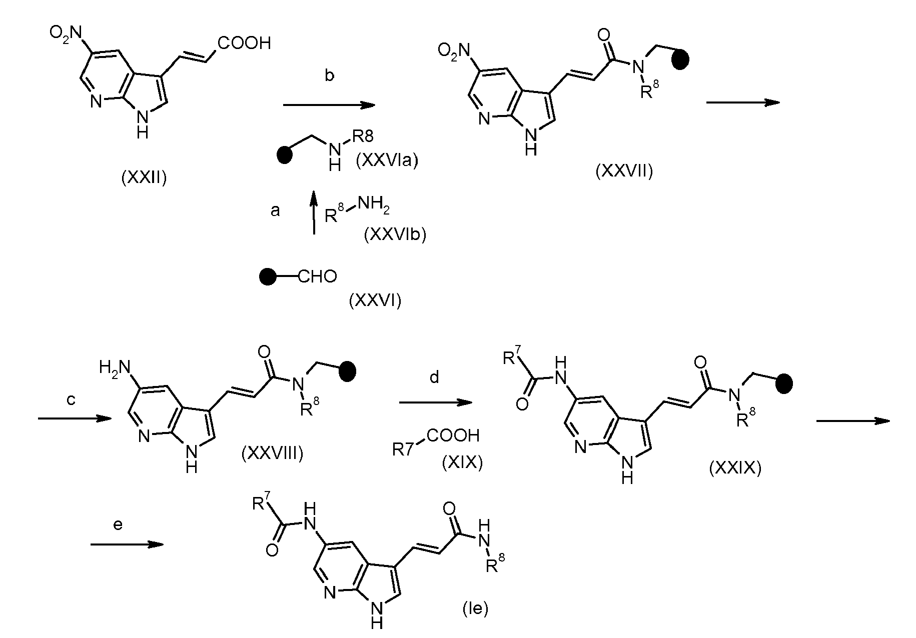
a) reacting the compounds of formula (XXVI) with the compounds of formula (XXVIb), wherein R⁸ is arylalkyl or a group (II) wherein R³ is arylalkyl, m is 0 and X is an amino group by reductive amination, thus obtaining the compounds of formula (XXVIa), wherein R⁸ is arylalkyl or a group (II) wherein R³ is arylalkyl, m is 0 and X is an amino group;
b) reacting the compound of formula (XXII) with the compounds of formula (XXVIa), thus obtaining the compounds of formula (XXVII), wherein R⁸ is arylalkyl or a group (II) wherein R³ is arylalkyl, m is 0 and X is an amino group;
c) reducing the compounds of formula (XXVII), thus obtaining the compounds of formula (XXVIII), wherein R⁸ is arylalkyl or a group (II) wherein R³ is arylalkyl, m is 0 and X is an amino group;
d) condensing the compounds of formula (XXVIII) with the compounds of formula (XIX), wherein R⁷ is alkylcarbonylamino, cycloalkylcarbonylamino, arylcarbonylamino, arylalkylcarbonylamino, heteroarylcarbonylamino, thus obtaining the compounds of formula (XXIX), wherein R⁷ is alkylcarbonylamino, cycloalkylcarbonylamino, arylcarbonylamino, arylalkylcarbonylamino, heteroarylcarbonylamino and R⁸ is is arylalkyl or a group (II) wherein R³ is arylalkyl, m is 0 and X is an amino group;
e) hydrolyzing the link with the resin in the compounds of formula (XXIX), thus obtaining the compounds of formula (Ie).

The compounds of formula (I) wherein R² is arylamino and R¹ is hydrogen (If), may be prepared by a process comprising: 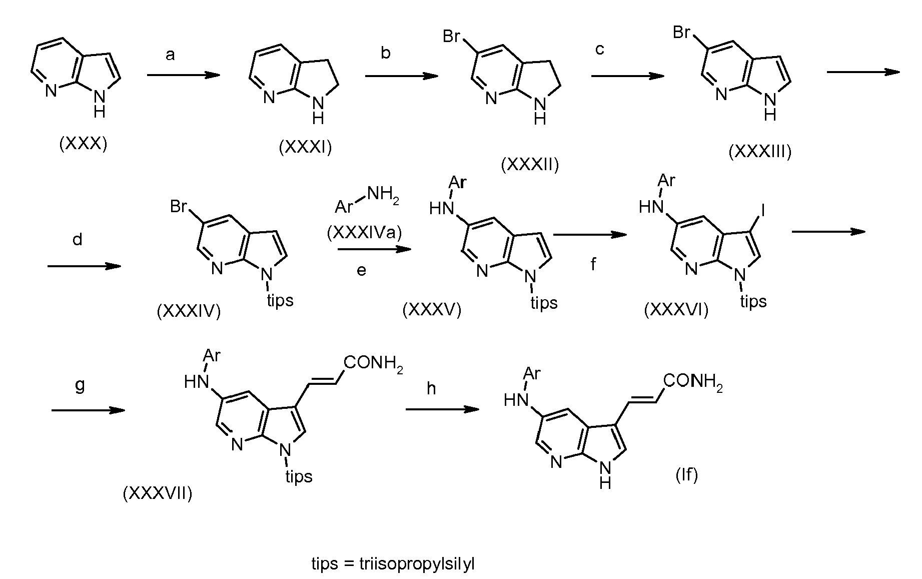
a) reducing the compound of formula (XXX) thus obtaining the compound of formula (XXXI);
b) reacting the compound of formula (XXXI) with bromine, thus obtaining the compound of formula (XXXII);
c) re-oxidizing the compound of formula (XXXII), thus obtaining the compound of formula (XXXIII);
d) protecting the compound of formula (XXXIII) with triisopropylsilyl chloride (TIPS-CI) on the pyrrolo moiety, thus obtaining the compound of formula (XXXIV);
e) reacting the compound of formula (XXXIV) with the suitable arylamine of formula (XXXIVa), thus obtaining the compounds of formula (XXXV);
f) reacting the compounds of formula (XXXV) with iodine, thus obtaining the compounds of formula (XXXVI);
g) condensing the compounds of formula (XXXVI) with acrylamide, thus obtaining the compounds of formula (XXXVII);
h) removing the protective group from the compounds of formula (XXXVII), thus obtaining the compounds of formula (If).

From the above it is clear to the skilled person that these reactions may be accomplished in a variety of ways and operative conditions, which are widely known in the art for the preparation of amides.

As an example, the reaction between the compounds of formula (IV) and (V) to give the compounds of formula (Ia) can be carried out in the presence of a coupling agent such as, for instance, 2-(1H-benzotriazol-1-yl)-1,1,3,3-tetramethyluronium tetrafluoroborate (TBTU), 1,3-dicyclohexylcarbodiimide (DCC), 1,3-diisopropylcarbodiimide (DIC), 1-(3-dimethylaminopropyl)-3-ethylcarbodiimide (EDCI), N-cyclohexylcarbodiimide-N'-propyloxymethyl polystyrene or N-cyclohexylcarbodiimide-N'-methyl polystyrene, 1.1'-carbonyldiimidazole (CDI), benzotriazol-1-yloxy-tripyrrolidinophosphonium hexafluorophosphate (PyBOP), in a suitable solvent such as, for instance, dichloromethane, chloroform, tetrahydrofuran, diethylether, 1,4-dioxane, acetonitrile, toluene, dimethylacetamide (DMA), N-methylpyrrolidone (NMP), or N,N-dimethylformamide (DMF), at a temperature ranging from about -10°C to reflux and for a suitable time, for instance from about 30 minutes to 96 hours. The said reaction is optionally carried out in the presence of a suitable catalyst, for instance 4-dimethylaminopyridine (DMAP), or in the presence of a further coupling reagent such as N-hydroxybenzotriazole (HOBT), or in the presence of a suitable base such as triethylamine (TEA) or N,N-diisopropyl-N-ethylamine (DIPEA).

Alternatively, this same reaction can be also carried out, for example, through a mixed anhydride method, by using an alkyl chloroformate such as ethyl, iso-butyl, or iso-propyl chloroformate, in the presence of a tertiary base such as triethylamine, N,N-diisopropyl-N-ethylamine or pyridine, in a suitable solvent such as, for instance, toluene, dichloromethane, chloroform, tetrahydrofuran, acetonitrile, diethylether, 1,4-dioxane, or N,N-dimethylformamide, at a temperature ranging from about -30°C to room temperature.

Analogously the reaction between the compounds of formula (IVa) with the compounds of formula (Va) to give the compounds of formula (VI) and the reaction of the compounds of formula (IVa) with the compounds of formula (Vb) to give the compounds of formula (VII) can be carried out.

The reaction of the compounds of formula (VI) to give the compounds of formula (Ib) can be carried out in the presence of an acid such as hydrochloric acid or trifluoroacetic acid or formic acid in a suitable solvent such as methanol, ethanol, 1,4-dioxane, dichloromethane at a temperature ranging from room temperature to 50 °C for a time ranging from 2 to 6 hours.

The reaction of the compounds of formula (VII) to give the compounds of formula (Ib) can be carried out in the presence of a suitable reducing agent such as metallic zinc and ammonium chloride or hydrochloric acid in a suitable solvent such as ethanol at a suitable temperature, ranging from room temperature to reflux, from a time ranging from 4 to 12 hours.

The reaction of the compounds of formula (VIII) with ammonia or an ammonia synthetic equivalent such as N-hydroxybenzotriazole ammonium salt to give the compounds of formula (IX) can be carried out with a suitable condensing agent such as ethyl chloroformate or 1-(3-dimethylaminopropyl)-3-ethylcarbodiimide or dicyclohexylcarbodiimide in the presence of a base such as triethylamine or N,N-diisopropyl-N-ethylamine in a suitable solvent such as 1,4-dioxane, tetrahydrofuran, acetonitrile, dichloromethane or dimethylformamide .

The reaction between the compounds of formula (IX) to give the compounds of formula (X) can be carried out in a first step by treating with trifluoroacetic acid in a suitable solvent such as dichloromethane at room temperature and in a second step by treating with triphenylmethyl chloride (TrCI) in the presence of a base such as triethylamine or N,N-diisopropyl-N-ethylamine in a suitable solvent such as toluene, dichloromethane or tetrahydrofuran at room temperature for a time ranging from 4 to 6 hours.

The reaction of the compounds of formula (X) to give the compounds of formula (XI) can be carried out with a reducing agent such as lithium aluminium hydride in a suitable solvent such as diethylether, tetrahydrofuran or 1,4-dioxane at a temperature ranging from room temperature to reflux for a time ranging from 4 to 18 hours.

The reaction of the compounds of formula (XI) to give the compounds of formula (Va) can be carried out in a first step by treating with ditertbutyldicarbonate (BOC anhydride) in the presence of a base such as triethylamine in a suitable solvent such as methanol at room temperature and in a second step by employing formic acid in a suitable solvent such as dichloromethane at a temperature ranging from -10 °C to 0 °C for a time ranging from 30 to 120 minutes.

The reaction of the compounds of formula (XII) to give the compounds of formula (XIII) can be carried out with sodium azide in a suitable solvent such as dimethylformamide at a temperature ranging from room temperature to 100 °C for a time ranging from 2 to 6 hours.

The reaction of the compounds of formula (XIII) to give the compounds of formula (Vb) can be carried out with an acid such as trifluoroacetic acid or hydrochloric acid in a suitable solvent such as dichloromethane or 1,4-dioxane at room temperature for a time ranging from 2 to 6 hours.

The reaction of the compounds of formula (XII) with the compounds of formula (III) to give the compounds of formula (XIV) can be carried out in a suitable solvent such as tetrahydrofuran, 1,4-dioxane, dichloromethane or dimethylformamide at a temperature ranging from room temperature to reflux for a time ranging from 4 to 12 hours.

The reaction of the compounds of formula (XIV) to give the compounds of formula (Vc) can be carried out with an organic acid such as trifluoroacetic acid in a suitable solvent such as dichloromethane or with an inorganic acid such as hydrochloric acid in a suitable solvent such as 1,4-dioxane at room temperature for a time ranging from 2 to 6 hours.

The reaction between the compound of formula (Vd), prepared as described in example 8 of the experimental part, with the compound of formula (IVa) (registry number 123530-68-7), may be carried out with a suitable condensing agent such as EDCI or TBTU or PyBOP, in a suitable solvent such as dichloromethane, tetrahydrofuran, 1,4-dioxane, acetonitrile, N,N-dimethylformamide, at a temperature ranging from 0°C to reflux for a suitable time, for instance from about 1 to 96 hours. The reaction may be optionally carried out in the presence of a base such as triethylamine or N,N-diisopropyl-N'-ethylamine, or in the presence of a further coupling agent such as N-hydroxybenzotriazole.

The reaction of the compound of formula (XV) to give the compound of formula (XVI) can be carried out with a Lewis acid such as boron tribromide in a suitable solvent such as dichloromethane at a temperature ranging from -75°C to 0°C.

The reaction of the compound of formula (XVI) to give the compound of formula (Ic) can be carried out with triphenylphophine in a suitable solvent such as tetrahydrofuran at room temperature.

The reaction of the compound of formula (XVII) (registry number 101083-92-5) to give the compound of formula (XVIII) can be carried out in a hydrogen atmosphere in the presence of a suitable catalyst such as palladium on charcoal in a suitable solvent such as ethylacetate at a pressure of about 40 psi for a time of about 2 hours.

The reaction of the compound of formula (XVIII) with the compounds of formula (XIX), to give the compounds of formula (XX) can be carried out with a suitable condensing agent such as EDCI or TBTU or PyBOP, in a suitable solvent such as dichloromethane, tetrahydrofuran, 1,4-dioxane, acetonitrile, N,N-dimethylformamide, at a temperature ranging from 0°C to reflux for a suitable time for instance from about 1 to 96 hours. The reaction may be optionally carried out in the presence of a base such as triethylamine or DIPEA, or in the presence of a further coupling agent such as N-hydroxybenzotriazole.

The reaction of the compounds of formula (XX) to give the compounds of formula (XXa) can be carried out with a formylating agent such as hexamethylenetetraamine in a suitable solvent such as a mixture of water and acetic acid at a temperature of about 120°C for a time of about 4 hours.

The reaction of the compounds of formula (XXa) to give the compounds of formula (XXI) can be carried out with malonic acid in the presence of piperidine in a suitable solvent such as pyridine at a temperature ranging from 60 to 100°c for a suitable time ranging from 4 to 8 hours.

The reaction of the compounds of formula (XXI) to give the compounds of formula (Id) can be carried out with a suitable coupling agent such as ethylchloroformate or EDCI or TBTU, optionally in the presence of a suitable base such as triethylamine or DIPEA, and ammonia or a synthetic equivalent of ammonia such as N-hydroxybenzotriazole amnmonium salt, in a suitable solvent such as dichloromethane, tetrahydrofuran, acetonitrile, N,N-dimethylformamide or a mixture of them, at room temperature for a time ranging from 4 to 16 hours.

The reaction of the compound of formula (XXII) to give the compound of formula (XXIII) can be carried out with a Rink resin in the presence of a coupling reagent such as TBTU or PyBOP, optionally in the presence of a suitable base such as triethylamine or DIPEA, in a suitable solvent such as N-methylpyrrolidone at room temperature.

The reaction of the compound of formula (XXIII) to give the compound of formula (XXIV) can be carried out with a suitable reducing agent such as tin chloride in a suitable solvent such as N,N-dimethylformamide at a temperature of about 50°C, for a time of about 16 hours.

The reaction of the compound of formula (XXIV) with the compounds of formula (XIX) to give the compounds of formula (XXV) can be carried out in the presence of a coupling agent such as PyBOP, optionally in the presence of N,N-diisopropyl-N-ethylamine (DIPEA) in a suitable solvent such as N,N-dimethylformamide (DMF) at room temperature for a time of about 16 hours. The reaction of the compounds of formula (XXV) to give the compounds of formula (Id) can be carried out with an acid such as trifluoroacetic acid in a suitable solvent such as dichloromethane at room temperature for a time ranging from 1 to 4 hours.

The reaction of the compounds of formula (XXVI) with the compounds of formula (XXVIb) can be carried out with a reducing agent such as triacetoxy sodiumborohydride or sodium cyanoborohydride in a suitable solvent such as acetic acid and dichloromethane at room temperature for a time ranging from 4 to 16 hours.

The reaction of the compounds of formula (XXII) with the compounds of formula (XXVIa) to give the compounds of formula (XXVII) can be carried out with a suitable coupling reagent such as TBTU, optionally in the presence of a suitable base such as triethylamine or N,N-diisopropyl-N-ethylamine, in a suitable solvent such as N-methylpyrrolidone (NMP) at room temperature for a time of about 16 hours.

The reaction of the compounds of formula (XXVII) to give the compounds of formula (XXVIII) can be carried out with a suitable reducing agent such as stanneous chloride hexahydrate in a suitable solvent such as N,N-dimethylformamide at about 50°C for a time of about 16 hours.

The reaction of the compounds of formula (XXVIII) with the compounds of formula (XIX) to give the compounds of formula (XXIX) can be carried out in the presence of a coupling agent such as PyBOP, optionally in the presence of N,N-diisopropyl-N-ethylamine in a suitable solvent such as N,N-dimethylformamide at room temperature for a time of about 16 hours.

The reaction of the compounds of formula (XXIX) to give the compounds of formula (Ie) can be carried out with an acid such as trifluoroacetic acid in a suitable solvent such as dichloromethane at room temperature for a time ranging from 1 to 4 hours.

The reaction of the compounds of formula (XXX) to give the compounds of formula (XXXI) can be carried out with a suitable reducing reagent such as formic acid in the presence of a suitable catalyst such as palladium on chaorcoal and triethylamine at a temperature of about 80°C for a time of about 4 days.

The reaction of the compounds of formula (XXXI) to give the compounds of formula (XXXII) can be carried out with bromine in a suitable solvent such as dichloromethane at a temperature ranging from -10 to 0°C for a time of about 2 hours.

The reaction of the compounds of formula (XXXII) to give the compounds of formula (XXXIII) can be carried out with a suitable oxidizing agent such as manganese bioxide in a suitable solvent such as toluene at a temperature of about 90°C for a time of about 2 hours.

The reaction of the compounds of formula (XXXIII) to give the compounds of formula (XXXIV) can be carried out with sodium hydride and triisopropylsilylchloride (TIPS-CI) in a suitable solvent such as 1,4-dioxane and N,N-dimethylformamide at room temperature for a time of about 16 hours.

The reaction of the compounds of formula (XXXIV) with the compounds of formula (XXXIVa) to give the compounds of formula (XXXV) can be carried out in the presence of a base such as sodium tert-butoxide, dipalladium dibenzylideneacetone (Pd₂(dba)₃) and 1,1'-bis(diphenylphosphino)ferrocene (dppf) in a suitable solvent such as toluene, at a temperature of about 100°C for a time of about 4 hours.

The reaction of the compounds of formula (XXXV) to give the compounds of formula (XXXVI) can be carried out with iodine in the presence of potassium hydroxide at room temperature for a time of about 4 hours.

The reaction of the compounds of formula (XXXVI) to give the compounds of formula (XXXVII) can be carried out with acrylamide in the presence of triethylamine, palladium acetate and tri-o-tolylphosphine in a suitable solvent such as acetonitrile at a temperature of about 80°C for a time of about 4 hours.

The reaction of the compounds of formula (XXXVII) to give the compounds of formula (If) can be carried out with an acid such as hydrochloric acid in a suitable solvent such as 1,4-dioxane at room temperature for a time ranging from 1 to 4 hours.

### PHARMACOLOGY

The compounds of formula (I) are active as protein kinase inhibitors and are therefore useful, for instance, to restrict the unregulated proliferation of tumor cells.

In therapy, they can be used in the treatment of various tumors, such as those formerly reported, as well as in the treatment of other cell proliferative disorders such as psoriasis, vascular smooth cell proliferation associated with atherosclerosis and post-surgical stenosis and restenosis and in the treatment of Alzheimer's disease.

The inhibiting activity of putative Cdc7 inhibitors and the potency of selected compounds is determined through a method of assay based on the use of Dowex resin capture technology.

The assay consists of the transfer of radioactivity labeled phosphate moiety by the kinase to an acceptor substrate. The resulting 33P-labeled product is separated from unreacted tracer, transferred into a scintillation cocktail and light emitted is measured in a scintillation counter.

### Method for AKT kinase inhibition assay: Dowex technique

### General principle for kinase inhibition assays

Specific peptide or protein substrates are trans-phosphorylated by their specific serine-threonine or tyrosine kinase, in the presence of ATP traced with ³³P-γ-ATP, and in the presence of their own optimal buffer and cofactors.

At the end of the phosphorylation reaction, more than 98% cold ATP and radioactive ATP is captured by an excess of the ion exchange dowex resin; the resin then settles down to the bottom of the reaction plate by gravity.

Supernatant, containing the phosphorylated substrate, is subsequently withdrawn and transferred into a counting plate, then evaluated by β-counting.

### REAGENTS/ASSAY CONDITIONS

### Dowex resin preparation

500 g of wet resin (SIGMA, custom prepared resin DOWEX 1x8 200-400 mesh, 2.5 Kg) are weighed out and diluted to 2 I in 150 mM sodium formate, pH 3.00.

The resin is allowed to settle down (some hours) and then the supernatant is discarded.

After three washes as above over a couple of days, the resin is allowed to settle, the supernatant is discarded and two volumes of 150 mM sodium formate buffer are added per volume of pellet.

The pH is then measured and should be around 3.00.

The washed resin is stable for more than one week; the stock resin is kept at 4° C before use.

### Kinase Buffer (KB):

TRIS 50 mM, pH 7.5
MgCl₂ 10 mM
DTT 1 mM
NaVO₃ 3 µM
BSA 0.2 mg/ml

### Assay conditions (final concentrations) for AKT1

Enzyme concentration (AKT1) = 5 nM
Substrate concentration (Aktide, 14 residues peptide, from PRIMM) = 30 µM
ATP = 132 µM
³³P-γ-ATP = 0.88 nM

### Robotized dowex assay

The "test" mixture consisted of:
1) 3x Enzyme mix (done in Kinase Buffer 3X), 5 µl/well
2) 3x substrate and ATP mix (done in ddH2O), together with ³³P-γ-ATP, 5 µl/well
3) 3x test compounds (diluted into ddH2O - 3% DMSO) - 5 µl/well

### Dilution of compounds

Test compounds are received as 100% DMSO solution at the indicated concentrations:
i - for % inhibition studies, individual dilution plates at 1 mM, 100 µM and 10 µM are prepared in 100% DMSO, then diluted at a 3X concentration (30, 3 and 0.3 µM) in ddH₂O 3% DMSO. A Multimek 96 (Beckman) is used for dilutions and compound pipetting into the test plates
ii - for IC50 determination, compounds are received as 1 mM, 100% DMSO solutions, plated into the first column of a microtiter plate (A1 to G1), 100 µl.

Well H1 is left empty for the internal standard inhibitor, staurosporine.

A Biomek 2000 (Beckman) is used for serial 1:3 dilutions in water, 3% DMSO, from column A1 to A10 and for all the seven compounds in the plate. In a standard experiment, the highest concentration of all compounds is 30 µM, then diluted in the final test mixture down to 10 µM.

Columns 11 and 12 are left available for total activity reference and background evaluation.

### Assay scheme

384-well plates, V bottom (test plates) are prepared with 5 µl of the compound dilution (3X) and then placed onto a PlateTrak 12 robotized station (Perkin Elmer; the robot has one 384-tips pipetting head for starting the assay plus one 96-tips head for dispensing the resin) together with one reservoir for the Enzyme mix (3X) and one for the ATP mix (3X).

At the start of the run, the robot aspirates 5 µl of ATP mix, makes an air gap inside the tips (3 µl) and aspirates 5 µl of AKT mix. The following dispensation into the plates allows the kinase reaction to start upon 3 cycles of mixing, done by the robot itself.

At this point, the correct concentration is restored for all reagents.

The robot incubates the plates for 60 minutes at room temperature, and then stops the reaction by pipetting 70 µl of dowex resin suspension into the reaction mix. Three cycles of mixing are done immediately after the addition of the resin.

The resin suspension has to be carefully stirred during the whole step of reaction stop because its settling velocity is extremely high.

The resin suspension is very dense; in order to avoid tip clogging, wide bore tips are used to dispense it.

Another mixing cycle is performed after all the plates are stopped, this time using normal tips: the plates are then allowed to rest for about one hour in order to maximize ATP capture. At this point, 20 µl of the supernatant are transferred into 384-Optiplates (Perkin-Elmer), with 70 µl of Microscint 40 (Perkin-Elmer); after 5 min of orbital shaking the plates are read on a Perkin-Elmer Top Count radioactivity counter.

Data are analysed by an internally customized version of the SW package "Assay Explorer" that provides either % inhibition for primary assays or sigmoidal fittings of the tendilutions curves for IC50 determination, for the secondary assays/hit confirmation routines. Analogously the inhibition assay of AKT2 or AKT3 activity were performed by employing the relative assay conditions:

### Assay conditions (final concentrations) for AKT2

Enzyme concentration (AKT2) = 10 nM
Substrate concentration (Aktide, 14 residues peptide, from PRIMM) = 8.5 µM
ATP = 396 µM
³³P-γ-ATP = 0.88 nM

### Assay conditions (final concentrations) for AKT3

Enzyme concentration (AKT2) = 2.5 nM
Substrate concentration (Aktide, 14 residues peptide, from PRIMM) = 30 µM
ATP = 180 µM
³³P-γ-ATP = 0.88 nM

### Cell-based assays for inhibitors of AKT1 kinase activity

### Inhibition of AKT induced S6 ribosomal protein phosphorylation in MCF7

MCF-7 cells (ATCC# HTB-22) were seeded in 96-well poly-lysine coated clear- and flat-bottomed black plates (Matrix Technologies Inc., Hudson, NH, USA), in E-MEM medium (MEM+ Earle's BSS + 2 mM glutamine + 0.1 mM non-essential amino acids) containing 10% of FCS at a density of 7000 cells/well, and incubated overnight at 37°C, 5% CO2, 100% relative humidity. After this incubation, cells were treated with increasing compound doses for 4 hour at 37°C. Afterwards, cells were fixed with PBS/3.7% paraformaldehyde for 15 min at room temperature, wash twice with 200 µl/well D-PBS and permeabilize with a D-PBS solution containing 0.1% Triton X-100 (Sigma-Aldrich, St. Louis, MO, USA) and 0.1% powder Milk for 15 minutes (staining solution). To the wells was then, add the primary anti-phospho-S6 (Ser 235/236) antibody (Cell Signaling, cat. # 2211) diluted 1/200 in staining solution (50 µl/well) and incubate for 1 hour at 37°C.Wells were then washed twice with 200 µl/well D-PBS and add the anti-rabbit Cy^{™}5-conjugated (Far-red) secondary antibody (Amersham Biosciences, Little Chalfont, Buckinghamshire, UK) (1/500) in staining solution (50 µl/well) containing 2 µg/ml DAPI (4,6-diamidino-2-phenylindole) and incubate for 1 hour at 37°C. Wells were then washed X2 with 200 µL/well D-PBS, and 200 microL PBS are left in each well for immunofluorescence analysis. Fluorescence images in the DAPI and Cy5™ channels were automatically acquired, stored and analysed using a Cellomics ArrayScan™ IV instrument (Cellomics, Pittsburgh, USA); the Cellomics Cytotoxicity Algorithm was used to quantify cytoplasmic fluorescence associated with phospho-S6 (Cy5™ signal parameter: "Mean Lyso Mass-pH") for each cell in 10 fields/well, and eventually expressed as a mean population value. Unless otherwise stated, reagents were obtained from Sigma-Aldrich, St. Louis, MO, USA.

### Inhibition assay of Cdc7 activity

The inhibiting activity of putative Cdc7 inhibitors and the potency of selected compounds is determined through a method of assay based on the use of Dowex resin capture technology.

The assay consists of the transfer of radioactivity labeled phosphate moiety by the kinase to an acceptor substrate. The resulting 33P-labeled product is separated from unreacted tracer, transferred into a scintillation cocktail and light emitted is measured in a scintillation counter.

### The inhibition assay of Cdc7/Dbf4 activity is performed according to the following protocol

The MCM2 substrate is trans-phosphorylated by the Cdc7/Dbf4 complex in the presence of ATP traced with γ³³-ATP. The reaction is stopped by addition of Dowex resin in the presence of formic acid. Dowex resin particles capture unreacted γ³³-ATP and drag it to the bottom of the well while ³³P phosphorylated MCM2 substrate remains in solution. The supernatant is collected, transferred into Optiplate plates and the extent of substrate phosphorylation is evaluated by β counting.

### The inhibition assay of Cdc7/Dbf4 activity was performed in 96 wells plate according to the following protocol

To each well of the plate were added:
- 10 µl test compound (10 increasing concentrations in the nM to uM range to generate a dose-response curve). The solvent for test compounds contained 3% DMSO. (final concentration 1 %)
- 10 µl substrate MCM2 (6 mM final concentration), a mixture of cold ATP (2 mM final concentration) and radioactive ATP (1/5000 molar ratio with cold ATP).
- 10 µl enzyme (Cdc7/Dbf4, 2 nM final concentration) that started the reaction. The buffer of the reaction consisted in 50 mM HEPES pH 7.9 containing 15 mM MgCl₂, 2 mM DTT, 3 uM NaVO₃, 2mM glycerophosphate and 0.2mg/ml BSA.
- After incubation for 60 minutes at room temperature, the reaction was stopped by adding to each well 150 µl of Dowex resin in the presence of 150 mM formic acid. After another 60 min incubation, 50 µL of suspension were withdrawn and transferred into 96-well OPTIPLATEs containing 150 µl of MicroScint 40 (Packard); after 5-10 minutes shaking the plates were read for 1 min in a Packard TOP-Count radioactivity reader.

IC₅₀ determination: inhibitors were tested at different concentrations ranging from 0.0005 to 10 µM. Experimental data were analyzed by the computer program Assay Explorer using the four parameter logistic equation:
y = bottom+(top-bottom)/(1+10^((logIC₅₀-x)*slope))
   where x is the logarithm of the inhibitor concentration, y is the response; y starts at bottom and goes to top with a sigmoid shape. In the columns 2-4 ot the following Table 1 biochemical assay data for representative compounds are shown; in column 5 the cell-based assay results.

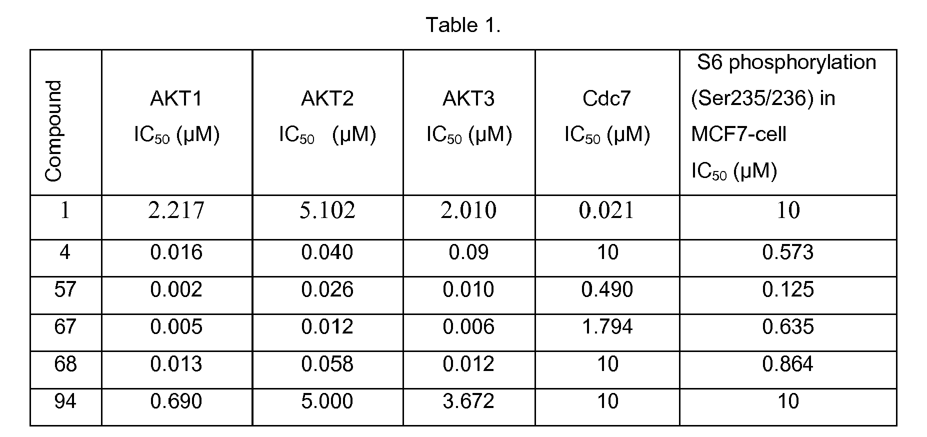

The compounds of the present invention can be administered either as single agents or, alternatively, in combination with known anticancer treatments such as radiation therapy or chemotherapy regimen in combination with cytostatic or cytotoxic agents, antibiotic-type agents, alkylating agents, antimetabolite agents, hormonal agents, immunological agents, interferon-type agents, cyclooxygenase inhibitors (e.g. COX-2 inhibitors), matrixmetalloprotease inhibitors, telomerase inhibitors, tyrosine kinase inhibitors, anti-growth factor receptor agents, anti-HER agents, anti-EGFR agents, anti-angiogenesis agents (e.g. angiogenesis inhibitors), farnesyl transferase inhibitors, ras-raf signal transduction pathway inhibitors, cell cycle inhibitors, other cdks inhibitors, tubulin binding agents, topoisomerase I inhibitors, topoisomerase II inhibitors, and the like.

If formulated as a fixed dose, such combination products employ the compounds of this invention within the dosage range described below and the other pharmaceutically active agent within the approved dosage range.

Compounds of formula (I) can be used sequentially with known anticancer agents when a combination formulation is inappropriate.

The compounds of formula (I) of the present invention, suitable for administration to a mammal, e.g., to humans, can be administered by the usual routes and the dosage level depends upon the age, weight, conditions of the patient and administration route.
For example, a suitable dosage adopted for oral administration of a compound of Formula (I) can range from about 10 to about 500 mg per dose, from 1 to 5 times daily. The compounds of the invention can be administered in a variety of dosage forms, e.g., orally, in the form tablets, capsules, sugar or film coated tablets, liquid solutions or suspensions; rectally in the form suppositories; parenterally, e.g., intramuscularly, or through intravenous and/or intrathecal and/or intraspinal injection or infusion.

The present invention also includes pharmaceutical compositions comprising a compound of formula (I) or a pharmaceutically acceptable salt thereof in association with a pharmaceutically acceptable excipient, which can be a carrier or a diluent.

The pharmaceutical compositions containing the compounds of the invention are usually prepared following conventional methods and are administered in a suitable pharmaceutical form.

For example, the solid oral forms can contain, together with the active compound, diluents, e.g., lactose, dextrose saccharose, sucrose, cellulose, corn starch or potato starch; lubricants, e.g., silica, talc, stearic acid, magnesium or calcium stearate, and/or polyethylene glycols; binding agents, e.g., starches, arabic gum, gelatine methylcellulose, carboxymethylcellulose or polyvinyl pyrrolidone; disintegrating agents, e.g., starch, alginic acid, alginates or sodium starch glycolate; effervescing mixtures; dyestuffs; sweeteners; wetting agents such as lecithin, polysorbates, laurylsulphates; and, in general, non-toxic and pharmacologically inactive substances used in pharmaceutical formulations. These pharmaceutical preparations can be manufactured in known manner, for example, by means of mixing, granulating, tabletting, sugar-coating, or film-coating processes.

The liquid dispersions for oral administration can be, e.g., syrups, emulsions and suspensions.

As an example, the syrups can contain, as carrier, saccharose or saccharose with glycerine and/or mannitol and sorbitol.

The suspensions and the emulsions can contain, as examples of carriers, natural gum, agar, sodium alginate, pectin, methylcellulose, carboxymethylcellulose, or polyvinyl alcohol.

The suspension or solutions for intramuscular injections can contain, together with the active compound, a pharmaceutically acceptable carrier, e.g., sterile water, olive oil, ethyl oleate, glycols, e.g., propylene glycol and, if desired, a suitable amount of lidocaine hydrochloride.

The solutions for intravenous injections or infusions can contain, as a carrier, sterile water or preferably they can be in the form of sterile, aqueous, isotonic, saline solutions or they can contain propylene glycol as a carrier.

The suppositories can contain, together with the active compound, a pharmaceutically acceptable carrier, e.g., cocoa butter, polyethylene glycol, a polyoxyethylene sorbitan fatty acid ester surfactant or lecithin.

With the aim to better illustrate the present invention, without posing any limitation to it, the following examples are now given.

### EXPERIMENTAL SECTION

The compounds of Formula I were prepared according to the previously described synthetic schemes and individual examples are detailed therein. It is to be noted that in the tables the cells can be splitted in two pages, and the header row is not repeated at the beginning each page. EMASS means Exact Mass, CALC calculated..
The compounds were named using ACDLABS 9.0.

As used herein the symbols and conventions used in the processes, schemes and examples are consistent with those used in the contemporary scientific literature, for example, the *Journal of the American Chemical Society or* the *Journal* of *Biological Chemistry.*

The following HPLC method was used in the analysis of the compounds, as specified in the synthetic examples set forth below. As used herein, the term "Rt" refers to the retention time (minutes) for the compound using the HPLC method specified below.

### LC-MS Method

HPLC/MS was performed on a Waters X Terra RP 18 (4.6 x 50 mm, 3.5 µm) column using a Waters 2790 HPLC system equipped with a 996 Waters PDA detector and a Micromass mod. ZQ single quadrupole mass spectrometer, equipped with an electrospray (ESI) ion source. Mobile phase A was ammonium acetate 5 mM buffer (pH 5.5 with acetic acid / acetonitrile 95:5), and Mobile phase B was water / acetonitrile (5:95). Gradient from 10 to 90% B in 8 minutes, hold 90% B 2 min. UV detection at 220 nm and 254 nm. Flow rate 1 mL/min. Injection volume 10 µl. Full scan, mass range from 100 to 800 amu. Capillary voltage was 2.5 KV; Source temperature was 120°C; Cone was 10 V. Retention Times (LC-MS Rt) are given in minutes at 220 nm or 254 nm. Mass are given as m/z ratio.

### Purification by HPLC

When necessary, compounds were purified by preparative HPLC on a Waters Symmetry C18 (19 x 50 mm, 5 um) column or on a Waters X Terra RP 18 (30 x 150 mm, 5 µm) column using a Waters preparative HPLC 600 equipped with a 996 Waters PDA detector and a Micromass mod. ZMD single quadrupole mass spectrometer, electron spray ionization, positive mode. Mobile phase A was water-0.01% trifluoroacetic acid, and mobile phase B was acetonitrile. Gradient from 10 to 90% B in 8 min, hold 90% B 2 min. Flow rate 20 mL/min. In alternative, mobile phase A was water-0.1% NH₃, and mobile phase B was acetonitrile. Gradient from 10 to 100% B in 8 min, hold 100% B 2 min. Flow rate 20 mL/min.

### Example 1

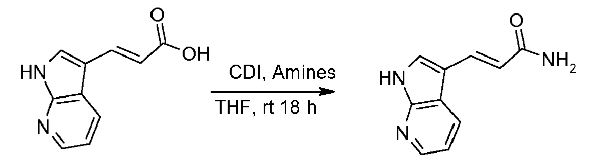

Registry number 123530-68-7

### (E)-3-(1H-Pyrrolo[2,3-b]pyridin-3-yl)-acrylamide

(E)-3-(1H-Pyrrolo[2,3-b]pyridin-3-yl)-acrylic acid (100mg 0.52mmol) was dissolved in 5 ml of THF with carbonyl diimidazole (0.172mg 0.75mmol 1.5 eq). The mixture was stirred at room temperature for 18h. A solution of ammonium hydroxyde (1ml) was added, after 1 h the reaction was complete, the solvent was removed and the residue was partitioned between water and ethylacetate, the organic solvent was dried over Na₂SO₄ and evaporated to afford 95 mg of (E)-3-(1H-Pyrrolo[2,3-b]pyridin-3-yl)-acrylamide as off-white solid (96 % yield).

1H NMR (400 MHz, DMSO-D6) δ ppm: ), 12.06 (bs, 1H); 8.31 (dd, J = 5.58, 1.99 Hz 1H), 8.27 (dd, J = 8.27, 1.59 Hz, 1H), 7.80 (d, J= 3.53 Hz, 1H), 7.58 (d, J= 15.28Hz, 1H), 7.33 (bs, 1H), 7.23 (dd, J =8.22, 4.71 Hz, 1H), 6.87 (bs, 1H), 6.66 (d, 1H, J= 15.28).

MS (ESI) (m/z) : 188.08 (M+1, 100).

HRMS (ESI): 188.0813 (C10 H9 N3 O, M+1; calc. 188.0818).

Analogously but employing MeNH₂ in place of NH₄OH, the following compound was obtained:

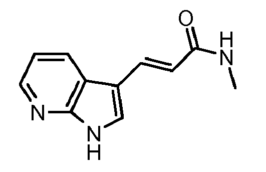

### (E)-N-Methyl-3-(1H-pyrrolo[2,3-b]pyridin-3-yl)-acrylamide

1H NMR (400 MHz, DMSO-D6) δ ppm: 12.05 (bs, 1H), 8.31 (dd, J= 5.06, 1.27Hz, 1H), 8.26 (dd, J= 7.59, 1.27Hz, 1H), 7.82 (bm, 2H), 7.89 (d, J= 3.16, 1H), 7.57 (d, J= 15.26 Hz, 1H), 7.23 (dd, J1 = 7.59 and J2 = 5.06 Hz, 1H), 6.62 (d, J= 15.26Hz, 1H), 2.73 (s, 3H),

MS (ESI) (m/z) : 202.23 (M+1, 100).

HRMS (ESI): 202.0973 (C11 H11 N3 O, M+1; calc. 202.0975.

### Example 2

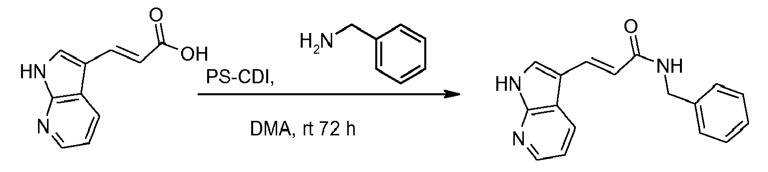

### (2E)-N-Benzyl-3-(1H-pyrrolo[2,3-b]pyridin-3-yl)-acrylamide

50 mg (0.26 mmol) of the (E)-3-(1H-Pyrrolo[2,3-b]pyridin-3-yl)-acrylic acid were dissolved in 3ml of DMA (Dimethylacetamide) and added of 34 µL (0.32 mmol 1.2 eq) of benzylamine, to the solution was added 400 mg (0.5 mmol, 2 eq) of Polymer-Supported-dicyclohexylcarbodiimide (1.25mmol/g). The slurry was stirred at room temperature for 72 h. The beads were filtered off and washed twice with 1ml of DMA, the organics washes were combined and solvent evaporated. The residue was washed with diethylether that dissolved only the excess of amine leaving the product as solid that was dried to afford 35 mg of (E)-N-Benzyl-3-(1H-pyrrolo[2,3-b]pyridin-3-yl)-acrylamide as pure off-white solid (50% yield).

1H NMR (400 MHz, DMSO-D6) δ ppm 12.07 (s, 1H), 8.32 (m, 3H), 7.91 (d, J= 3.46Hz, 1H), 7.63 (d, J= 15.59 Hz, 1H), 7.34 (m, 4H), 7.27 (m, 1H), 7.21 (dd, J= 4.33, 7.79Hz, 1H), 6.75 (d, J= 15.59, 1H), 4.44 (d, J= 6.14 Hz, 2H).

MS (ESI) (m/z): 278.12 (M+1, 100).

HRMS (ESI): 278.1295 (C17 H15 N3 O M+H; calc 278.1288)

Analogously the following compounds have been obtained:

| R¹ | Name | EMASS CALC | EMASS FOUND | MOL FORMULA | 1H NMR (400 MHz, DMSO-D6) δ ppm |
|---|---|---|---|---|---|
| 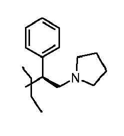 | (2E)-N-[(1S)-1-phenyl-2-pyrrolidin-1-ylethyl]-3-(1H-pyrrolo[2,3-b]pyridin-3-yl)acrylamide | 361.2023 | 361.2021 | C₂₂ H₂₄ N₄ O | 12.06(bs, 1H), 8.33 (m 3 H), 7.88 (d, J= 2.91Hz, 1H), 7.55 (d, J= 15.56 Hz, 1H), 7.36 (m, 4H), 7.26 (m, 2H), 6.78 (d, J= 15.56 Hz, 1H), 5.08 (m, 1H), 2.84 (m, 1H), 2.64 (m, 1H), 2.52 (m, 4H), 1.68 (m, 4 H). |
| 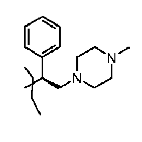 | (2E)-N-[(1S)-2-(4-methylpiperazin-1-yl)-1-phenylethyl]-3-(1H-pyrrolo[2,3-b]pyridin-3-yl)acrylamide | 390.2288 | 390.2296 | C₂₃ H₂₇ N₅ O | 12.06 (bs, 1H), 8.32 (m, 2H), 8.28 (d, J= 8.36 Hz, NH), 7.89 (d, J= 2.57Hz, 1H), 7.55 (d, J= 15.74 Hz, 1H), 7.36 (m, 4H), 7.25 (m, 2 H), 6.78 (d, J= 15.74 Hz, 1H), 5.14 (ddd, J= 9.87, 8.36, 5.55 Hz, 1H), 2.68 (dd, J= 12.84, 9.87 Hz, 1H9, 2.49 (m, 9H), 2.14 (s, 3H). |
| 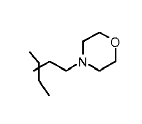 | (2E)-N-(2-morpholin-4-ylethyl)-3-(1H-pyrrolo[2,3-b]pyridin-3-yl)acrylamide | 301.1659 | 301.1645 | C₁₆ H₂₀ N₄ O₂ | 12.05 (s, NH), 8.32 (bs, 1H), 8.30 (dd, J= 3.43, 1.52 Hz, 1H), 7.89 (d, J= 2.70 Hz, 1H), 7.83 (dd, J= 6.86, 5.63 Hz, NH), 7.55 (d, J= 16.33 Hz, 1H), 7.23 (m, 1H), 6.70 (d, J= 16.33 Hz, 1H), 3.60 (m, 4H), 3.35 (m, 2H), 2.43 (m, 6H). |
| 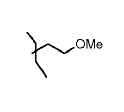 | (2E)-N-(2-methoxyethyl)-3-(1H-pyrrolo[2,3-b]pyridin-3-yl)acrylamide | 246.1237 | 246.1243 | C₁₃ H₁₅ N₃ O₂ | 12.05 (s, NH), 8.32 (dd, J= 3.65, 1.43 Hz, 1H), 8.31 (bs, 1H), 7.95 (dd, 5.77, 5.41Hz, NH), 7.89 (d, J= 2.86Hz, 1H), 7.55 (d, J= 16.01Hz, 1H), 7.23 (m, 1H), 6.74 (d, J= 16.01 Hz, 1H), 3.40 (m, 4H), 3.30 (s, 3H). |
| 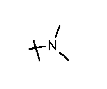 | (2E)-N,N-dimethyl-3-(1H-pyrrolo[2,3-b]pyridin-3-yl)acrylamide | 216.1131 | 216.1139 | C₁₂ H₁₃ N₃ O | 12.09(s, NH), 8.37(dd, J= 1.46, 8.90 Hz, 1 H), 8.31 (dd J= 1.46 4.76 Hz 1H), 8.00( d, J= 2.80 Hz, 1H), 7.67 (d, J= 15.49 Hz, 1H), 7.19 (dd J= 4.76, 8.90 Hz 1H), 6.95 (d, J= 15.49 Hz, 1H), 3.19 (s, 3H), 2.96 (s, 3H). |
| 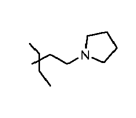 | (2E)-N-(2-pyrrolidin-1-ylethyl)-3-(1H-pyrrolo[2,3-b]pyridin-3-yl)acrylamide | 285.171 | 285.1711 | C₁₆ H₂₀ N₄ O | 12.05 (s, NH), 8.31 (m, 2H), 7.88 (d, J= 2.88 Hz, 1H), 7.86 (bt, NH), 7.55 (d, J= 15.85 Hz 1H), 721 (dd, J= 8.90 5.12 Hz, 1H), 6.72 (d, J= 15.85 Hz, 1H), 3.34 (m, 2H), 2.52 (m, 6H), 1.72 (m, 4H). |
| 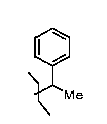 | (2E)-N-(1-phenylethyl)-3-(1H-pyrrolo[2,3-b]pyridin-3-yl)acrylamide | 292.1444 | 292.144 | C₁₈ H₁₇ N₃ O | 12.06 (bs, NH), 8.32 (m, 3H), 7.89 (d, J= 2.80 Hz, 1H), 7.55 (d, J= 15.85 Hz, 1H), 7.37 (m, 4H), 7.25 (m, 2H), 6.76 (d, J= 15.85Hz, 1H), 5.07 ( dq, J= 6.95, 7.80 Hz, 1H), 1.44 (d, J= 6.95 Hz, 3H). |
| 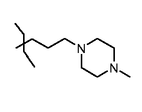 | (2E)-N-[3-(4-methylpiperazin-1-yl)propyl]-3-(1H-pyrrolo[2,3-b]pyridin-3-yl)acrylamide | 328.2132 | 328.2144 | C₁₈ H₂₅ N₅ O | 12.05 (bs, NH), 8.31 (dd, 1.46, 4.70 Hz, 1H), 8.27 (dd, J= 1.46, 7.9 Hz, 1H), 7.89 (m, 2H), 7.54 (d, J= 15.85 Hz, 1H), 7.21 J= 7.89, 4.70 Hz, 1H), 6.65 (d, J= 15.85 Hz, 1H), 3.30 (m, 6H), 2.35 (m, 6H), 2.20 (s, 3H), 1.63 (m, 2H). |
| 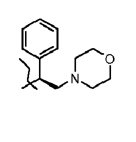 | (2E)-N-[(1S)-2-morpholin-4-yl-1-phenylethyl]-3-(1H-pyrrolo[2,3-b]pyridin-3-yl)acrylamide | 377.1972 | 377 1985 | C₂₂ H₂₄ N₄ O₂ | 12.07 (s, NH), 8.32 (m, 3H), 7.90 (d, J= 2.56 Hz, 1H), 7.54 (d, J= 15.85 Hz, 1H9, 7.37 (m, 4H), 7.27 (m, 1H), 7.25 (dd J= 4.88, 8.90 Hz, 1H), 6.78 (d, J= 15.85 Hz, 1H), 5.17 (m, 1H), 3.56 (m, 4H), 2.69 (m, 4H), 2.36 (m, 2H). |
| 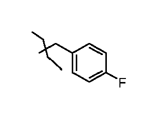 | (2E)-N-(4-fluorobenzyl)-3-(1H- 296.1194 pyrrolo[2,3-b]pyridin-3-yl)acrylamide | 296.1194 | | C₁₇ H₁₄ F N₃ O | 12.07 8s, NH), 8.37 (bt, J= 6.83 Hz, 1H), 8..32 (dd, J= 4.70, 1.47 Hz, 1H), 8.27 (dd, J= 7.93, 1.47 Hz, 1H), 7.91 (d, J= 2.82 Hz, 1H), 7.61 (d, J= 15.85 Hz, 1H), 7.35 (m, 2H), 7.21 (dd, J= 7.93, 4.70 Hz, 1H), 7.18 (m, 2H), 6.73 (d, J= 15.85 Hz, 1H), 4.42 (d, J= 6.83 Hz, 2H). |
| 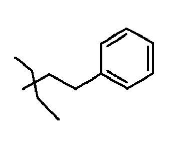 | (2E)-N-(2-phenylethyl)-3-(1H-pyrrolo[2,3-b]pyridin-3-yl)acrylamide | 292.1444 | 292.1457 | C₁₈ H₁₇ N₃ O | 12.05 (s, NH), 8.32 (dd, J= 4.63, 1.43 Hz, 1H), 8.26 (dd, J= 8.16, 1.43 Hz, 1H), 7.96 (t, J= 5.72 Hz, 1H), 7.89 (d, J= 2.89 Hz 1H 7.56 (J= 15.85 Hz, 1H), 7.28 (m, 6H), 6.66 (d, J= 15.85 Hz, 1H), 3.46 (m, 2 H), 2.17 (t, J= 7.49 Hz, 1H). |
| 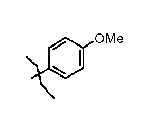 | (2E)-N-(4-methoxyphenyl)-3-(1H-pyrrolo[2,3-b]pyridin-3-yl)acrylamide | 294.1237 | | C₁₇ H₁₅ N₃ O₂ | 12.14 (s, NH), 9.86 (s, NH), 8.35 (m, 2H), 7.97 (d, J= 2.80 1H), 7.70 (d, J= 15.73 Hz,1H), 7.62 (d, J= 6.95 Hz, 2H), 7.26 (m, 1H), 6.94 (d, J= 6.95 Hz, 2H), 6.84 (d, J= 15.73 Hz, 1H), 3.75 (s, 3H). |
| 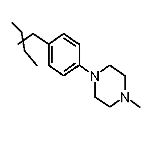 | (2E)-N-[4-(4-methylpiperazin-1-yl)benzyl]-3-(1H-pyrrolo[2,3-b]pyridin-3-yl)acrylamide | 376.2132 | 376.2138 | C₂₂ H₂₅ N₅ O | 12.05 (s, NH), 8.31 (m, 2H), 8.21 (t, J= 5.85 1H), 7.90 (d, J= 2.68 Hz, 1H), 7.59 (d, J= 15.85 Hz, 1H), 7.21 (dd, J= 8.17, 4.63Hz 1H), 7.17 (d, J= 8.66 Hz, 2H), 6.93 (d, J= 8.66 Hz, 2H), 6.73 (d, J= 15.85 Hz, 1H), 4.32 (d, J= 5.85 Hz, 2H), 3.12 (m, 4H), 2.48 (m, 4H), 2.25 (s, 3H). |
| 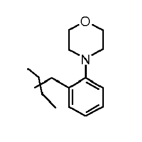 | (2E)-N-(2-morpholin-4-ylbenzyl)-3-(1H-pyrrolo[2,3-b]pyridin-3-yl)acrylamide | 363.1815 | | C₂₁ H₂₂ N₄ O₂ | 12.07 (bs, NH), 8.30 (m, 2H), 8.23 (t, J= 5.73 Hz, 1H), 7.91 (s, 1H), 7.62 (d, J= 15.85 Hz, 1H), 7.17 (m, 5H), 6.78 (d, J= 15.85 Hz, 1H), 4.53 (d, J= 5.73 Hz, 2H), 3.77 (m, 4H), 2.88 (m, 4H). |
| 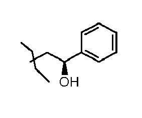 | (2E)-N-[(2R)-2-hydroxy-2-phenylethyl]-3-(1H-pyrrolo[2,3-b]pyridin-3-yl)acrylamide | 308.1393 | 308.1397 | C₁₈ H₁₇ N₃ O₂ | 12.04 (bs, NH), 8.31 (m, 2H), 7.99 (t, J= 5.49 Hz, 1H), 7.89 (d, J= 2.80 Hz, 1H), 7.55 (d, J= 15.85 Hz, 1H), 7.39 (m, 4H), 7.27 7.39 (m, 4H), 7.27 (m 1H), 7.21 (dd, J= 8.90, 4.76 Hz, 1H), 6.79 (d, J= 15.85Hz, 1H), 5.56 (d, J= 4.39 Hz, OH), 4.68 (m, 1H), 3.49 (m, 2H). |
| 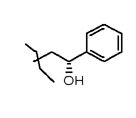 | (2E)-N-[(2S)-2-hydroxy-2-phenylethyl]-3-(1H-pyrrolo[2,3-b]pyridin-3-yl)acrylamide | 308.1393 | 308.1396 | C₁₈ H₁₇ N₃ O₂ | 12.04 (bs, NH), 8.31 (m, 2H), 7.99 (t, J= 5.49 Hz, 1H), 7.89 (d, J= 2.80 Hz, 1H), 7.55 (d, J= 15.85 Hz, 1H), (d, J= 15.85 Hz, 1H), 7.39 (m, 4H), 7.29 7.39 (m, 4H), 7.29 8.09, 4.76 Hz, 1H), 8.09, 4.76 Hz, 1H), 6.79 (d, J= 15.85Hz, 1H), 5.56 (d, J= 4.53 Hz, OH), 4.68 (m, 1H), 3.49 (m, 2H). |
| 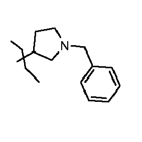 | (2E)-N-(1-benzylpyrrolidin-3-yl)-3-(1H-pyrrolo[2,3-b]pyridin-3-yl)acrylamide | 347.1866 | 347.1863 | C₂₁ H₂₂ N₄ O | 12.04 (bs, NH), 8.30 (m, 2H), 8.05 (d, J= 7.19 Hz, 1H), 7.87 (d, J= 2.87 Hz, 1H), 7.52 (d, J= 15.73 Hz, 1H), 7.34 (m, 6H), 6.69 (d, J= 15.73Hz, 1H), 4.31 (m, 1H), 3.61 (bs, 2H), 2.69 (m, 2H), 2.41 (m, 2H), 2.13 (m, 1H), 1.62 (m, 1H). |
| 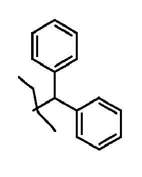 | (2E)-N-(diphenylmet hyl)-3-(1H-pyrrolo[2,3-b]pyridin-3-yl)acrylamide | 354.1601 | | C₂₃ N₁₉ N₃ O | 12.07 (bs, NH), 8.75 (d, J= 8.66 Hz, 1H), 8.33 (m, 2H), 7.91 d, J= 2.68 hz, 1H), 7.61 (d, J0 15.85 Hz, 1H), 7.30 (m, 10H) 7.21 (dd, J= 8.13, 4.73 7.21 1H), 6.89 (d, J0 15.85 Hz, 1H), 6.27 (d, J= 8.66 Hz, 1H). |
| 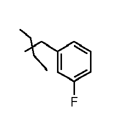 | (2E)-N-(3-fluorobenzyl)-3-(1H-pyrrolo[2,3-b]pyridin-3-yl)acrylamide | 296.1194 | 296.12 | C₁₇ H₁₄ F N₃ | 12.08 (bs, NH), 8.41 (t, J= 6.10Hz, NH), 8.31 (dd, J= 4.63, 1.46 Hz, 1H), 8.28 (dd, J= 8.05, 1.46, Hz, 1H), 7.92 (d, J=2.80 Hz, 1H), 7.62 (d, J= 15.85 Hz, 1H), O 7.40 (m, 1H), 7.21 (dd, J= 8.05 4.63 Hz, 1H), 7.18 (m, 1H), 7.12 (m, 3H), 6.75 (d, J= 15.85Hz, 1H), 4.45 (d, J= 6.10 Hz, 2H). |
| 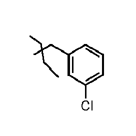 | (2E)-N-(3-chlorobenzyl)-3-(1H)-pyrrolo[2,3-b]pyridin-3-yl)acrylamide | 312.0898 | | C₁₇H₁₄ Cl N₃ O | 12.08 (s, NH), 8.42 (t, J= 5.98 Hz, 1H), 8.32 (dd, J= 4.60, 1.46 Hz, 1H), 8.29 (dd, J= 7.93, 1.46 Hz, 1H), 7.92 (d, J= 2.80Hz, 1H), 7.64 (d, J= 15.85, 1H), 7.35 (m, 4H), 7.23 (dd, J= 7.93, 4.60 Hz, 1H), 6.70 (d, J= 15.85, 1H), 4.44 (d, J= 5.98 Hz, 2H). |
| 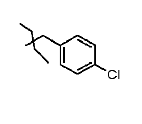 | (2E)-N-(4-chlorobenzyl)-3-(1H-pyrrolo[2,3-b]pyridin-3-yl)acrylamide | 312.0898 | | C₁₇ H₁₄ Cl N₃ O | 12.07 (bs, NH), 8.39 (t, J= 6.10 Hz, 1H), 8.31 (dd, J= 4.75, 1.46 Hz, 1H), 8.28 (dd, J= 8.06, 1.46 Hz, 1H), 7.91 (d, J= 2.81, 1H), 7.63 (d, J= 15.85 Hz, 1H), 7.41 (m, 2H), 7.35 (m, 2H), 7.23 (dd, J= 8.06, 4.75 Hz, 1H), 6.71 (d, J= 15.85, 1H), 4.41 (d, J= 6.10 Hz, 2H). |
| 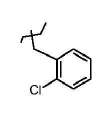 | (2E)-N-(2-chlorobenzyl)-3-(1H-pyrrolo[2,3-b]pyridin-3- yl)acrylamide | 312.0898 | | C₁₇ H₁₄ Cl N₃ O | 12.08 8 bs, NH), 8.37 (t, J= 5.85 Hz, 1H), 8.32 (m, 2H), 7.92 (d, J= 2.68 Hz, 1H), 7.62 7.47 (m, 1H), 7.41 7.47 (m, 1H), 7.41 (dd J= 7.35, 2.45 Hz, 2H), 7.35 (m, 2H), 7.22 (dd, J= 8.05, 4.55 Hz, 1H), 6.80 (d, J= 15.85 Hz, 1H), 4.51 (d, J= 5.85 Hz, 2H). |
| 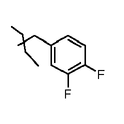 | (2E)-N-(3,4-difluorobenzyl)-3-(1H-pyrrolo[2,3-b]pyridin-3-yl)acrylamide | 314.1099 | | C₁₇ H₁₃ F₂ N₃ O | 12.09 (bs, NH), 8.43 (t, J= 5.97 Hz, 1H), 8.31 (dd, J= 4.81, 1.45 Hz, 1H), 8.28 (dd, J= 7.90, 1.37 Hz, 1H), 7.92 (d, J= 2.86 Hz,1H),7.65(d,J= 15.68 Hz, 1H), 7.38 (m 2H), 7.23 dd, J= 7.90, 4.81 Hz, 1H), 7.18 (m, 1H), 6.71 (d, J= 15.68 Hz, 1H), 6.73 (d, J= 5.97, 2H). |
| 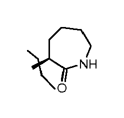 | (2E)-N-[(3S)-2-oxoazepan-3-yl]-3-(1H-pyrrolo[2,3-b]pyridin-3-yl)acrylamide | 299.1502 | 299.1496 | C₁₆ H₁₈ N₄ O₂ | 12.03 (bs, NH), 8.47 (dd, J= 8.04, 1.10 Hz, 1H), 8.30 (dd, J= 4.73, 1.46 Hz, 1H), 7.99 (d, J= 6.95 Hz, 1H), 7.89 (d, J= 2.80 Hz, 1H), 7.82 (m, NH), 7.54 (d, J= 15.85 Hz, 1H), 7.19 (dd, J= 8.04, 4.73 Hz, 1H), 6.97 (d, J= 15.85 Hz, 1H), 4.57 (m, 1H), 3.29 (m, 2H), 1.90 (m, 3H), 1.46 (m, 3H). |
| 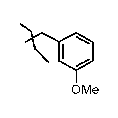 | (2E)-N-(3-methoxybenzyl)-3-(1H-pyrrolo[2,3-b]pyridin-3-yl)acrylamide | 308.1393 | 308.1405 | C₁₈ H₁₇ N₃ O₂ | 12.07 (bs, NH), 8.32 (m, 3H), 7.91 (d, J= 2.80 Hz, 1H), 7.61 (d, J= 15.85 Hz, 1H),7.27 (m, 2H), 6.90 (m, 3H), 6.86 (m, 2H), 6.75 (d, J= 15.85 Hz, 1H), 4.41 (d, J= 5.97 Hz, 2H), 3.75 (s, 3H). |
| 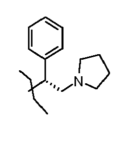 | (2E)-N-[(1R)-1-phenyl-2-pyrrolidin-1-ylethyl]-3-(1H- pyrrolo[2,3-b]pyridin-3-yl)acrylamide | 361.2023 | 361.2015 | C₂₂ H₂₄ N₄ O | 12.06 (bs, NH), 8.33 (m, 3H), 7.89 (d, J= 2.56 Hz, 1H), 7.53 (d, J= 15.85 Hz, 1H), 7.37 (m, 4H), 7.26 (m, 2H), 6.80 (d, J= 15.85 Hz, 1H), 5.09 ( b, 1 H), 2.82 (b, 1 H), 2.65 (b, 3H), 1.68 (m, 4H). |
| 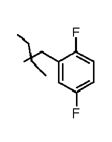 | (2E)-N-(2,5-difluorobenzyl)-3-(1H-pyrrolo[2,3-b]pyridin-3-yl)acrylamide | 314.1099 | | C₁₇ H₁₃ F₂ N₃ O | 12.09 (bs, NH), 8.40 (t, J= 5.73 Hz, 1H), 8.32 (dd, J= 4.82, 1.48 Hz, 1H), 8.30 (dd, J= 8.01, 1.48 Hz, 1H), 7.93 (d, J= 2.80 Hz, 1H), 7.63 (d, J= 15.85 Hz, 1H), 7.25 (m, 4H), 6.74 (d, J= 15.85 Hz, 1 H), 4.44 (d, J= 5.73 Hz, 2H), |
| 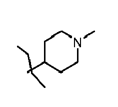 | (2E)-N-(1-methylpiperidin-4-yl)-3-(1H-pyrrolo[2,3-b]pyridin-3-yl)acrylamide | 285.171 | 285.171 | C₁₆ H₂₀ N₄ O | 12.04 (bs, NH), 8.31 (m, 2H), 7.88 (d, J= 2.80 Hz, 1H), 7.81 (d, J= 7.56 Hz, 1H), 7.54 (d, J= 15.85 Hz, 1H), 7.23 (dd, J= 7.83, 4.53 Hz, 1H), 6.57 (d, J= 15.85 Hz, 1H), 3.67 (m, 1H), 2.80 (m, 2H), 2.20 (s, 3H), 1.97 (m, 2H), 1.79 (m, 2H), 1.47 (m, 2H). |
| 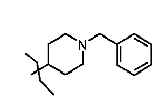 | (2E)-N-(1-benzylpiperidin-4-yl)-3-(1H-pyrrolo[2,3-b]pyridin-3-yl)acrylamide | 361.2023 | 361.2028 | C₂₂ H₂₄ N₄ O | 12.04 (bs, NH), 8.30 (m, 2H), 7.88 (d, J=1.78 Hz, 1H), 7.79 (d, J= 7.68 Hz, 1H), 7.54 (d, J= 15.85 Hz, 1H), 7.33 (m, 6H), 6.67 (d, J= 15.85 Hz, 1H), 3.68 (m, 1H), 3.48 (m, 2H), 2.76 (m, 2H), 2.09 (m, 2H), 1.80 (m, 2H), 1.46 (m, 2H). |
| 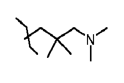 | (2E)-N-[3-(dimethylamino)-2,2-dimethylpropyl]-3-(1H-pyrrolo[2,3-b]pyridin-3-yl)acrylamide | 301.2023 | 301.2015 | C₁₇ H₂₄ N₄ O | 12.04 (bs, NH), 8.33 (m, 2H), 7.89 (d, J= 2.80Hz, 1H), 7.63 (b, 1 H), 7.55 (d, J= 15.85 Hz, 1H), 7.22 dd, J= 8.17, 4.63 Hz, 1H), 6.88 (d, J= 15.85 Hz, 1H), 3.13 (m, 2H), 2.26 (bs, 6H), 2.13 (m, 2H), 0.88 (s, 6H). |
| 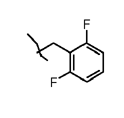 | (2E)-N-(2,6-difluorobenzy I)-3-(1H- pyrrolo[2,3-b]pyridin-3-yl)acrylamide | 314.1099 | 314.1096 | C₁₇ H₁₃ F₂ N₃ | 12.06 (bs, NH), 8.30 (dd, J= 4.78, 1.59 Hz, 1H), 8.25 ( dd, J= 8.15, 1.59 Hz, 1H), 8.22 (t, J= 5.37 Hz, 1H), 7.90 (d, J= 2.80, 1 H), 7.57 (d, J= 15.85 Hz, 1H), 7.44 (m, 1H), 7.21 (dd, J= 8.15, 4.78 Hz, 1 H), 7.14 (m, 2H), 6.70 (d, J= 15.85 Hz, 1H), 4.48 (d, J= 5.37 Hz, 2H). |
| 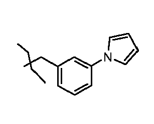 | (2E)-3-(1H-pyrrolo[2,3-b]pyridin-3-yl)-N-[3-(1H-pyrrol-1-yl)benzyl]acry lamide | 343.1553 | | C₂₁ H₁₈ N₄ O | 12.08 (bs, NH), 8.40 (t, J= 5.73 Hz, NH), 8.30 (m, 2H), 7.92 (d, J= 2.80 Hz, 1H), 7.62 (d, J= 15.85 Hz, 1H), 7.53 (m, 1H), 7.47 (m, 2H), 7.35 (d, J= 2.32 Hz, 2H), 7.21 (m, 2H), 6.76 (d, J= 15.85 Hz, 1H), 6.28 (d, J= 2.32 Hz, 2H), 4.48 (d, J= 5.73 Hz, 2H). |
| 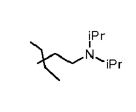 | (2E)-N-[2-(diisopropylamino)ethyl]-3-(1H-pyrrolo[2,3-b]pyridin-3-yl)acrylamide | 315.2179 | 315.2169 | C₁₈ H₂₆ N₄ O | 12.05, (bs, NH), 8.32 (dd, J= 4.72, 1.59 Hz, 1H), 8.28 (dd, J= 8.07, 1.59 Hz, 1H), 7.89 (d, J= 2.80 Hz, 1H), 7.78 (m, 1H), 7.55 (d, J= 15.85 Hz, 1H), 7.23 ( dd, J= 8.07, 4.72 Hz, 1H), 6.65 (d, J= 15.85 Hz, 1H), 3.14 (bm, 2H), 2.35 (bm, 4H), 0.99 (m, 12H). |
| 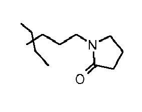 | (2E)-N-[3-(2-oxopyrrolidin-1-yl)propyl]-3-(1H-pyrrolo[2,3-b]pyridin-3-yl)acrylamide | 313.1659 | 313.1654 | C₁₇ H₂₀ N₄ O₂ | 12.06 (bs, NH), 8.32 (dd, J= 4.73, 1.46 Hz, 1 H), 8.28 (dd, J= 8.09, 1.46 Hz, 1 H), 7.89 (m, 2H), 7.56 (d, J= 15.85 Hz, 1H), 7.21 (dd, J= 8.09, 4.73 Hz, 1H), 6.66 (d, J= 15.85 Hz, 1H), 3.36 (m, 3H), 3.24 (m, 3H), 2.23 (m, 2H), 1.94 (m, 2H), 1.67 (m, 2H). |
| 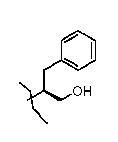 | (2E)-N-[(1S)-1-benzyl-2-hydroxyethyl] -3-(1H-pyrrolo[2,3-b]pyridin-3-yl)acrylamide | 322.155 | 322.1553 | C₁₉ H₁₉ N₃ O₂ | 12.03 (bs, NH), 8.32 (m, 2H), 7.87 (d, J= 2.68 Hz, 1H), 7.79 (d, J= 8.41 Hz, 1H), 7.50 (d, J= 15.85 Hz, 1H), 7.27 (m, 6H), 6.71 (d, J= 15.85 Hz, 1H), 4.86 (bt, OH), 4.08 (m, 1H), 3.44 (m, 2H), 2.92 (dd, J= 14.02, 5.98 Hz, 1H), 2.73 (dd, J= 14.02, 5.98 Hz, 1H). |
| 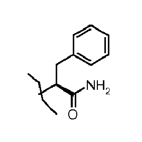 | N-α-[(2E)-3-(1H-pyrrolo[2,3-b]pyridin-3-yl)prop-2-enoyl]-L-phenylalanin amide | 335.1502 | 335.1512 | C₁₉H₁₈H₄O₂ | 12.05 (bs, NH), 8.10 (b, 2H), 8.07 (d, J= 8.41 Hz, 1H), 7.87 (d, J= 2.80 Hz, 1H), 7.51 (d, J= 15.85 Hz, 1H), 7.28 (m, 6H), 7.06 (m, 2H), 6.78 (d, J= 15.85 Hz, 1H), 4.65 (m,1H). 2.96 (m, 1H), 2.85 (m, 1H). |
| 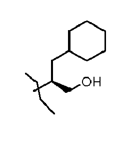 | (2E)-N-[(1S)-2-cyclohexyl-1-(hydroxymethyl)ethyl]-3-(1H-pyrrolo[2,3-b]pyridin-3-yl)acrylamide | 328.2019 | 328.2024 | C₁₉ H₂₅ N₃ O₂ | 12.04 (bs, NH), 8.31 (m, 2H), 7.87 (d, J= 2.80 Hz, 1H), 7.52 (d, J= 15.85 Hz, 1H), 7.21 (dd, J= 8.66, 4.76 Hz, 1H), 6.73 (d, J= 15.85 Hz, 1H), 5.25 (b, 1H), 3.96 (m, 1H), 3.60 (dd, J= 12.07, 3.05 Hz, 1H), 3.39 (m, 1H), 3.15 (m, 1H), 1.65 (m, 13H). |
| 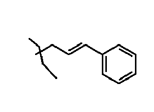 | (2E)-N-[(2E)-phenylprop-2-en-1-yl]-3-(1H-pyrrolo[2,3-b]pyridin-3-yl)acrylamide | 304.1444 | | C₁₉ H₁₇ N₃ O | 12.07 (bs, NH), 8.31 (m, 2H), 8.13 (t, J= 5.49 Hz, NH), 7.91 (d, J= 2.68 Hz, 1H), 7.60 (d, J= 15.85 Hz, 1H), 7.44 (m, 2H), 7.35 (m, 2H), 7.25 (m, 2H), 6.74 (d, J= 15.85 Hz, 1H), 6.56 (d, J= 15.97 Hz, 1H), 6.36 (dt J= 15.97, 5.85, 1H), 4.03 (m, 2H), |
| 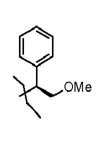 | (2E)-N-[(1S)-1-benzyl-2-methoxyethyl ]-3-(1H-pyrrolo[2,3-b]pyridin-3-yl)acrylamide | 336.1706 | 336.1708 | C₂₀ H₂₁ N₃ O₂ | 12.04 (bs, NH), 8.32 (m, 2H), 7.87 (m, 2H), 7.53 (d, J= 15.85Hz, 1H), 7.26 (m, 6H), 6.71 (d, J= 15.85 Hz, 1H), 4.23 (m, 1H), 3.30 (m, 5H), 2.87 (dd, J= 13.66, 6.22 Hz, 1H), 2.77(dd, J= 13.66, 6.22 Hz, 1H). |
| 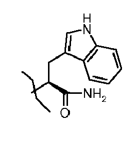 | N-α-[(2E)-3-(1H-pyrrolo[2,3-b]pyridin-3-yl)prop-2-enoyl]-L-tryptophanam ide | 374.1611 | 374.162 | C₂₁H₁₉N₅O₂ | 12.04 (bs, NH), 10.78 (s, NH), 8.34 (dd, J= 8.05, 1.59 Hz, 1H), 8.32 (dd, J= 4.76, 1.59 Hz, 1H), 8.03 (d, J= 8.43 Hz, 1H), 7.87 (bs, 1H), 7.52 (d, J= 15.85 Hz, 1H), 7.50 (s, 1H), 7.42 (bs, 2H), 7.31 (m, 2H), 7.20 (dd, J= 8.05, 4.76 Hz, 1H), 7.06 (m, 2H), 6.81 (d, J= 15.85 Hz, 1H), 4.70 (m, 1H), 3.19 (m, 1H), 3.00 (m, 1H). |
| 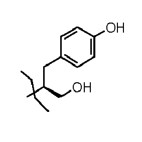 | (2E)-N-[(1S)-2-hydroxy-1-(4-hydroxybenzyl)ethyl]-3-(1H-pyrrolo[2,3-b]pyridin-3-yl)acrylamide | 338.1499 | 338.1502 | C₁₉ H₁₉ N₃ O₃ | 12.03 (bs, NH), 9.13 (bs, OH), 8.31 (m, 2H), 7.83 (bs, 1H), 7.72 (d, J= 8.29 Hz, 1H), 7.53 (d, J= 15.85 Hz, 1H), 7.22 (dd, J= 7.56, 5.00 Hz, 1H), 7.03 (m, 2H), 6.69 (d, J= 15.85 Hz, 1H), 6.66 (m, 2H), 4.80 (t, J= 5.49 Hz, OH), 3.98 (m, 1H), 3.34 (m, 2H), 2.60 (m, 2H). |
| 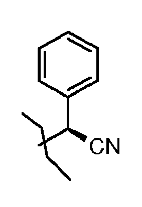 | (2E)-N-[cyano(pheny I)methyl]-3-(1H-pyrrolo[2,3-b]pyridin-3-yl)acrylamide | 303.124 | 303.1238 | C₁₈ H₁₄ N₄ O | 12.17 (bs, NH), 9.06 (d, J= 7.68 Hz, 1H), 8.32 (dd, J= 4.63, 1.46 Hz, 1H), 8.25 (dd, J= 7.93, 1.46 Hz, 1H), 7.99 (d, J= 2.80 Hz, 1H), 7.71 (d, J= 15.85 Hz, 1H), 7.51 (m, 5H), 7.22 (dd, J= 7.93, 4.63 Hz, 1H), 6.72 7.53 (d, J= 15.85 Hz, 1H), 6.28 (d, J= 7.68 Hz, 1H). |
| 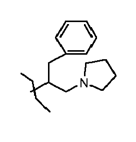 | (2E)-N-(1-benzyl-2-pyrrolidin-1-ylethyl)-3-(1H-pyrrolo[2,3-b]pyridin-3-yl)acrylamide | 375.2179 | 375.2186 | C₂₃H₂₆N₄O | 12.04 (bs, NH), 8.32 (dd, J= 4.36, 1.46 Hz, 1H), 8.28 (dd, 8.05, 1.46 Hz, 1H), 7.87 (s, 1H), 7.72 (d, J= 8.41 Hz, 1H), 7.49 (d, J= 15.85 Hz, 1H), 7.24 (m, 6H), 6.66 (d, J= 15.85 Hz, 1H), 4.20 (m, 1H), 2.91 (m, 1H), 2.73 (m, 1H), 2.51 (m, 6H), 1.69 (m, 4H). |
| 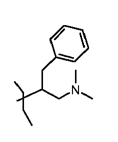 | (2E)-N-[1-benzyl-2-(dimethylamino)ethyl]-3-(1H-pyrrolo[2,3-b]pyridin-3-yl)acrylamide | 349.2023 | 349.2018 | C₂₁ H₂₄ N₄ O | 12.04 (bs, NH), 8.31 (dd, J= 4.76, 1.59 Hz, 1H), 8.27 (dd, J= 7.93, 1.59 Hz, 1H), 7.87 (s, 1H), 7.72 (d, J= 8.29 Hz, 1H), 7.49 (d, J= 15.85 Hz, 1H), 7.21 (m, 6H), 6.64 (d, J= 15.85 Hz, 1H), 4.21 (m, 1H), 2.91 (m, 1H), 2.69 (m, 1H), 2.33-2.15 (bm, 8H). |
| 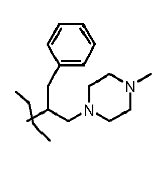 | (2*E*)-*N*-[1-benzyl-2-(4-methylpiperazin-1-yl)ethyl]-3-(1H-pyrrolo[2,3-b]pyridin-3-yl)acrylamide | 404.2445 | 404.2433 | C₂₃ H₂₉ N₅ O | 12.05 (bs, NH), 8.32 (dd, J= 4.76, 1.59 Hz, 1H), 8.27 (dd, J= 7.93, 1.59 Hz, 1H), 7.87 (s, 1H), 7.71 (d, J= 8.29 Hz, 1H), 7.51 (d, J= 15.85 Hz, 1H), 7.22 (m, 6H), 6.64 (d, J= 15.85 Hz, 1H), 4.25 (m, 1H), 2.93 (m, 1H), 2.68 (m, 1H), 2.33 (m, 8H), 2.15 (m, 5H). |
| 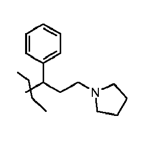 | (2E)-N-(1-phenyl-3-pyrrolidin-1-ylpropyl)-3-(1H-pyrrolo[2,3-b]pyridin-3-yl)acrylamide | 375.2179 | 375.2175 | C₂₃ H₂₆ N₄ O | 12.06 (bs, NH), 8.32 (m, 3H), 7.89 (d, J= 2.80 Hz, 1H), 7.53 (d, J= 15.85 Hz, 1H), 7.35 (m, 4H), 7.25 (m, 2H), 6.75 (d, J= 15.85 Hz, 1H), 5.00 (m, 1H), 2.44 (m, 6H), 1.91 (m, 2H), 1.69 (m, 4H). |
| 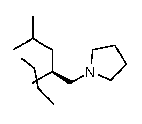 | (2E)-N-[(1S)-3-mthyl-1-(pyrrolidin-1-ylmethyl)butyl]-3-(1H-pyrrolo[2,3-b]pyridin-3-yl)acrylamide | 341.2336 | 341.233 | C₂₀ H₂₈ N₄ O | 8.31 (m, 2H), 7.87 (m, 1H), 7.6-7.51 (m, 2H, 7.22 (m, 1H) 2H), 7.22 (m,1H), 4.09 15.85 Hz, 2.51-2.39 (m, 6H), 1.72-1.59 (m, 5H), 1.42 (m, 1H), 1.31 (m, 1H), 0.9 (t, 6H). |
| 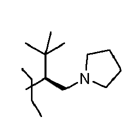 | (2E)-N-[(1S)-2,2-dimethyl-1-(pyrrolidin-1-ylmethyl)prop yl]-3-(1H-pyrrolo[2,3-b]pyridin-3-yl)acrylamide | 341.2336 | 341.2337 | C20 H28N4O | 12.03 (bs, 1H), 8.33 (m, 2H), 7.87 (m, 1H), 7.57-7.49 (m, 2H), 7.23 (m, 1H), 6.77 (d, J= 15.85 Hz, H), 3.9 (m, 1H), 2.58-2.31 (m, 6H), 1.68-1.59 (m, 4H), 0.91 (s, 9H). |
| 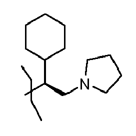 | (2E)-N-[(1S)-1-cyclohexyl-2-pyrrolidin-1-ylethyl]-3-(1H-pyrrolo[2,3-b]pyridin-3-yl)acrylamide | 313.2022 | 313.2026 | C18 H24 N4 O | 12.03 (bs, 1H), 8.32 (m, 2 H), 7.87 (m, 1H), 7.57-7.5 (m, 2H), 7.23 (m, 1H), 6.74 (d, J= 15.85 Hz, 1H), 3.96 (m, 1H), 2.58-2.35 (m, 6H), 1.87-0.93 (m, 15H). |
| 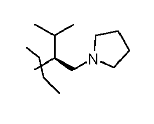 | (2E)-N-[(1S)-2-methyl-1-(pyrrolidin-1-ylmethyl)propyl]-3-(1H-pyrrolo[2,3-b]pyridin-3-yl)acrylamide | 327.2178 | | C19 H26 N4 O | 0.88 (d, J=6.83 Hz, 3 H), 0.88 (d, J=6.83 Hz, 3 H), 1.67 (bs, 4H), 1.87 (m, 1H), 2.53-2.41 (m, 6H), 3.98 (m, 1H), 6.75 (d, J=15.85 Hz, 1 H), 7.23 (dd, J=7.93, 4.76 Hz, 1 H), 7.52-7.58 (m, 2H), 7.88 (m, 1H), 8.33 (m, 2 H), 12.04 (bs, 1H). |
| 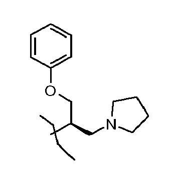 | (2E)-N-[(1R)-2-phenoxy-1-(pyrrolidin-1-ylmethyl)ethyl]-3-(1H-pyrrolo[2,3-b]pyridin-3-yl)acrylamide | 391.2128 | 391.2110 | C₂₃ H₂₆ N₄ O2 | 12.06 (bs, 1H), 8.32 (m, 2H), 7.96 (d, J=7.93 Hz, 1H), 7.9 (m, 1H), 7.59 (d, J= 15.85 Hz, 1H), 7.3 (m, 2H), 7.23 (m, 1H), 6.99 (m, 2H), 6.95 (m, 1H), 6.75 (d, J= 15.85 Hz, 1H), 4.34 (m, 1H), 4.06 (m, 2H), 2.79-2.48 (m, 6H), 1.7 (bs, 4H). |
| 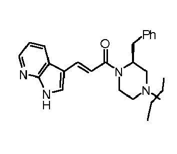 | 3-{(1E)-3-[(2S)-2-benzyl-4-methylpipera zin-1-yl]-3-oxoprop-1-en-1-yl}-1H-pyrrolo[2,3-b]pyridine | 361.2023 | 361.2027 | C₂₂ H₂₄ N₄ O | 12.04 (bs, NH), 8.32 (m, 2H), 7.87 (m, 1 H), 7.53 (d, J= 15.85 Hz, 1 H), 7.26 (m, 6H), 6.51 (d, J= 15.85 Hz, 1 H), 4.23 (m, 1 H), 3.30 (m, 1 H), 2.87 (m, 1 H), 2.77(m, 6H), 2.35 (bs, 3H). |

Example 3

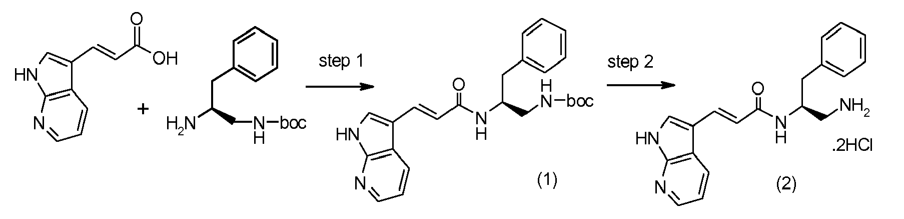

### (2E)-N-[(1S)-2-amino-1-benzylethyl]-3-(1H-pyrrolo[2,3-b]pyridin-3-yl)acrylamide

### Step 1

(E)-3-(1H-Pyrrolo[2,3-b]pyridin-3-yl)-acrylic acid (100mg, 0.52mmol) was dissolved in 5 ml of DCM and 1 ml of DMF, EDCI (110 mg, 0.57mmol, 1.1 eq.) and HOBt (77.80 mg, 0.57 mmol, 1.1 eq.) were added to the mixture at 0°C. The mixture was stirred at room temperature for 1 h, afterward the amine (S)-2-amino-3-phenyl-propyl)-carbamic acid tert-butyl ester (130 mg,0.52mmol, 1 eq.) was added. The mixture was stirred at room temperature for 18 h. Upon completion (monitor by TLC, by disappearance of acid). The solution was diluted with saturated aqueous solution of NaHCO3 (10mlx3), the organic solution was dried over Na₂SO₄ and evaporated, to afford a light yellow oil that was subjected to column chromatography (silica gel, 9:1 DCM:MeOH) to give 131 mg (60% yield) of [(S)-3-Phenyl-2-(E)-3-1H-pyrrolo[2,3-b]pyridin-3-yl-acryloylamino)-propyl]-carbamic acid tert-butyl ester (1) of a viscous oil.

1H NMR (400 MHz, DMSO-D6) δ ppm: 12.04 (bs, 1H), 8.31 (dd, J1 = 5.39 and J2= 1.80 Hz, 1H), 8.26 (dd, J1= 8.39 and J2= 1.80Hz, 1H), 7.87 (dd, J= 3.00 Hz, 1H), 7.07 (bd, J= 8.99 Hz, 1H), 7.50 (d, J= 16.78Hz, 1H), 7.26 (m, 6H), 6.85 (m, 1H), 6.59 (d, J= 16.78Hz, 1H), 4.14 (m, 1H), 3.09 (m, 2H), 2.83 (m, 1H), 2.69 (m, 1H), 1.39 (s, 9H).

MS (ESI) (m/z) : 421.22 (M+1, 100).

HRMS (ESI): 421.2242 (C24 H28 N4 O3 M+1; calc. 421.2234)

### Step 2

[(S)-3-Phenyl-2-(E)-3-1H-pyrrolo[2,3-b]pyridin-3-yl-acryloylamino)-propyl]-carbamic acid tert-butyl ester (1) was dissolved in 10 ml of dioxane and 5 ml of 4M HCl in dioxane were added slowly, after a while a off-white solid separates from the solution, the mixture was stirred at room temperature for 1 h. Upon completion (monitored by TLC by disappearance of starting material) solvent evaporation resulted 110 mg of a solid which was suspended with diethylether for 2 h at room temperature and filtered to afford 109 mg (98% yield) of the title compound (2) as off-white high hygroscopic solid.

1H NMR (400 MHz, DMSO-D6) δ ppm: 12.25 (bs, 1H), 8.36 (m, 2 H), 8.26 (d, J= 8.57 Hz), 8.07 (bs, 3H), 7.95 (d, J= 3.29 Hz, 1H), 7.59 (d, J= 16.46 Hz, 1H), 7.31 (m, 6 H), 6.69 (d, 16.46 Hz), 4.32 (m, 1H), 2.91 (m, 2H).

MS (ESI) (m/z) : 321.17 (M+1, 100).

HRMS (ESI): 321.1717 (C19 H20 N4 O . HCl, M+1; calc. 321.1710).

Analogously the following compounds have been obtained:

| R¹ | Name | EMASS CALC | EMASS FOUND | MOLFORMULA | 1H NMR (400 MHz, DMSO-D6) δ ppm |
|---|---|---|---|---|---|
| 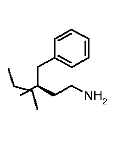 | (3S)-4-phenyl-3{[(2E)-3-(1H-pyrrolo[2,3-b]pyridin-3-yl)prop-2-enoyl]amino}butan-1-aminium chloride | 335.1866 | 335.1867 | C₂₀ H₂₂ N₄ O . HCl | 12.13 (bs, NH), 8.34 (dd, J= 4.76, 1.46 Hz, 1H), 8.28 (dd, J= 8.00, 1.46 Hz, 1H), 8.06 (d, J= 8.54 Hz, 1H), 7.91 (d, J= 2.68 Hz, 1H), 7.76 (b, 3H), 7.54 (d, J= 15.85 Hz, 1H), 7.29 (m, 6H), 6.68 (d, J= 15.85 Hz, 1H), 4.17 (m, 1H), 2.85 (m, 4H), 1.69 (m, 2H). |
| 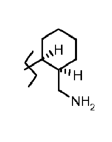 | [(1S,2S)-2-{[(2E)-3-(1H-pyrrolo[2,3-b]pyridin-3-yl)prop-2-enoyl]amino}cyclohexyl]m ethanaminiu m chloride | 298.3911 | | C₁₇ H₂₂ N₄ O HCl | 12.17 (bs, NH), 8.39 (dd, J= 8.11, 1.56 Hz, 1H), 8.34 (dd, J= 4.74, 1.56 Hz, 1H), 7.99 (d, J= 8.79 Hz, 1H), 7.94 (d, J= 2.80 Hz, 1H), 7.87 (b, 3H), 7.62 (d, J= 15.85 Hz, 1H), 7.26 (dd, J= 8.11, 4.74 Hz, 1H), 6.94 (d, J= 15.85 Hz, 1H), 4.20 (m, 1H), 1.92 (m, 1H), 1.29 (m, 8H). |
| 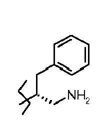 | (2R)-3-phenyl-2-{[(2E)-3-(1H-pyrrolo[2,3-b]pyridin-3-yl)prop-2-enoyl]amino}propan-1-aminium chloride | 321.171 | 321.1726 | C₁₉ H₂₀ N₄ O .2H Cl | 12.16 (bs, NH), 8.34 (dd, J= 4.81, 1.46 Hz, 1 H), 8.30 (dd, J= 8.00, 1.46 Hz, 1H), 8.13 (d, J= 8.29 Hz, 1H), 7.95 (m, 3H), 7.93 (d, J= 2.80 Hz, 1H), 7.56(d, J= 15.85Hz, 1H), 7.30 (m, 6H), 6.66 (d, J= 15.85 Hz, 1H), 4.32 (m, 1H), 3.20 (m, 2H), 2.96 (m, 4H). |
| 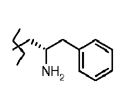 | (2S)-1-phenyl-3-{[(2E)-3-(1H-pyrrolo[2,3-b]pyridin-3-yl)prop-2-enoyl]amino}propan-2-aminium chloride | 322.1788 | 322.1781 | C₁₉ H₂₀ N₄ O . HCl | 12.06 (bs, NH), 8.33 (m, 3H), 8.06 (bs, 3H), 7.94 (d, J= 2.80 Hz, 1H), 7.62 (d, J= 15.85 Hz, 1H), 7.33 (m, 6H), 6.72 (d, J= 15.85 Hz, 1H), 3.40 (m, 3H), 2.95 (m, 2H). |
| 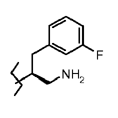 | (2E)-N-[(1S)-2-amino-1-(3-fluorobenzyl) ethyl]-3-(1H-pyrrolo[2,3-b]pyridin-3-yl)acrylamidedihydrochlori de | 339.1616 | 339.1614 | C₁₉ H₁₉ F N₄ O .2 Cl H | 8.33 (m, 1 H), 8.29 (m, 1H), 8.15 (d, J= 8.17 Hz, 1H), 8.0-7.9 (m, 3H), 7.58 (d, J= 15.85 Hz, 1H), 7.36 (m, 1H), 7.26 (m, 1H), 7.13 (m, 2H), 7.06 (m, 1H), 6.62 (d, J= 15.85 Hz, 1H), 4.33 (m, 1H), 3.05-2.8 (m, 4H). |
| 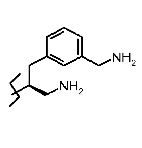 | (2E)-N-{(1S)-2-amino-l-[3-(aminomethyl )benzyl]ethyl}-3-(1H-pyrrolo[2,3-b]pyridin-3-yl)acrylamide trihydrochlori de | 350.1975 | 350.1992 | C₂₀ H₂₃ N₅ O . 3 HCl | 8.38-8.27 (m, 4H), 8.21 (d, J= 8.17 Hz, 1H), 8.01 (bs, 2H), 7.92 (d, J= 2.8 Hz, 1H), 7.58 (d, J= 15.85 Hz, 1H), 7.44-7.3 (m, 4H), 7.25 (m, 1H), 6.69 (d, J= 15.85 Hz, 1H), 4.36 (m, 1H), 4.0 (m, 2H), 3.03-2.82 (m, 4H). |
| 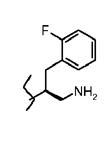 | (2E)-N-[(1S)-2-amino-1-(2-fluorobenzyl) ethyl]-3-(1H-pyrrolo[2,3-b]pyridin-3-yl)acrylamidedihydrochlori de | 339.1616 | 339.1628 | C₁₉ H₁₉ F N₄ O. 2HCl | 8.33 (m, 1 H), 8.28 (m, 1H), 8.1 (d, J= 8.41 Hz, 1H), 8.0-7.9 (m, 4H), 7.56 (d, J= 15.85 Hz, 1H), 7.4-7.1 (m, 5H), 6.59 (d, J= 15.85 Hz, 1H), 4.39 (m, 1H), 3.1-2.8 (m, 4H). |
| 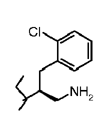 | (2E)-N-[(1S)-2-amino-1-(2-chlorobenzyl) ethyl]-3-(1H-pyrrolo[2,3-b]pyridin-3-yl)acrylamidedihydrochlori de | 355.132 | 355.1323 | C₁₉ H₁₉ Cl N₄ O . 2HCl | 8.3(m, 2H), 8.14 (m, 1H), 8.0-7.9 (m, 4H), 7.56 (d, J= 15.85 Hz, 1H), 7.48-7.23 (m, 5H), 6.6 (d, J= 15.85 Hz, 1H), 4.44 (m, 1H), 3.15-2.85 (m, 4H). |
| 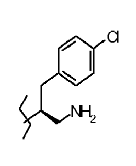 | (2E)-N-r(1S)-2-amino-1-(4-chlorobenzyl) ethyl]-3-(1H-pyrrolo[2,3-b]pyridin-3-yl)acrylamidedihydrochlori de | 355.132 | 355.1325 | C₁₉ H₁₉ Cl N₄ O . 2 HCl | 12.15 (bs, 1H), 8.33 (m, 1 H), 8.29 (m, 1H), 8.13 (d, J= 8.41 Hz, 1H), 8.01-7.89 (m, 4H), 7.57 (d, J= 15.85 Hz, 1H), 7.38 (m, 2H), 7.31 (m, 2H), 7.25 (m, 1H), 6.61 (d, J= 15.85 Hz, 1H), 4.3 (m, 1H), 3.05-2.75 (m, 4H). |
| 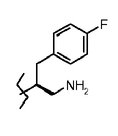 | (2E)-N-[(1S)-2-amino-1-(4-fluorobenzyl) ethyl]-3-(1H-pyrrolo[2,3-b]pyridin-3-yl)acrylamidedihydrochlori de | 339.1616 | 339.1616 | C₁₉ H₁₉ F N₄ O . 2 HCl | 12.15 (bs, 1H), 8.33 (m, 1 H), 8.29 (m, 1H), 8.12 (m, 1H), 8.01-7.87 (m, 4H), 7.57 (d, J= 15.85 Hz, 1H), 7.32 (m, 2H), 7.25 (m, 1H), 7.14 (m, 2H), 6.62 (d, J= 15.85 Hz, 1H), 4.28 (m, 1H), 3.05-2.75 (m, 4H). |
| 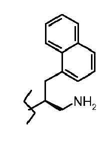 | (2E)-N-[(1S)-(2E)-N-[(1S)-2-amino-1-(1-naphthylmethyl)ethyl]-3-(1H-pyrrolo[2,3-b]pyridin-3-yl)acrylamide dihydrochlori de | 371.1866 | 371.1864 | C₂₃ H₂₂ N₄ O . 2 HCl | 12.24 (bs, 1H), 8.47-8.33 (m, 3H), 8.06 (bs, 2H), 7.95 (m, 2H), 7.83 (m, 1H), 7.65-7.42 (m, 6H), 7.28 (m, 1H), 6.7 (d, J= 15.85 Hz, 1H), 4.48 (m, 1H), 3.35 (m, 2H), 3.03 (m, 2H). |
| 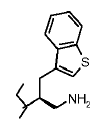 | (2E)-N-[(1S)-2-amino-1-(1-benzothien-3-ylmethyl)ethyl]-3-(1H-pyrrolo[2,3-b]pyridin-3-yl)acrylamide dihydrochlori de | 377.1431 | 377.1449 | C₂₁ H₂₀ N₄ O S . 2 HCl | 8.34 (m, 1H), 8.32-7.91 (m, 4H), 7.62 (d, J= 15.85 Hz, 1H), 7.56 (bs, 1H), 7.44 (m, 2H), 7.25 (m, 1H), 6.65 (d, J= 16.10 Hz, 1H), 4.47 (m, 1H), 3.2-2.94 (m, 4H). |
| 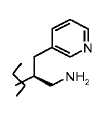 | (2E)-N-[(1S)-2-amino-1-(pyridin-3-ylmethyl)ethylpyrrolo[2,3-b]pyridin-3-yl)acrylamide trihydrochlori de | 322.1662 | 322.1661 | C₁₈ H₁₉ N₅ O . 3 HCI | 8.85 (bs, 1H), 8.75 (m, 1 H), 8.39 (m, 1H), 8.32 (m, 2H), 8.1 (bs, 3H), 7.91 (m, 2H), 7.52 (d, J= 15.85 Hz, 1H), 7.25 (m, 1H), 6.62 (d, J= 15.97 Hz, 1H), 4.4 (m, 1H), 3.4-2.9 (m, 4H). |
| 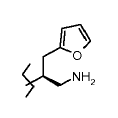 | (2E)-N-f(1S)-2-amino-1-(2-furylmethyl)et hyl]-3-(1H-pyrrolo[2,3-b]pyridin-3-yl)acrylamide dihydrochlori de | 311.1502 | 311.15 | C₁₇ H₁₈ N₄ O₂ . 2 HCl | 12.16 (bs, 1H), 8.33 (m, 1 H), 8.3 (m, 1H), 8.13 (m, 1H), 8.0-7.92 (m, 4H), 7.62 (d, J= 15.85 Hz, 1H), 7.58 (m, 1H), 7.25 (m, 1H), 6.64 (d, J= 15.85 Hz, 1H), 6.39 (m, 1H), 6.23 (m, 1H), 4.38 (m, 1H), 3.07-2.85 (m, 4H). |
| 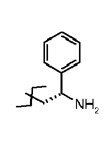 | (2E)-N-[(2S)-2-amino-2-phenylethyl]-3-(1H-pyrrolo[2,3-b]pyridin-3-yl)acrylamide | 307.1553 | 307.1553 | C₁₈ H₁₈ N₄ O | 12.05 (bs, 1 H), 8.31 (m, 2 H), 7.93-7.87 (m, 2H), 7.57 (d, J= 15.73 Hz, 1H), 7.41 (m, 2H), 7.34 (m, 2H), 7.27-7.2 (m, 2H), 6.7 (d, J= 15.83 Hz, 1H), 3.97 (m, 1H), 3.41 (m, 1H), 3.24 (m, 1H), 2.01 (bs, 2H). |
| 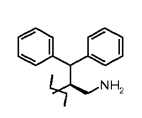 | (2E)-N-[(1S)-1-(aminomethyl)-2,2-diphenylethyl]-3-(1H-pyrrolo[2,3-b]pyridin-3-yl)acrylamidedihydrochlori de | 397.2023 | 397.2028 | C₂₅ H₂₄ N₄ O . 2 HCl HCI | 12.13 (bs, 1H), 8.31 (m, 1 H), 8.21 (m, 1H), 7.9 (m, 1H), 7.87 (bs, 2H), 7.53 (d, J= 15.97 Hz, 1H),7.45-7.09 (m, 11H), 6.47 (d, J= 15.85 Hz, 1H), 5.07 (m, 1H), 4.2 (d, J= 11.34, 1H), 2.81 (m, 1H), 2.7 (m, 1H). |
| 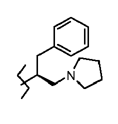 | (2E)-N-[(1S)-1-benzyl-2-pyrrolidin-1-ylethyl]-3-(1H-pyrrolo[2,3-b]pyridin-3-yl)acrylamide | 375.2179 | 375.2189 | C₂₃ H₂₆ N₄ O | 12.04 (bs, 1 H), 8.3(m,2H),7.88 (m, 1H), 7.72 (d, J= 8.41 Hz, 1 H), 7.51 (d, J= 15.85 Hz, 1 H), 7.28 (m, 2H), 7.25-7.21 (m, 3H), 7.18 (m, 1H), 6.64 (d, J= 15.85 Hz, 1H), 4.21 (m, 1 H), 2.94 (m, 1 H), 2.73 (m, 1H), 2.51-2.45 (m, 6H), 1.69 (m, 4H). |
| 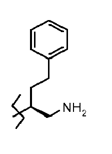 | (2E)-N-[(1S)-1-(aminomethyl)-3-phenylpropyl] -3-(1H- pyrrolo[2,3-b]pyridin-3-yl)acrylamide dihydrochlori de | 335.1866 | 335.1866 | C₂₀ H₂₂ N₄ O . 2 HCl | 12.16 (bs, 1H), 8.34 (m, 2H), 8.1 (d, J= 8.17 Hz, 1H), 7.95 (m, 1H), 7.89 (bs, 3H) 7.65 (d, J= 15.85 Hz, 1H), 7.35-7.16 (m, 5H), 6.74 (d, J= 15.97 Hz, 1H, 4.08 (m, 1H), 2.93 (m, 2H), 2.66 (m, 2H), 1.84 (m, 2H). |
| 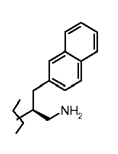 | (2E)-N-[(1S)-2-amino-1-(2-naphthylmeth yl)ethyl]-3-(1H-pyrrolo[2,3-b]pyridin-3-yl)acrylamidedihydrochloride | 371.1866 | 371.1856 | C₂₃ H₂₂ N₄ O . 2 HCl | 12.14 (bs, 1 H), 8.33 (m, 1 H), 8.28 (m, 1H), 8.02-7.82 (m, 6H), 7.79 (bs, 1H), 7.56 (d, J= 15.85 Hz, 1H), 7.52-7.44 (m, 3H), 7.25 (m, 1H), 6.63 (d, J= 15.85 Hz, 1H), 4.44 (m, 1H), 3.17-2.88 (m, 4H). |
| 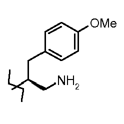 | (2E)-N-[(1S)-2-amino-1-(4-methoxybenz yl)ethyl]-3-(1 H- pyrrolo[2,3-b]pyridin-3-yl)acrylamide dihydrochlori de | 351.1815 | 351.1817 | C₂₀ H₂₂ N₄ O₂ . 2 HCl | 12.13 (bs, 1H), 8.32 (m, 1 H), 8.28 (m, 1H), 8.08 (d, J= 8.17 Hz, 1 H), 7.99-7.82 (m, 3H), 7.56 (d, J= 15.85 Hz 1 H), 7.24 (m, 1H), 7.18 (m, 2H), 6.87 (m, 2H), 6.63 (d, J= 15.85 Hz, 1H), 4.24 (m, 1H), 3.71 (s, 3H), 3.01-2.69 (m, 4H). |
| 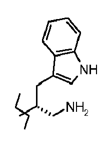 | (2E)-N-[(2R)-2-amino-3-(1H-indol-3-yl)propyl]-3-(1H-pyrrolo[2,3-b]pyridin-3-yl)acrylamide | 360.1819 | 360.1825 | C₂₁ H₂₁ N₅ O | 12.05 (bs, 1H), 10.83 (bs, 1H), 8.35-8.3 (m, 2H), 7.89 (m, 2H), 7.61-7.53 (m, 2H), 7.36 (m, 1H , 7.25-7.2 (m, 1H), 7.25-7.2 (m, 1H), 6.98 (m, 1H),6.75 (d, J= 15.85 Hz, 1H), 3.33 bs,1H 3.14-3.05 (m, 2H), 2.84 (m, 1H), 2.65 (m, 1H), 1.71 (bs, 2H). |
| 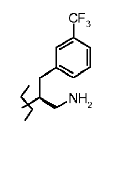 | (2E)-N-{(1S)-2-amino-1-[3-(trifluorometh yl)benzyl]ethyl}-3-(1H-pyrrolo[2,3-b]pyridin-3-yl)acrylamide dihydrochlori de | 389.1584 | 389.1574 | H₁₉ F₃ N₄ O C₂₀ H₁₉ F₃ N₄ O 2 HCl | 12.14(s, 1 H), 8.32(d, J=6.10Hz, 1 H), 8.27(d, J=7.68 Hz, 1H), 8.16(d, J=8.54 Hz, 1H), 7.96(bs, 2H), 7.92(m, 1H), 7.5-7.7(3m, 3H), 7.55(d, J=15.73 Hz, 725(dd Hz, 1H), 7.25(dd, J=4.76-8.05 Hz, 1H), 6.62(d, J=15.73 Hz, 1H), 4.32(m, 1H), 2.90-3.00(2m, 4H). |
| 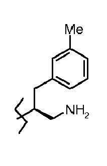 | (2E)-N-[(1S)-2-amino-1-(3-methylbenzyl )ethyl]-3-(1H-pyrrolo[2,3-b]pyridin-3-yl)acrylamide | 335.1866 | 335.1853 | C₂₀ H₂₂ N₄ O | 12.09(s, 1H), 8.31(2m, 2H), 7.87(s, 1H), 7.74(d, J=8.29 Hz, 1H), 7.53(d, J=15.97 Hz, 1H), 7.22(dd, J=4.76-7.93 Hz, 1H), 7.16(t, J=7.56 Hz, 1H), 6.95-7.08(3m, 3H), 6.69(d, J=15.85 Hz, 1H), 3.98(m, 1H), 2.28(s, 3H), 2.70-2.81(m, 2H), 2.58(m, 2H), 1.76(d, J=9.39 Hz, 2H). |
| 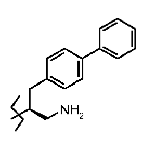 | (2E)-N-[(1S)-2-amino-1-(biphenyl-4-ylmethyl)ethyl]-3-(1H-pyrrolo[2,3-b]pyridin-3-yl)acrylamidedihydrochloride | 397.2023 | 397.2027 | C₂₅ H₂₄ N₄ O . 2 HCl | 12.15(s, 1H), 8.34(dd, J=1.34-4.64 Hz, 1H), 8.30(d, J=7.93 Hz, 1H), 8.18(d, J=8.17 Hz, 1H), 7.98(bs, 3H), 7.93(d, J=8.17 Hz, 1H), 4.35(m, 1H), 2.85-3.1(2m, 4H). |
| 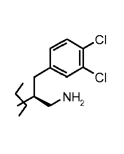 | (2E)-N-[(1S)-2-amino-1-(3,4-dichlorobenz yl)ethyl]-3-(1H-pyrrolo[2,3-b]pyridin-3-yl)acrylamide | 389.093 | 389.0932 | C₁₉ H₁₈ CI₂ N₄ O | 12.04(s, 1 H), 8.29(d, J= 3.66 Hz, 1H), 8.26(d, J=7.93 Hz, 1H), 7.86(s, 1H), 7.76(d, J=8.24 Hz, 1H), 7.45-7.55(3m, 3H), 7.20-7.25(2m, 2H), 6.64(d, J=15.87 Hz, 1H), 3.97(m, 1H), 2.91(dd, J=5.19-13.43 Hz, 1H), 2.68(dd, J=8.24-13.43 Hz, 1H), 2.58(d, J=5.80 Hz, 2H). |
| 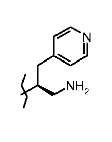 | (2E)-N-[(1S)-2-amino-1-(pyridin-4-ylmethyl)ethyl ]-3-(1H- pyrrolo[2,3-b]pyridin-3-yl)acrylamide dihydrochlori de | 322.1662 | 322.1677 | C₁₈ H₁₉ N₅ O . 2 HCl | 12.16(s, 1H), 8.79(2 m, 2H), 8.37(m, 1H), 8.33(d, J=3.97 Hz, 1H), 8.26(d, J=7.32 Hz, 1H), 8.09(bs, 3H), 7.80-7.95(2m 2H), 7.53(d, J=15.87 Hz, 1H), 7.24(dd, J=4.58-7.63 Hz, 1H), 6.60(d, J=15.87 Hz, 1H), 4.47(m, 1H), 3.07(2m, 4H). |
| 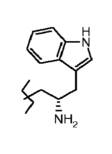 | (2E)-N-[(2S)-2-amino-3-(1H-indol-3-yl)propyl]-3-(1H-pyrrolo[2,3-b]pyridin-3-yl)acrylamide | 360.1819 | 360.1829 | C₂₁ H₂₁ N₅ O | 12.08(s, 1H), 10.92(s, 1H), 8.25-8.35(2m, 2H), 8.04(m, 1H), 7.91(s, 1H), 7.59(d, J=15.73 Hz, 1H), 7.37(d, J=8.05 Hz, 1H), 7.27(d, J2.07 Hz, 1H), 7.24(dd, J=5.00-7.68 Hz, 1H), 7.10(dd, J=4.76-12.93 Hz, 1H), 7.01(dd, J=5.85-12.93 Hz, 1H), 6.73(d, J=15.73 Hz, 1H), 3.34(several m, 5H). |
| 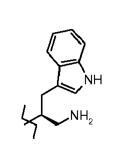 | (2E)-N-[(1S)-2-amino-1- (1H-indol-3-ylmethyl)ethyl]-3-(1H-pyrrolo[2,3-b]pyridin-3-yl)acrylamide | 360.1819 | 360.1823 | C₂₁ H₂₁ N₅ O | 10.84(s, 1H), 8.32(dd, J=1.22-4.88 Hz, 1H), 8.30(dd, J=1.22-7.93 Hz, 1H), 7.93(m, 1H), 7.66(d, J=7.80 Hz, 1H), 7.58(d, Hz, 1H), 7.58(d, J=15.85 Hz, 1H), 7.34(d, J=7.93 Hz, 1H), 7.24(m, 1H), 7.09(m, 1H), 6.70(d, J=15.85 Hz, 1H), 4.25(m, 1H), 2.7-3.0(several m, 4H). |

### Example 4

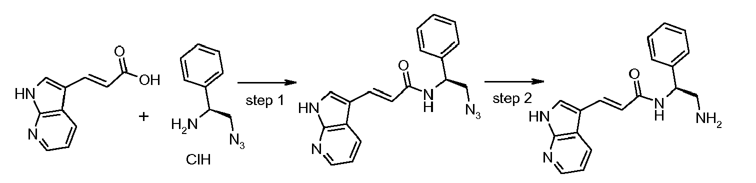

### (E)-N-(S)-2-Amino-1-phenyl-ethyl)-3-(1H-pyrrolo[2,3-b]pyridin-3-yl)-acrylamide

### Step 1

(E)-3-(1H-Pyrrolo[2,3-b]pyridin-3-yl)-acrylic acid (100mg 0.52mmol) was dissolved in 5 ml of DCM and 1 ml of DMF EDCI ( 110 mg, 0.57mmol, 1.1 eq.) and HOBT (77.80 mg, 0.57 mmol, 1.1 eq.), were added to the mixture at 0°C. The mixture was stirred at room temperature for 1 h, afterward the amine (S)-2-Azido-1-phenyl-ethylamine hydrochloride (113 mg, 0.57 mmol, 1.1 eq,) was added. The mixture was stirred at room temperature for 18 h. Upon completion (monitored by TLC, by disappearance of acid) the solution was diluted with saturated aqueous solution of NaHCO₃ (10mlx3), and portioned with ethyl acetate. The organic solution was separated, dried over Na₂SO₄ and evaporated, to afford a light yellow oil that was subjected to column chromatography (silica gel, 9:1 DCM: MeOH) to give 111 mg (64% yield ) of (E)-N-(S)-2-azido-1-phenyl-ethyl)-3-(1H-pyrrolo[2,3-b]pyridin-3-yl)-acrylamide of a light yellow oil.

1 H NMR (400 MHz, DMSO-D6) δ ppm: 12.09 (s, 1 H), 8.52 (d, J= 9.05 Hz, 1 H), 8.33 (d, J= 6.46 Hz, 1H), 7.92 (d, J= 3.01 Hz, 1H).

### Step 2

To a solution of 100 mg (0.30 mmol) (E)-N-((S)-2-azido-1-phenyl-ethyl)-3-(1H-pyrrolo[2,3-b]pyridin-3-yl)-acrylamide in 10 mL of ethanol, 65 mg (1.2 mmol) of NH₄CI dissolved in 3.2 mL of water and 39 mg of zinc (0.7 mmol) were added and the mixture heated at 50°C for 5 h. The suspension was cooled to room temperature and filtered. After removal of ethanol, solid Na₂CO₃ was added portionwise to the aqueous phase up to pH 10 and the product was then extracted with dichloromethane. Flash chromatography of the crude (eluant dichloromethane/methanol 95/5) yielded 92 mg of the title compound (100% yield).

¹H NMR (400 MHz, DMSO-d₆) δ ppm 12.06 (bs, NH), 8.33 (m, 3H), 7.89 (bs, 1H), 7.55 (d, J= 15.85 Hz, 1H), 7.35 (m, 4H), 7.23 (m, 2H), 6.81 (d, J= 15.85Hz, 1H), 4.93 (m, 1H), 2.87 (m, 2H).

MS (ESI) (m/z): 307.15 (M+1, 100).

HRMS (ESI): 307.1557 (C18 H18 N4 O, M+1; calc. 307.1553).

By working according to the above method the following compounds were prepared:

| R¹ | Name | EMASS CALC | EMASS FOUND | MOLFORMULA | 1 H NMR (400 MHz, DMSO-D6) δ ppm |
|---|---|---|---|---|---|
| 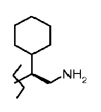 | (2E)-N-[(1S)-2-amino-1-cyclohexylethyl]-3-(1H-pyrrolo[2,3-b]pyridin-3-yl)acrylamide | 313.2023 | 313.2026 | C₁₈ H₂₄ N₄ O | 12.18 (bs, 1H), 8.3-8.36 (m, 2 H), 7.87 (bs, 1H), 7.58-7.5 (m, 2H), 7.22 (m, 1H), 6.76 (d, J= 15.85 Hz, 1H), 3.68 (m, 1H), 2.64 (m, 2H), 1.8-0.92 (m, 11H). |
| 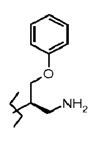 | (2E)-N-[(1R)-2-amino-1-(phenoxymethyl)ethyl]-3- (1H-pyrrolo[2,3-b]pyridin-3-yl)acrylamide | 337.1659 | 337.1667 | C₁₉ H₂₀ N₄ O₂ | 12.07 (bs, 1H), 8.36-8.29 (m, 2H), 7.94-7.88 (m, 2H), 7.6 (d, J= 15.85 Hz, 1H), 7.3 (m, 2H), 7.22 (m, 1H), 7.01 (m, 2H), 6.95 (m, 1H), 6.75 (d, J= 15.85 Hz, 1H), 4.18-4.0 (m, 3H), 2.81 (m, 2H), 1.85 (bs, 2H). |
| 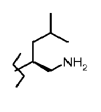 | (2E)-N-[(1S)-1-(aminomethyl)-3-methylbutyl]-pyrrolo[2,3-b]pyridin-3-yl)acrylamide | 287.1866 | 287.188 | C₁₆ H₂₂ N₄ O | 11.99 (bs, 1H), 8.31 (m, 2H), 7.88 (m, 1H), 7.6-7.52 (m, 2H), 7.22 (m, 7.52 (m, 2H), 7.22 (m, 1H), 6.69 (d, J= 15.85 Hz, 1H), 3.89 (m, 1H), 2.58 (m, 2H), 1.63 (m, 1H), 1.33 (m, 2H), 0.9 (m, 6H). (m, 6H). |
| 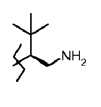 | (2E)-N-[(1S)-1-(aminomethyl)-2,2-dimethylpropyl]-3-(1H-pyrrolo[2,3-b]pyridin-3-yl)acrylamide | 287.1866 | 287.1873 | C₁₆ H₂₂ N₄ O | 12.05 (bs, 1H), 8.34 (dd, J=7.99, 1.52 Hz, 1 H), 8.30 (dd, J=4.69, 1.40 Hz, 1 H), 7.88 (bs, 1H), 7.55 (d, J=15.85 Hz, 1 H), 7.45 (d, J=9.88 Hz, 1 H), 7.22 (dd, J=7.86, 4.69 Hz, 1 H), 6.80 (d, J=15.85 Hz, 1 H), 3.67 (dt, J=9.97, 2.99 Hz, 1 H), 2.82 (dd, J=12.86, 2.99 Hz, 1 H), 2.43 (dd, J=12.80, 10.24 Hz, 1 H), 0.88 (s, 9H), |
| 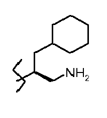 | (2E)-N-[(1S)-2-amino-1(cyclohexylmethyl)ethyl]-3-(1H-pyrrolo[2,3-b]pyridin-3-yl)acrylamide | 327.2179 | 327.2186 | C₁₉ H₂₆ N₄ O | 8.31 (m, 2 H), 7.87 (bs, 1H), 7.59-7.52 (m, 2H), 7.22 (m, 1 H), 6.68 (d, J= 15.85 Hz, 1H), 3.9 (m, 1H), 3.48-2.47 (m, 2H), 1.88-0.73 (m, 13H).13H). |
| 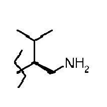 | (2E)-N-[(1S)-1-(aminomethyl)-2-methylpropyl]-3-(1H-pyrrolo[2,3-b]pyridin-3-yl)acrylamide | 273.171 | 273.1717 | C₁₅ H₂₀ N₄ O | 11.96 (bs, 1H), 8.32 (dd, J=7.99, 1.52 Hz, 1 H), 8.30 (dd, J=4.69, 1.40 Hz, 1H), 7.88 (bs, 1H), 7.54 (d, J=15.73 Hz, 1 H), 7.52 (d, Hz, 1 H), 7.52 (d, J=9.15 Hz, 1 H), 7.21 (dd, J=7.93, 4.76 Hz 1 H), 6.75 (d, J=15.85 Hz, 1H), 3.66 (m, 1H), 2.66 (dd, J=12.90, 4.80 Hz, 1 H), 2.56 (dd, J=12.90, 7.20 Hz, 1 H), 1.77 - 1.90 (m, J=6.70Hz, 1 H), 0.87 (d, J=6.83 Hz, 3 H), |

### Example 5

### tert-butyl [(2S)-2-amino-3-biphenyl-4-ylpropyl]carbamate

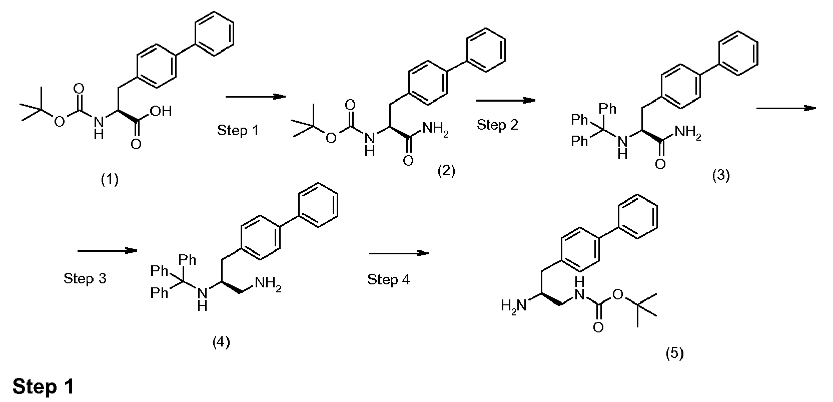

1g (2.9mmol) of commercial (2S)-3-biphenyl-4-yl-2-[(*tert-*butoxycarbonyl)amino]propanoic acid (1) was dissolved in a mixture of 30 ml of tetrahydrofuran and 30 ml of N,N'dimethylformamide. 1 ml (5.8 mmol) of N,N-diisopropyl-N-ethylamine, 1.1 g (5.8 mmol) of N-(3-dimethylaminopropyl)-N'-ethylcarbodiimide hydrochloride and 0.87 g (5.8 mmol) of N-hydroxy benzotriazole ammonium salt were added successively. The mixture was stirred at room temperature overnight. The solvent was then removed in vacuo, the residue redissolved with dichloromethane and washed with water. The organic layer was dried over sodium sulphate and evaporated. Trituration with diisopropylether gave 900 mg of *tert*-butyl [(1*S*)-2-amino-1-(biphenyl-4-ylmethyl)-2-oxoethyl]carbamate (2) (91 % yield).

### Step 2

900 mg (2.65 mmol) of (2) were dissolved in 20 ml of dichloromethane and 4 ml of trifluoroacetic acid were added. The solution was stirred overnight and then the solvent evaporated. The residue was redissolved with 15 ml of toluene and 5 ml of triethylamine and 900 mg of trityl chloride were added. The mixture was maintained at room temperature under stirring until the reaction was complete (5 h). The solvent was then removed, the residue redisolved with dichloromethane and washed with water. The organic layer was dried over sodium sulphate and evaporated to give 482 mg of (2*S*)-3-biphenyl-4-yl-2-(tritylamino)propanamide (3) (38 % yield).

### Step 3

482 mg (1 mmol) of (3) were dissolved in 20 ml of dry tetrahydrofuran and 7 ml of lithium aluminium hydride 1 M in THF were added dropwise at 0°C under nitrogen athmosphere. Then the mixture was refluxed for 7 h. After that time the reaction was cooled to 0°C and 11.5 ml of sodium hydroxide 2 N and 10 ml of tetrahydrofuran were added. The mixture was finally partitioned between dichloromethane and water giving, after drying with sodium sulphate and evaporation, 421 mg of (2S)-3-biphenyl-4-yl-N-tritylpropane-1,2-diamine (4) (90 % yield).

### Step 4

421 mg (0.9 mmol) of (4) were dissolved in a mixture of 14 ml of methanol and 6 ml of triethylamine. 212 mg (0.99 mmol) of ditertbutyldicarbonate were added at room temperature and the mixture stirred for 6 h. The solvent was then removed, the residue redissolved with 10 ml of dichloromethane, cooled to 0°C and then 2 ml of formic acid 98% were added. The reaction mixture was maintained 3 h at the same temperature under stirring.The solution was finally diluted with dichloromethane and washed several times with water. The aqueous layer was basified with solid sodium hydrogenocarbonate and extracted again with dicloromethane. The organic layer was dried over sodium sulphate and evaporated giving 250 mg of *tert*-butyl [(2S)-2-amino-3-biphenyl-4-ylpropyl]carbamate (5) (85 % yield).

1H NMR (400 MHz, DMSO-D6) δ ppm: 7.65(2m, 2H), 7.58(2m, 2H), 7.45(m, 2H), 7.29-7.39(5m, 3H), 6.80(t, J=4.76 Hz, 1H), 2.99(2m, 2H), 2,81(m, 1H), 2.68(dd, J=5.12-13.19Hz, 1H), 2.45(dd, J05.61-13.19 Hz, 1 H), 1.40(s, 9H).

MS (ESI) (m/z): 327 (M+1, 100).

HRMS (ESI): 327.2060 (C17 H15 N3 O M+H; calc 327.2067)

According to the same methodology the following derivatives may be prepared:

| Structure | Name | MS(ESI) (m/z): (M+1, 100) |
|---|---|---|
| 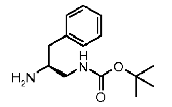 | *tert*-butyl [(2S)-2-amino-3-phenylpropyl]carbamate | 251.17 |
| 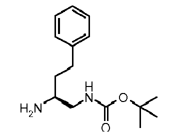 | *tert*-butyl [(2*S*)-2-amino-4-phenylbutyl]carbamate | 265.19 |
| 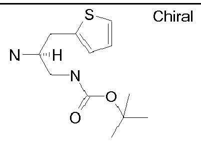 | *tert*-butyl[(2*S*)-2-amino-3-(2-thienyl)propyl]carbamate | 257.17 |
| 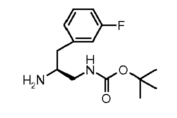 | *tert*-butyl[(2*S*)-2-amino-3-(3-fluorophenyl)propyl]carbamate | 269.16 |
| 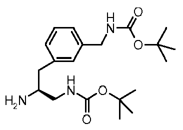 | *tert*-butyl[(2*S*)-2-amino-3-(3-{[(*tert*-butoxycarbonyl)amino]methyl}phenyl)propyl]carbamate | 380.25 |
| 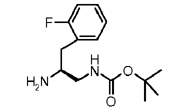 | *tert*-butyl[(2*S*)-2-amino-3-(2-fluorophenyl)propyl]carbamate | 269.16 |
| 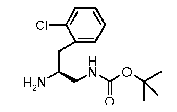 | *tert*-butyl[(2*S*)-2-amino-3-(2-chlorophenyl)propyl]carbamate | 285.13 |
| 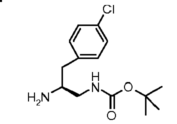 | *tert*-butyl[(2*S*)-2-amino-3-(4-chlorophenyl)propyl]carbamate | 285.13 |
| 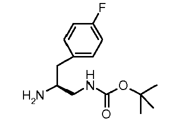 | *tert*-butyl[(2*S*)-2-amino-3-(4-fluorophenyl)propyl]carbamate | 269.16 |
| 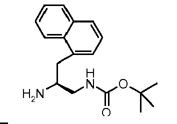 | *tert*-butyl[(2*S*)-2-amino-3-(1-naphthyl)propyl]carbamate | 301.19 |
| 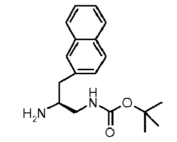 | *tert*-butyl[(2*S*)-2-amino-3-(2-naphthyl)propyl]carbamate | 301.19 |
| 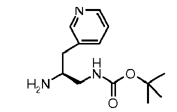 | *tert*-butyl[(2*S*)-2-amino-3-pyridin-3-ylpropyl]carbamate | 252.17 |
| 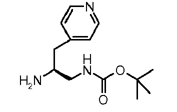 | *tert*-butyl[(2*S*)-2-amino-3-pyridin-4-ylpropyl]carbamate | 252.33 |
| 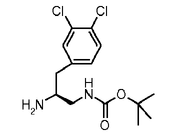 | *tert*-butyl[(2*S*)-2-amino-3-(3,4-dichlorophenyl)propyl]carbamate | 319.23 |
| 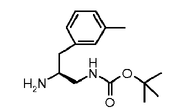 | *tert*-butyl[(2*S*)-2-amino-3-(3-methylphenyl)propyl]carbamate | 265.37 |
| 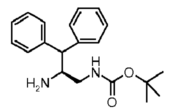 | *tert*-butyl [(2*S*)-2-amino-3,3-diphenylpropyl]carbamate | 327.20 |
| 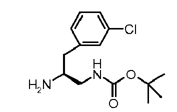 | tert-butyl[(2S)-2-amino-3-(3-chlorophenyl)propyl]carbamate | 285.13 |
| 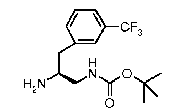 | *tert*-butyl{(2*S*)-2-amino-3-[3-(trifluoromethyl)phenyl]propyl}carbamate | 319.34 |
| 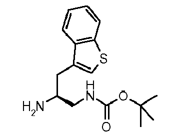 | *tert*-butyl[(2*S*)-2-amino-3-(1-benzothien-3-yl)propyl]carbamate | 307.14 |
| 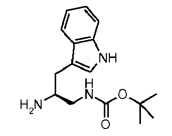 | *tert*-butyl[(2*S*)-2-amino-3-(1 *H*-indol-3-yl)propyl]carbamate | 290.18 |
| 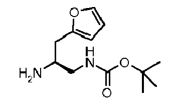 | *tert*-butyl [(2*S*)-2-amino-3-(2-furyl)propyl]carbamate | 241.15 |
| 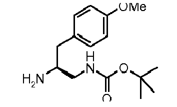 | *tert*-butyl[(2*S*)-2-amino-3-(4-methoxyphenyl)propyl]carbamate | 281.18 |
| 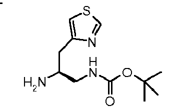 | *tert*-butyl[(2*S*)-2-amino-3-(1,3-thiazol-4-yl)propyl]carbamate | 258.36 |

### Example 6

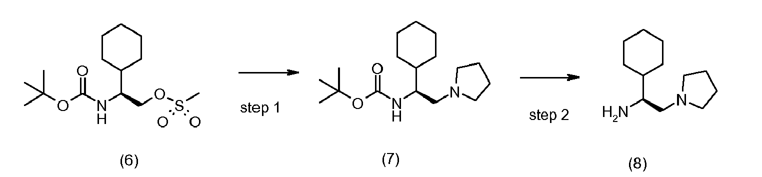

### (1S)-1-cyclohexyl-2-pyrrolidin-1-ylethanamine

### Step 1

2 g (6.22 mmol) of (2S)-2[(tert-butoxycarbonyl)amino]-2-cyclohexylethyl methanesulfonate (6) were dissolved in 8 ml of dry tetrahydrofuran and 5 ml (62 mmol) of pyrrolidine were added. The solution was refluxed under stirring for 6 h. The solution was then cooled and 80 ml of diethylether were added to precipitate pirrolidinium salts that were removed by filtration. The filtrate was evaporated to give a crude that was purified by flash-chromatography (eluent: dichloromethane-methanol-ammonium hydrate 30% 97-3-0.3) to yield 1.42 g of a tert-butyl[(1S)-1-cyclohexyl-2-pyrrolidin-1-ylethyl]carbamate (7) (73 % yield).

### Step 2

1.42 g (4.8 mmol) of (7) were dissolved in 10 ml of dichloromethane and 10 ml of HCI 4 M in dioxane were added dropwise. The solution was stirred at room temperature for 3 h and then the solvent evaporated. The residue was triturated with diethylether, giving 1.35 g of (1S)-1-cyclohexyl-2-pyrrolidin-1-ylethanamine di hydrochloride (8) (100% yield).

1H NMR (400 MHz, DMSO-D6) δ ppm:10.87(bs, 1H), 8.40(bs, 1H), 3.58(2m, 2H), 3.08(2m, 2H), 3.45(m, 3H), 1.8-2.1(2m, 4H), 1.6-1.8(2m, 5H), 1.18(m, 6H).

MS (ESI) (m/z) : 197.20 (M+1, 100).

According to the same methodology the following derivatives may be prepared:

| Structure | Name | MS(ESI) (m/z): (M+1, 100) |
|---|---|---|
| 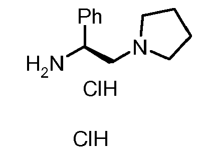 | (1*S*)-1-phenyl-2-pyrrolidin-1-ylethanamine dihydrochloride | 191.15 |
| 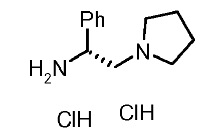 | (1*R*)-1-phenyl-2-pyrrolidin-1-ylethanamine dihydrochloride | 191.15 |
| 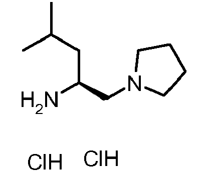 | (2*S*)-4-methyl-1-pyrrolidin-1-ylpentan-2-am ine dihydrochloride | 171.18 |
| 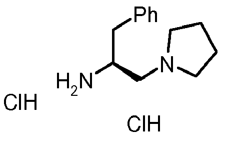 | (2*S*)-1-phenyl-3-pyrrolidin-1-ylpropan-2-amine dihydrochloride | 205.16 |
| 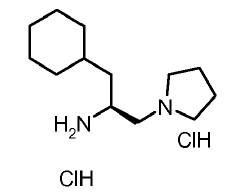 | (2*S*)-1-cyclohexyl-3-pyrrolidin-1-ylpropan-2-amine dihydrochloride | 211.21 |
| 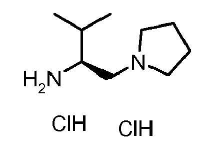 | (2*S*)-3-methyl-1-pyrrolidin-1-ylbutan-2-amine dihydrochloride | 157.16 |
| 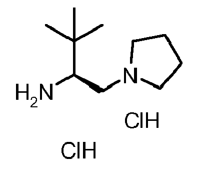 | (2*S*)-3,3-dimethyl-1-pyrrolidin-1-ylbutan-2-amine dihydrochloride | 171.30 |
| 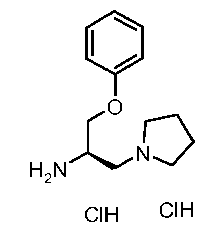 | (1R)-2-phenoxy-1-(pyrrolidin-1-ylmethyl)ethanamine dihydrochloride | 221.16 |

### Example 7

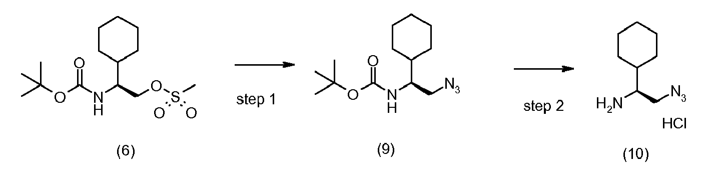

### (1S)-2-azido-1-cyclohexylethanamine

### Step 1

2 g (6.22 mmol) of (2S)-2[(tert-butoxycarbonyl)amino]-2-cyclohexylethyl methanesulfonate (6) were dissolved in 8 ml of dry DMF and 1.21 g (18.6 mmol) of sodium azide were added. After heating at 80°C for 3 h, the solution was cooled, water (50 ml) was added and the mixture was extracted with EtOAc (50 ml). The organic layer was washed with brine (50 ml), dried (MgSO₄) and concentrated. The residue was purified by flash chromatography (eluent: cyclohexane / EtOAc = 8:2) to furnish a light yellow oil (833 mg, 50%).

1 H NMR (400 MHz, DMSO-D6) δ ppm 6.84 (d, J= 9.15 Hz, 1 H), 4.32 (m, 1 H), 3.55 (m, 1 H), 3.41 (m, 2H), 1.60-0.90 (m, 19H).

MS (ESI) (m/z) : 269.19 (M+1, 100)

### Step 2

0.833 g (3.1 mmol) of (9) were dissolved in 10 ml of dichloromethane and 10 ml of HCI 4 M in dioxane were added dropwise. The solution was stirred at room temperature for 3 h and then the solvent evaporated. The residue was triturated with diethylether, giving 0.634 g of (S)-2-Azido-1-cyclohexyl-ethylamine hydrochloride (9) (100% yield).

1 H NMR (400 MHz, DMSO-D6) δ ppm:7.98(bs, 3H), 3.73 (dd, J= 13.41, 3.78 Hz, 1 H), 3.63(dd, J= 13.41, 6.71 Hz, 1H), 3.06(m, 1 H), 1.71-1.00(m, 11 H).

MS (ESI) (m/z) : 170.14 (M+1, 100)

Reported below are the analytical HPLC/Mass data for some compounds.

| Structure | Name | MS(ESI) (m/z): (M+1, 100) |
|---|---|---|
| 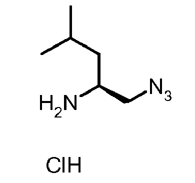 | (2*S*)-1-azido-4-methylpentan-2-amine hydrochloride | 144.12 |
| 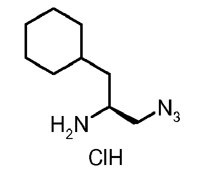 | (2*S*)-1-azido-3-cyclohexylpropan-2-amine hydrochloride | 184.16 |
| 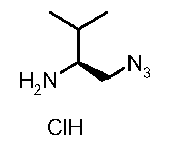 | (2*S*)-1-azido-3-methylbutan-2-amine hydrochloride | 130.11 |
| 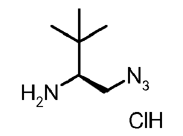 | (2*S*)-1-azido-3,3-dimethylbutan-2-amine hydrochloride | 144.12 |
| 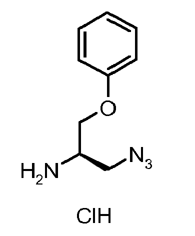 | (R)-2-Azido-1-phenoxymethyl-ethylamine hydrochloride | 194.10 |

The employed starting materials were prepared from the corresponding commercially available aminoalcohols as described in Tetrahedron Asymmetry (1996), 7(7), 2145-2150

### Example 8

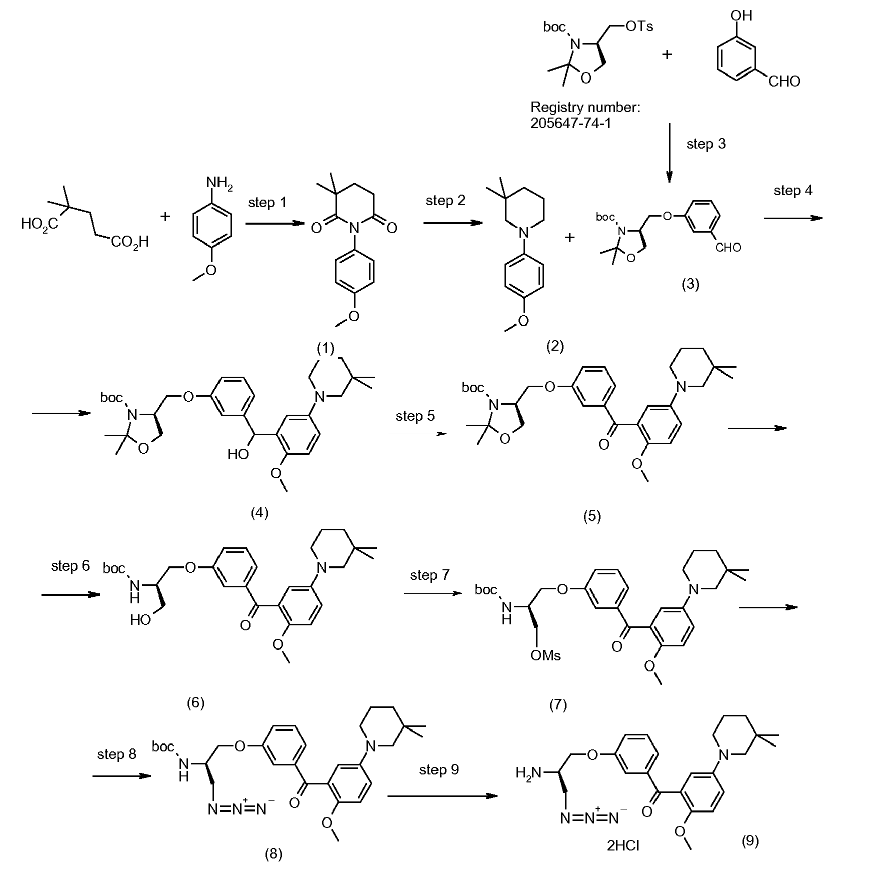

### [3-(( R )-2-Amino-3-azido-propoxy)-phenyl]-[5-(3,3-dimethyl-piperidin-1-yl)-2-methoxy-phenyl]-methanone dihydrochloride

### Step 1

2,2-Dimethylglutaric acid (15.0 g, 93 mmol) mixed with SOCI₂ (70 ml) was refluxed for 5 h, under stirring. Then the excess of SOCI₂ was removed under reduced pressure. To a solution of the acyl chloride in 1,2-dimetoxy-ethane (40 ml), a solution of p-methoxy-aniline (11.5 g, 93 mmol) was added dropwise in the same solvent (50 ml) and the crude mixture was refluxed for 15 h, under stirring. Then the solvent was evaporated in vacuo and the crude residue was heated for 8 h at 230°C. The residue was chromatographed (hexane/ethyl acetate 7:3, as eluent) to give 16.3g 1-(4-methoxy-phenyl)-3,3-dimethyl-piperidine-2,6-dione (1) as a beige solid (71% yield).

### Step 2

A solution of 1-(4-methoxy-phenyl)-3,3-dimethyl-piperidine-2,6-dione (1) (15.7 g, 63 mmol) in anhydrous THF (150 ml) was added dropwise to a cooled 1 M solution of LiAIH₄ in THF (190 ml, 190 mmol). The mixture was refluxed for 7 h, under stirring. The reaction mixture was cooled, excess LiAlH₄ decomposed by careful addition of water (100 ml) and stirring was continued for 1 h more. The mixture was then filtered through Celite and the filtrate concentrated to dryness. The residue was taken up with CH₂Cl₂. This solution was washed with water, dried over Na₂SO₄ and concentrated under reduced pressure. The residue was chromatographed (cyclohexane/ethyl acetate 98:2, as eluent) to give 1-(4-methoxy-phenyl)-3,3-dimethyl-piperidine (2) as a yellow oil (9.2 g, 66% yield).

1H NMR (400 MHz, DMSO-D6) δ ppm: 6.86 (m, 2H); 6.8 (m, 2H), 3.68 (s, 3H), 2.9 (m, 2H), 2.65 (m, 2H), 1.66 (m, 2H), 1.3 (m, 2H), 0.98 (s, 6H).

MS (ESI) (m/z): 220.17 (M+1, 100).

### Step 3

A suspension of (R)-2,2-dimethyl-4-(toluene-4-sulfonyloxymethyl)-oxazolidine-3-carboxylic acid tert-butyl ester (17.5 g, 45 mmol), 3-hydroxybenzaldeyde (6.8 g, 54 mmol) and Cs₂CO₃ (18.5 g, 57 mmol) in DMF (800 ml) was heated to 80°C for 7 h under nitrogen. The reaction was then diluted with EtOAc (2 I), quenched with water (1 I), and washed with 10% aqueous NH₄Cl. The organic layers were dried (MgSO₄) and the solvent removed in vacuo to afford a residue that was purified by flash chromatography, using 90% cyclohexane-10% EtOAc as eluent, to afford 10.1 g (66% yield) of (S)-4-(3-formyl-phenoxymethyl)-2,2-dimethyloxazolidine-3-carboxylic acid tert-butyl ester (3) as an oil.

1H NMR (400 MHz, DMSO-D6) δ ppm: 9.99 (s, 1H); 7.6-7.3 (m, 4H), 4.25-3.93 (m, 5H), 1.57-1.37 (m, 15H).

MS (ESI) (m/z): 336, (M+1), 353 (M+18).

### Step 4

sec-BuLi solution (1.4 M in cyclohexane, 22 ml, 30.8 mmol) was added to a solution of 1-(4-methoxy-phenyl)-3,3-dimethyl-piperidine (2) (3.3 g, 15 mmol) in anhydrous THF (50 ml) at -70°C. The solution was stirred for 1 h at -70°C and then (S)-4-(3-formyl-phenoxymethyl)-2,2-dimethyl-oxazolidine-3-carboxylic acid tert-butyl ester (3) (10 g, 30 mmol) in anhydrous THF (30 ml) was added dropwise at -70°C. The mixture was stirred at the same temperature for 3 h 30' then warmed to r.t. and stirred for additional 1 h 30'. Water (30 ml) and EtOAc (30 ml) were added, and the separated aqueous layer was re-extracted with EtOAc (2 x 60 ml). The combined organic layers were washed with brine and dried over Na₂SO₄. After filtration and evaporation, the residue was purified by flash chromatography (eluent:Cyclohexane/EtOAc = 9:1) to yield (S)-4-(3-{[5-(3,3-dimethyl-piperidin-1-yl)-2-methoxy-phenyl]-hydroxy-methyl}-phenoxymethyl)-2,2-dimethyl-oxazolidine -3-carboxylic acid tert-butyl ester (4) as a yellow oil (6.6 g, 79% yield).

1H NMR (400 MHz, DMSO-D6) δ ppm: 7.16 (m, 1H); 7.07 (bs, 1H), 6.9 (bs, 2H), 6.82-6.77 (m, 2H), 6.75-6.71 (m, 1H), 5.895 (d, 1H, J = 4.51 Hz), 5.62-5.59 (m, 1H), 4.2-3.75 (m, 5H), 3.68 (s, 3H), 2.88 (m, 2H), 2.63 (bs, 2H), 1.64 (m, 2H), 1.51 (bs, 3H), 1.44 (bs, 9H), 1.38 (bs, 3H), 1.3-1.26 (m, 2H), 0.97 (s, 6H).

MS (ESI) (m/z) : 555.34 (M+1, 100).

HRMS (ESI): 555.3411 (C32 H46 N2 06, M+1; calc. 555.3429).

### Step 5

Tetrapropylamonium perruthenate (TPAP) (30 mg, 0.08 mmol) was added to a stirring solution of (S)-4-(3-{[5-(3,3-dimethyl-piperidin-1-yl)-2-methoxy-phenyl]-hydroxy-methyl}-phenoxymethyl)-2,2-dimethyl-oxazolidine-3-carboxylic acid tert-butyl ester (4) (460 mg, 0.83 mmol), N-methylmorpholine N-oxide (175 mg, 1.25 mmol) and molecular sieves (460 mg) in DCM (6 ml). The mixture was stirred at r.t. under an atmosphere of argon for 1h. Et₂O was added (15 ml) and the mixture vigorously stirred for 10 min. The mixture was allowed to stand for 5 min then filtered and concentrated. The residue was purified by flash chromatography on silica gel (eluent: Cyclohexane / EtOAc = 9:1) to yield (S)-4-{3-[5-(3,3-Dimethyl-piperidin-1-yl)-2-methoxy-benzoyl]-phenoxymethyl}-2,2-dimethyl-oxazolidine-3-carboxylic acid tert-butyl ester (5) as a yellow oil that solidified on standing (385 mg, 84% yield).

1H NMR (400 MHz, DMSO-D6) δ ppm: 7.43 (m, 1H); 7.3-7.2 (m, 3H), 7.11-7.05 (m, 1H), 7.05-7.01 (m, 1H), 6.82 (m, 1H), 4.2-3.88 (m, 5H), 3.58 (s, 3H), 2.95 (m, 2H), 2.70 (bs, 2H), 1.64 (m, 2H), 1.5 (bs, 3H), 1.44 (bs, 9H), 1.38 (bs, 3H), 1.29 (m, 2H), 0.96 (s, 6H).

MS (ESI) (m/z): 553.33 (M+1, 100).

HRMS (ESI): 553.3270 (C32 H44 N2 06, M+1; calc. 553.3272).

### Step 6

To a solution of (S)-4-{3-[5-(3,3-dimethyl-piperidin-1-yl)-2-methoxy-benzoyl]-phenoxymethyl}-2,2-dimethyl-oxazolidine-3-carboxylic acid tert-butyl ester (5) (495 mg, 0.9 mmol) in MeOH (8 ml) at r.t., p-toluensulfonic acid (205 mg, 1.08 mmol) was added. The mixture was stirred for 20 h then cooled and hydrolyzed by a saturated aqueous solution of NaHCO₃ until pH 9. After removing of MeOH under reduced pressure, the aqueous layer was extracted by AcOEt. The organic layer was washed with brine, dried over MgSO₄, filtered and concentrated under reduced pressure. The residue was purified by flash chromatography (eluent: cyclohexane / AcOEt = 7:3) to furnish ((R)-1-{3-[5-(3,3-Dimethyl-piperidin-1-yl)-2-methoxy-benzoyl]-phenoxymethyl}-2-hydroxyethyl)-carbamic acid tert-butyl ester (6) as a yellow oil (355 mg, 77% yield).

1H NMR (400 MHz, DMSO-D6) δ ppm: 7.44-7.38 (m, 2H); 7.25-7.19 (m, 2H), 7.1-7.05 (m, 1H), 7.05-7.01 (m, 1H), 6.815 (m, 1H), 6.73 (d. 1H, J = 8.41 Hz), 4.77 (t, 1H, J = 5.61 Hz), 4.06-3.91 (m, 2H), 3.77 (m, 1H), 3.59 (s, 3H), 3.445 (m, 2H), 2.95 (m, 2H), 2.71 (bs, 2H), 1.64 (m, 2H), 1.37 (s, 9H), 1.29 (m, 2H), 0.96 (s, 6H).

MS (ESI) (m/z) : 513.3 (M+1, 100).

HRMS (ESI): 513.2950 (C29 H40 N2 06, M+1; calc. 513.2959).

### Step 7

Mesylchloride (180 µl, 2.32 mmol) was added dropwise to a solution of ((R)-1-{3-[5-(3,3-Dimethyl-piperidin-1-yl)-2-methoxy-benzoyl]-phenoxymethyl}-2-hydroxyethyl)-carbamic acid tert-butyl ester (6) (900 mg, 1.76 mmol) and TEA (295 µl, 2.12 mmol) in DCM (8 ml) at - 10°C. The solution was stirred at 0°C under an atmosphere of argon for 4h. The reaction mixture was poured into ice/water and the separated aqueous layer was re-extracted with DCM (5 ml). The combined organic layers were washed with 0.5N HCl, water, saturated aqueous NaHCO₃ sol and brine, dried (MgSO₄) and the solvent was removed in vacuo to afford methanesulfonic acid (S)-2-tert-butoxycarbonylamino-3-{3-[5-(3,3-dimethyl-piperidin-1-yl)-2-methoxy-benzoyl]-phenoxyl-propyl ester (7) as an orange oil (1 g) that was used in the next step without purification.

### Step 8

Methanesulfonic acid (S)-2-tert-butoxycarbonylamino-3-{3-[5-(3,3-dimethyl-piperidin-1-yl)-2-methoxy-benzoyl]-phenoxy}-propyl ester (1 g, 1.7 mmol) was dissolved in DMF (10 ml) and NaN₃ was added (180 mg, 2.7 mmol). After heating at 80°C for 3 h, the solution was cooled, water (50 ml) was added and the mixture was extracted with EtOAc (50 ml). The organic layer was washed with brine (50 ml), dried (MgSO₄) and concentrated. The residue was purified by flash chromatography (eluent: cyclohexane / EtOAc = 8:2) to furnish ((R)-2-azido-1-{3-[5-(3,3-dimethyl-piperidin-1-yl)-2-methoxy-benzoyl]-phenoxymethyl}-ethyl)-carbamic acid tert-butyl ester as a yellow oil that solidified on standing (450 mg, 49% yield).

1 H NMR (400 MHz, DMSO-D6) δ ppm: 7.42 (m, 1 H), 7.27-7.19 (m, 3H), 7.17 (d. 1 H, J = 7.56 Hz), 7.1-7.05 (m, 1H), 7.05-7.01 (m, 1H), 6.815 (m, 1H), 4.06-3.89 (m, 3H), 3.59 (s, 3H), 3.455 (d, 2H, J = 4.76 Hz), 2.95 (m, 2H), 2.71 (bs, 2H), 1.64 (m, 2H), 1.38 (s, 9H), 1.29 (m, 2H), 0.96 (s, 6H).

MS (ESI) (m/z) : 538.3 (M+1, 100).

HRMS (ESI): 538.3014 (C29 H39 N5 05, M+1; calc. 538.3024).

### Step 9

HCI solution (4 M in dioxane, 1.6 ml, 6.4 mmol) was added to a solution of ((R)-2-azido-1-{3-[5-(3,3-dimethyl-piperidin-1-yl)-2-methoxy-benzoyl]-phenoxymethyl}-ethyl)-carbamic acid tert-butyl ester (8) (430 mg, 0.8 mmol) in anhydrous DCM (3 ml) at r.t.. The solution was stirred for 3 h and then concentrated. The residue was triturated in Et₂O filtered and dried in vacuo to afford the title compound (9) as a yellow solid (410 mg) (100% yield).

1H NMR (400 MHz, DMSO-D6) δ ppm: 8.6-8.3 (m, 4H), 7.5-6.8 (m, 7H), 4.3-4.12 (m, 2H), 3.87-3.67 (m, 3H), 3.57 (s, 3H), 3.2-2.68 (m, 4H), 1.72 (bs, 2H), 1.35 (bs, 2H), 1.0 (s, 6H).

MS (ESI) (m/z): 438.25 (M+1, 100).

HRMS (ESI): 438.2508 (C24 H31 N5 03, M+1; calc. 438.2500).

### Example 9

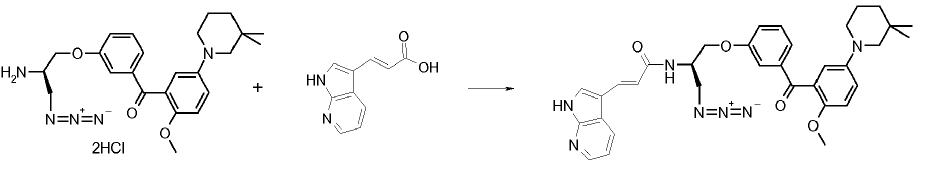

### (E)-N-(( R )-2-Azido-1-{3-[5-(3,3-dimethyl-piperidin-1-yl)-2-methoxy-benzoyl]-phenoxymethyl}-ethyl)-3-(1H-pyrrolo[2,3-b]pyridin-3-yl)-acrylamide

(E)-3-(1H-Pyrrolo[2,3-b]pyridin-3-yl)-acrylic acid (150 mg 0.8 mmol) was dissolved in DCM (14 ml) and DMF (7 ml), DIPEA (560 µl, 3.2 mmol), EDCI (170 mg, 0.88 mmol) and HOBt (120 mg, 0.88 mmol) were added to the mixture at 0°C. The mixture was stirred at 0°C for 30', afterward the amine [3-((R)-2-amino-3-azido-propoxy)-phenyl]-[5-(3,3-dimethyl-piperidin-1-yl)-2-methoxy-phenyl]-methanone di-hydrochloride (410 mg, 0.8 mmol) was added. The mixture was stirred at room temperature for 18 h. The solution was washed with saturated aqueous solution of NaHCO₃ and brine, dried over Na₂SO₄ and evaporated. The residue was subjected to flash chromatography (eluent: DCM / MeOH = 98:2) to give a yellow solid (280 mg, 57% yield)

1H NMR (400 MHz, DMSO-D6) δ ppm: 8.30 (dd, J=4.76, 1.40 Hz, 1 H), 8.28 (dd, J=8.00, 1.40 Hz, 1 H), 8.21 (d, J=8.05 Hz, 1 H), 7.91 (d, 1H, J = 2.68 Hz), 7.61 (d, 1H, J = 15.85 Hz), 7.44 (m, 1H), 7.31-7.24 (m, 3H), 7.22 (dd, J=7.93, 4.76 Hz, 1 H), 7.1-7.05 (m, 1H), 7.05-7.01 (m, 1H), 6.825 (m, 1H), 6.71 (d, 1H, J = 15.85 Hz), 4.39 (m, 1 H), 4.14 (dd, J = 9.88, 5.12 Hz, 1H), 4.065 (dd, J = 9.88, 5.37 Hz, 1H), 3.62 (m, 2H), 3.58 (s, 3H), 2.94 (m, 2H), 2.70 (bs, 2H), 1.63 (m, 2H), 1.27 (m, 2H), 0.95 (s, 6H).

MS (ESI) (m/z) : 608.3 (M+1, 100).

HRMS (ESI): 608.2991 (C34 H37 N7 04, M+1; calc. 608.2980).

### Example 10

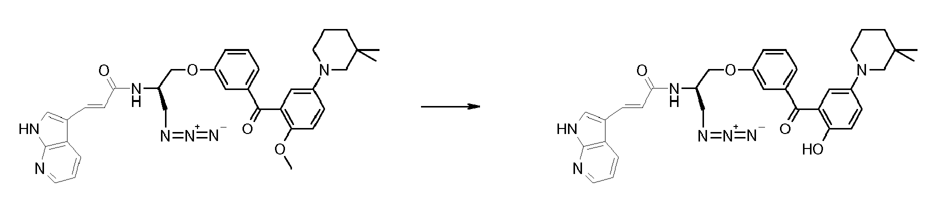

### (E)-N-(( R )-2-Azido-1-{3-[5-(3,3-dimethyl-piperidin-1-yl)-2-hydroxy-benzoyl]-phenoxymethyl}-ethyl)-3-(1H-pyrrolo[2,3-b]pyridin-3-yl)-acrylamide

A DCM solution (13 ml) of (E)-N-(( R )-2-azido-1-{3-[5-(3,3-dimethyl-piperidin-1-yl)-2-methoxy-benzoyl]-phenoxymethyl}-ethyl)-3-(1H-pyrrolo[2,3-b]pyridin-3-yl)-acrylamide (130 mg, 0.21 mmol) was cooled at -75°C and treated dropwise with BBr₃ (1M in DCM, 1.3 ml, 6 equiv). The reaction was stirred at the same temperature for 3 h 30' then quenched with MeOH (2.5 ml). The reaction was allowed to reach r.t. and the solvent was removed under reduced pressure. Water (10 ml) was added to the crude mixture and the aqueous layer neutralized with a saturated aqueous NaHCO₃ solution and extracted with EtOAc (20 ml). The organic layer was washed with brine, dried over Na₂SO₄ and concentrated in vacuo. Purification by flash chromatography (eluent: DCM / MeOH = 98:2) afforded an orange solid (86 mg, 68% yield).

1H NMR (400 MHz, DMSO-D6) δ ppm: 12.1 (bs, 1H), 9.91 (bs, 1H), 8.31 (m, 2H), 8.23 (d, *J*=8.17 Hz, 1 H), 7.92 (d, 1H, J = 2.80), 7.63 (d, 1H, J = 15.85 Hz), 7.47 (m, 1H), 7.34-7.27 (m, 3H), 7.23 (m, 1H), 7.12 (m, 1H), 6.88 (m, 1H), 6.84 (m, 1H), 6.73 (d, 1H, J = 15.85 Hz), 4.40 (m, 1H), 4.16 (dd, J = 9.75, 5.12 Hz, 1H), 4.09 (dd, J = 9.75, 5.37 Hz, 1H), 3.65 (m, 2H), 2.87 (m, 2H), 2.63 (bs, 2H), 1.63 (m, 1H), 1.26 (m, 3H), 0.95 (s, 6H).

MS (ESI) (m/z) : 594.28 (M+1, 100).

HRMS (ESI): 594.2828 (C33 H35 N7 04, M+1; calc. 594.2823).

### Example 11

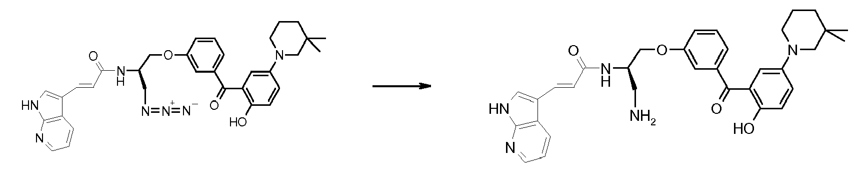

### (E)-N-(( R )-2-Amino-1-{3-[5-(3,3-dimethyl-piperidin-1-yl)-2-hydroxy-benzoyl]-phenoxymethyl}-ethyl)-3-(1H-pyrrolo[2,3-b]pyridin-3-yl)-acrylamide

To a solution of (E)-N-((R)-2-azido-1-{3-[5-(3,3-dimethyl-piperidin-1-yl)-2-hydroxybenzoyl]-phenoxymethyl}-ethyl)-3-(1H-pyrrolo[2,3-b]pyridin-3-yl)-acrylamide (88 mg, 0.148 mmol) in THF (5 ml) was added PPh₃ (70 mg, 0.267 mmol) and this solution was stirred at r.t. for 20 min. After addition of water (90 µl), the mixture was stirred at r.t. for 20 h. The solution was diluted with Et₂O (30 ml) and extracted with 1N aq HCI (25 ml). The aqueous phase was treated with solid NaHCO₃ and extracted with EtOAc (50 ml). The organic layer was dried over Na₂SO₄, the solvent evaporated under reduced pressure and chromatographed (eluent: DCM / MeOH = 90:10 then DCM / MeOH / 30% aq NH₃ = 95:5:0.5) to afford the title compound as an orange solid (53 mg, 63% yield).

1H NMR (400 MHz, DMSO-D6) δ ppm: 12.01 (bs, 1H), 8.3 (m, 2H), 7.91 (d, *J*=6.83 Hz, 1 H), 7.88 (bs, 1H), 7.58 (d, 1H, J = 15.85 Hz), 7.44 (m, 1H), 7.3-7.25 (m, 3H), 7.2 (m, 1H), 7.095 (m, 1 H), 6.86 (m, 1 H), 6.815 (m, 1 H), 6.73 (d, 1H, J = 15.85 Hz), 4.11 (m, 3H), 2.85 (m, 2H), 2.8 (m, 2H), 2.61 (bs, 2H), 1.61 (m, 2H), 1.24 (m, 2H), 0.94 (s, 6H).

MS (ESI) (m/z): 568.29 (M+1, 100).

HRMS (ESI): 568.2921 (C33 H37 N5 04, M+1; calc. 568.2918).

### Example 12

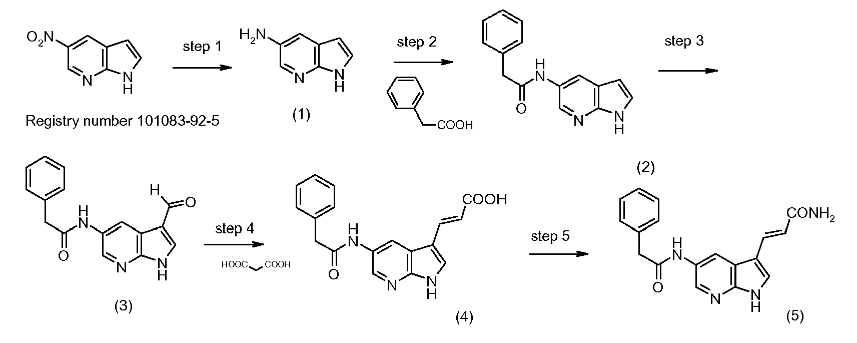

### (E)-3-(5-Phenylacetylamino-1H-pyrrolo[2,3-b]pyridin-3-yl)-acrylamide

### Step 1

4 gr. (25 mmole) of 5-nitroazaindole (registry number 101083-92-5) were dissolved in 500 ml of ethanol and 150 ml of tetrahydrofuran and hydrogenated with a Parr apparatus after addition of 2 gr of Pd/C 10% in 2 hrs. After filtration through a plug of celite and recrystallization from toluene/dicloromethane 3 gr of pure 5-aminoazaindole (1) were obtained (90% yield).

1H NMR (400 MHz, DMSO-D6) δ ppm 4.62 (s, 2 H) 6.17(dd, *J*=3.29,1.95 Hz, 1 H) 7.10 (dd, *J*=2.50,0.55 Hz, 1 H) 7.24 (t, *J*=2.87 Hz, 1 H) 7.72 (d, *J*=2.56 Hz, 1 H) 11.04 (s, 1 H )

### Step 2

600 mg (4.56 mmole ) of 5-aminoazaindole were dissolved in 12 ml of anhydrous DMF and, after addition of 675 mg (4.96 mmole) of phenylacetic acid, 1.6 gr (4.96 mmole) of TBTU, 758 mg (4.96 mmole) of HOBT and 3.4 ml (19.4 mmole) of DIPEA, the reaction was stirred overnight at r.t. Evaporation of the solvent and estraction with ethyl acetate gave, after alkaline washing, 950 mg of 2-phenyl-N- (1H-pyrrolo[2,3-b]pyridin-5-yl)-acetamide (2) (83% yield).

1H NMR (400 MHz, DMSO-D6) δ ppm 3.67 (s, 2 H) 6.40(dd, J=3.41,1.83 Hz, 1 H) 7.26-7.36 (m, 5 H) 7.44 (t, J=3.05 Hz, 1 H) 8.20 (d, J=2.32 Hz, 1 H) 8.30 (d, J=5.28 Hz, 1 H) 10.16 (s, 1 H) 11.52 (bs, 1 H)

### Step 3

800 mg (3.22 mmole) of 2-phenyl-N- (1H-pyrrolo[2,3-b]pyridin-5-yl)-acetamide were suspended in a mixture of water/acetic acid 2:1 ( ml 40 ) and refluxed after addition of 900 mg ( 6.44 mmole ) of hexamethylenetetramine for 4 hrs.The solution was evaporated, diluted with water and extracted with ethyl acetate, then purified by recrystallization from dichloromethane/methanol obtaining 560 mg of N-(3-formyl-1H-pyrrolo[2,3-b]pyridin-5-yl)-2-phenyl-acetamide (3) (62% yield).

1H NMR (400 MHz, DMSO-D6) δ ppm 3.69 (s, 2 H) 7.27-7.37 (m, 5 H) 8.43-8.50 (2d, *J*=3.3 Hz, 1 H) 8.52-8.60 (2d, *J*=2.44 Hz, 1 H) 8.73-8.77 (2d, *J*=2.33 Hz, 1 H) 9.89-9.93 (2s, 1 H) 11.36-10.42 (2s, 1 H) 12.61 (bs, 1 H)

### Step 4

230 mg (0.84 mmole) of N-(3-formyl-1H-pyrrolo[2,3-b]pyridin-5-yl)-2-phenyl-acetamide were suspended in 4.5 ml of pyridine and, after addition of 260 mg (2.52 mmole) of malonic acid and 0.025 ml of piperidine, were heated at 70° C for 4 hrs. The dried residue was diluted with water and filtered giving, after several washings with diethyl ether, 150 mg of (E)-3-(5-phenylacetylamino-1H-pyrrolo[2,3-b]pyridin-3-yl)-acrylic acid (4) (55% yield). The product was used for the following reaction without any further purification.

### Step 5

150 mg (0.46 mmole) of (E)-3-(5-phenylacetylamino-1H-pyrrolo[2,3-b]pyridin-3-yl)-acrylic acid were dissolved in 8 ml of dichloromethane/dimethylformamide 1:1 and stirred overnight at r.t. after addition of 140 mg (0.92 mmole ) of HOBT ammonium salt, 295 mg (0,92 mmole) of TBTU and 0.31 ml (1.84 mmole) of DIPEA. The solution was diluted with water, extracted with ethyl acetate, concentrated in vacuo and purified by silica gel chromatography with dichloromethane/methanol 9:1 as eluent. 100 mg of the title compound (5) were obtained (68% yield).

1H NMR (400 MHz, DMSO-D6) δ ppm 6.44 (d, J=15.97 Hz, 1 H) 6.82 (s, 1 H) 7.23 - 7.32 (m, 1 H) 7.31 - 7.43 (m, 4 H) 7.51 (s, 1 H) 7.55 (d, J=15.97 Hz, 1 H) 7.88 (d, J=2.80 Hz, 1 H) 8.36 (d, J=2.19 Hz, 1 H) 8.54 (d, J=2.19 Hz, 1 H) 10.30 (s, 1 H) 11.99 - 12.07 (m, 1 H).

Analogously the following compounds have been obtained:

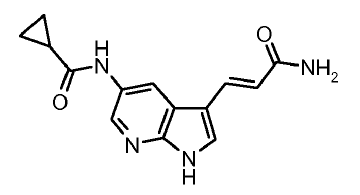

### Cyclopropanecarboxylic acid [3-((E)-2-carbamoyl-vinyl)-1H-pyrrolo[2,3-b]pyridin-5-yl]-amide

1 H NMR (400 MHz, DMSO-D6) δ ppm 0.79 - 0.92 (m, 4 H) 1.78 - 1.89 (m, 1 H) 6.46 (d, J=15.97 Hz, 1 H) 6.83 (bs, 1 H) 7.47 (bs, 1 H) 7.55 (d, J=15.85 Hz, 1 H) 7.87 (d, J=2.80 Hz, 1 H) 8.33 (d, J=2.32 Hz, 1 H) 8.52 (d, J=2.32 Hz, 1 H) 10.29 (s, 1 H).

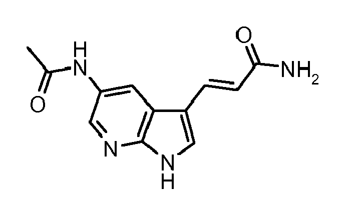

### (E)-3-(5-acetylamino-1H-pyrrolo[2.3-b]pyridin-3-yl)-acrylamide

1 H NMR (400 MHz, DMSO-D6) δ ppm 2.04 (s, 3 H) 6.47 (d, J=15.85 Hz, 1 H) 6.84 (s, 1 H) 7.42 - 7.50 (m, 1 H) 7.55 (d, J=15.85 Hz, 1 H) 7.87 (d, J=2.80 Hz, 1 H) 8.31 (d, J=2.32 Hz, 1 H) 8.48 (d, J=2.32 Hz, 1 H) 10.04 (s, 1 H) 12.02 (s, 1 H).

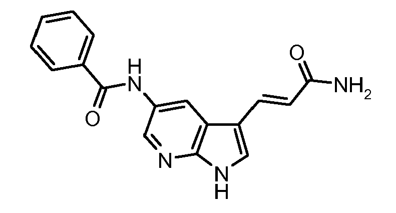

### N-[3-((E)-2-carbamoyl-vinyl)-1H-pyrrolo[2,3-b]pyridin-5-yl]-benzamide

1 H NMR (400 MHz, DMSO-D6) δ ppm 6.53 (d, J=15.97 Hz, 1 H) 6.71 - 6.99 (m, 1 H) 7.46 (s, 1 H) 7.52 - 7.69 (m, 4 H) 7.91 (d, J=2.80 Hz, 1 H) 8.01 - 8.07 (m, 2 H) 8.61 (d, J=2.07 Hz, 1 H) 10.41 (s, 1 H) 12.08 (s, 1 H).

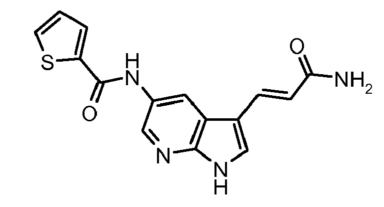

### Thiophene-2-carboxylic acid [3-((E)-2-carbamoyl-vinyl)-1H-pyrrolo[2,3-blpyridin-5-yl]-amide

1 H NMR (400 MHz, DMSO-D6) δ ppm 6.54 (d, J=15.97 Hz, 1 H) 6.85 (s, 1 H) 7.28 (dd, J=5.00, 3.78 Hz, 1 H) 7.42 - 7.50 (m, 1 H) 7.58 (d, J=15.97 Hz, 1 H) 7.89 (dd, J=5.00, 1.10 Hz, 1 H) 7.92 (d, J=2.80 Hz, 1 H) 8.07 (dd, J=3.78, 0.98 Hz, 1 H) 8.48 (d, J=2.19 Hz, 1 H) 8.55 (d, J=2.32 Hz, 1 H) 10.42 (s, 1 H) 12.06 - 12.13 (m, 1 H).

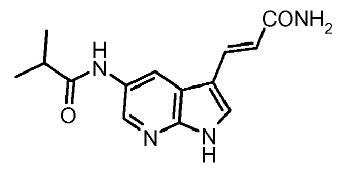

### (E)-3-(5-Isobutyrylamino-1H-pyrrolo[2,3-b]pyridin-3-yl)-acrylamide

1H NMR (400 MHz, DMSO-D6) δ ppm 1.18 (d, J=6.83 Hz, 6 H) 2.60 - 2.71 (m, 1 H) 6.46 (d, J=15.85 Hz, 1 H) 6.83 (s, 1 H) 7.51 (s, 1 H) 7.56 (d, J=15.85 Hz, 1 H) 7.87 (d, J=2.80 Hz, 1 H) 8.35 (d, J=2.19 Hz, 1 H) 8.55 (d, J=2.19 Hz, 1 H) 9.93 (s, 1 H) 11.96 - 12.06 (m, 1 H).

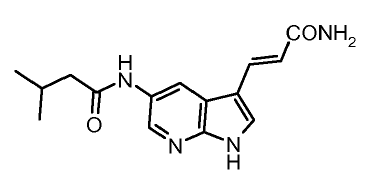

### N-[3-((E)-2-Carbamoyl-vinyl)-1H-pyrrolo[2,3-b]pyridin-5-yl]-3-methyl-butyramide

1H NMR (400 MHz, DMSO-D6) δ ppm 0.97 (s, 6 H) 2.11 (dd, J=6.71 Hz, J=6.83 Hz, 1 H) 2.21 (d, J=7.07 Hz, 2 H) 6.39 (d, *J*=15.98 Hz, 1 H) 6.81 (s, 1 H) 7.48 (s, 1 H) 7.57 (d, J=15.82 Hz, 1 H) 7.84 (d, J=2.80 Hz, 1 H) 8.31 (d, J=2.19 Hz, 1 H) 8.49 (d, J=2.32 Hz, 1 H) 9.95(s, 1 H) 11.99 (s, 1 H).

### Example 13

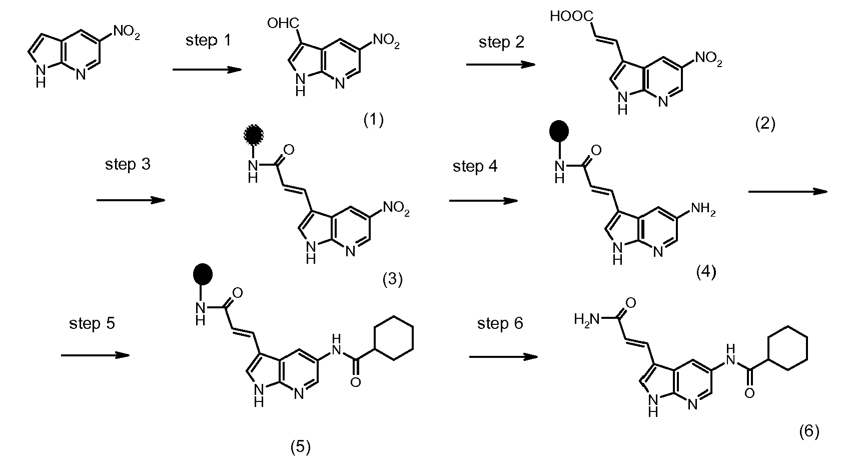

### Cyclohexanecarboxylic acid [3-((E)-2-carbamoyl-vinyl)-1H-pyrrolo[2,3-b]pyridin-5-yl]-amide

### Step 1

4 g (24.5 mmole) of 5-nitro-1H-pyrrolo[2,3-b] pyridine (registry number 101083-92-5) were suspended in a mixture of water/acetic acid 2:1 (ml 60) and refluxed after addition of 10 mg (71.3 mmole ) of hexamethylenetetramine for 4 hrs. The solution was evaporated, diluted with water and extracted with ethyl acetate, then purified by recrystallization from dichloromethane/methanol obtaining 2.5 mg of 5-nitro-1H-pyrrolo[2,3-b] pyridine -3-carbaldehyde (1) (56 % yield).

1H NMR (400 MHz, DMSO-D6) δ ppm 8.77 (d,1 H) 9.12 (d, J=2.56 Hz, 1 H) 9.25 (d, J=2.56 Hz, 1 H) 10.04 (s, 1 H) 13.41 (bs, 1 H)

### Step 2

1.7 g (8.9 mmole) of 5-nitro-1H-pyrrolo[2,3-b] pyridine -3-carbaldehyde were suspended in 22 ml of pyridine and, after addition of 2.77 g (26.7 mmole) of malonic acid and 0.45 ml of piperidine, were heated at 70° C for 4 hrs. The dried residue was diluted with water and filtered giving, after several washings with diethyl ether, 1.3 g of (E)-3-(5-nitro-1H-pyrrolo[2,3-b]pyridin-3-yl)-acrylic acid (2) (58% yield). The product was used for the following reaction without any further purification.

### Step 3

Before the coupling, Rink resin was shaken in a 20% solution of piperidine in DMF for 3 times respectively 1',3',15'min to remove Fmoc protection of the amino group then it was washed with DMF, DCM, MeOH, DCM and dried.

Loading of the azaindole: theRink resin was swollen in NMP and 2 eq. of 5-NO2 azaindolyl 3-acrilacetic acid (2), 2 eq. of TBTU and 2.5 eq. of DIPEA were added. The suspension was shaken overnight at r.t. The resin was then washed with NMP, DCM, MeOH, dried and weighed. Purity and identity of the loaded product was verified by cleavage of 50 mg of resin (3).

### Step 4

10 eq. of SnCl₂.2H₂O were added to the resin (3) in DMF. The suspension was shaken overnight, then washed with DMF, a mixture of DMF and H₂O previously heated at 50°C, DMF, DCM, MeOH, DCM and dried.

30 mg of dried resin (4) were cleaved. The HPLC trace showed that the reaction was gone to completion.

### Step 5

About 200 mg of resin (4) 2 eq. of cyclohexancarboxylic acid, 2 eq. of PyBOP, 2 eq. of DIPEA and 3 ml of DMF were added. After an overnight shaking, the resin was washed with DMF, DCM, MeOH, DCM and a few milligrams were cleaved with a TFA/DCM 50% solution, in order to verify the purity of the final product (5). As the use of PyBOP brought to the formation of bis-acetilated product, the resin was dissolved in DMF/conc. NH₄OH 4/1 (v/v) and shaken for 4 hours at r.t. to remove the byproduct.

### Step 6

The resin (5) was cleaved with a TFA/DCM 50% solution for 1 hour and the title compound (6) was analyzed and subjected to preparative HPLC/MS.
1H NMR (400 MHz, DMSO-D6) δ ppm 1.11 - 1.37 (m, 7 H) 1.36 - 1.56 (m, 1 H) 1.58 - 1.72 (m, 1 H) 1.70 - 1.94 (m, 1 H) 2.27 - 2.43 (m, 1 H) 6.42 (d, J=15.97 Hz, 1 H) 6.81 (s, 1 H) 7.48 (s, 1 H) 7.53 (d, J=15.85 Hz, 1 H) 7.83 (d, J=2.68 Hz, 1 H) 8.31 (d, J=2.19 Hz, 1 H) 8.53 (d, J=2.20 Hz, 1 H) 9.88 (s, 1 H) 11.97 (s, 1 H).

Analogously the following compounds have been obtained:

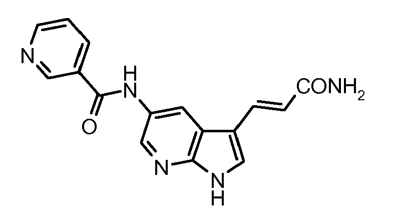

### N- [3-((E)-2-carbamoyl-vinyl)-1H-pyrrolo[2,3-b]pyridin-5-yl]-nicotinamide

1H NMR (400 MHz, DMSO-D6) δ ppm 6.54 (d, *J*=15.86 Hz, 1 H) 6.86 (s, 1 H) 7.46(s, 1 H) 7.59(d, *J*=15.73 Hz, 1 H) 7.63 (dd, *J*=4.76 Hz, *J*=7.87 Hz ,1 H) 7.92 (d, *J*=2.81 Hz, 1 H) 8.38 (m, 1H ) 8.52 (d, *J*=2.19 Hz, 1 H) 8.62 (d, *J*=2.20 Hz, 1 H) 8.80 (dd, *J*=4.75Hz, *J*=1.58 Hz ,1 H) 9.21(d, *J*=1.95 Hz, 1 H) 10.62 (s, 1 H) 12.11 (s, 1 H).

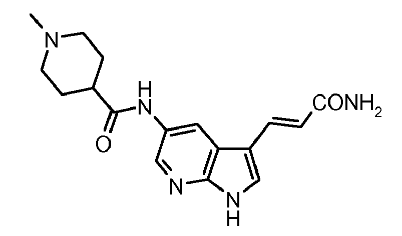

### 1-Methyl-piperidine-4-carboxilic acid [3-((E)-2-carbamoyl-vinyl)-1H-pyrrolo[2,3-b]pyridin-5-yl]-amide

1 H NMR (400 MHz, DMSO-D6) δ ppm 1.87 - 2.06 (m, 4 H) 2.55 - 2.66 (m, 1 H) 2.80 (s, 3 H) 2.98 - 3.49 (m, 4 H) 6.42 (d, *J*=15.98 Hz, 1 H) 6.84(s, 1 H) 7.48 (s, 1 H) 7.54 (d, *J*=15.98 Hz, 1 H) 7.86 (d, *J*=2.80 Hz, 1 H) 8.32 (d, J=2.20 Hz, 1 H) 8.51 (d, J=2.31 Hz, 1 H) 10.16 (s, 1 H) 12.03 (s, 1 H).

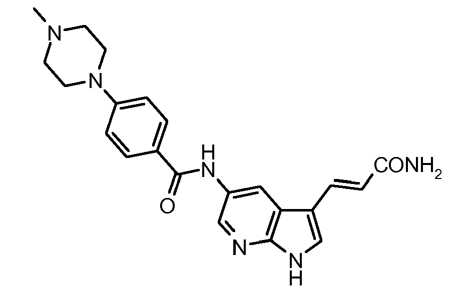

### N-[3-((E)-2-carbamoyl-vinyl)-1H-pyrrolo[2,3-b]pyridin-5-yl]-4-(4 methyl-piperazin-1-yl)-benzamide

1H NMR (400 MHz, DMSO-D6) δ ppm 2.87 (s, 3 H) 3.05 - 4.07 (m, 8 H) 6.47 (d, *J*=15.85 1 H) 6.83,7.43 (2 bs, 2 H) 7.11 - 7.13 (2 s, 2 H) 7.57 (d, *J*=15.85 1 H) 7.87 (d, *J*=2.68 Hz, 1 H) 7.97,7.99 (2 s, 2 H) 8.49 (d, *J*=2.19 Hz, 1 H) 8.56 (d, *J*=2.20 Hz, 1 H) 9.69 ( bs, 1 H ) 10.14 (s, 1 H) 12.02 (s, 1 H).

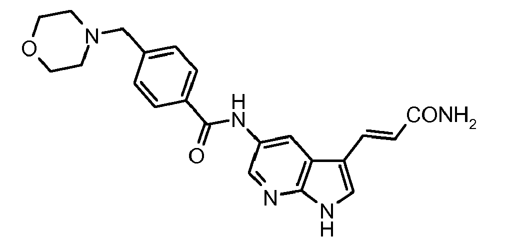

### N-[3-((E)-2-carbamoyl-vinyl)-1H-pyrrolo[2,3-b]pyridin-5-yl]-4-morpholin-4-ylmethyl-benzamide

1H NMR (400 MHz, DMSO-D6) δ ppm 3.1-4.45 (m, 10 H) 6.50 (d, J=15.97 Hz, 1 H) 6.85 (s, 1 H) 7.41 (s, 1 H) 7.56 (d, J=15.97 Hz, 1 H) 7.66-7.68 (2s, 2 H) 7.89 (d, J=2.81 Hz, 1 H) 8.11-8.13 (2s, 2 H) 8.50 (d, J=2.20 Hz, 1 H) 8.58 (d, J=2.20 Hz, 1 H) 10.46 (s, 1 H) 12.08 (s, 1 H).

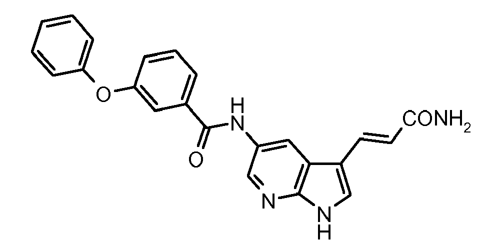

### N- [3-((E)-2-carbamoyl-vinyl)-1H-pyrrolo[2,3-b]pyridin-5-yl]-3-phenoxy-benzamide

1 H NMR (400 MHz, DMSO-D6) δ ppm 6.48 (d, J=15.85 Hz, 1 H) 6.84, 8.0 (2s, 2 H) 7.06 (d, J=9.76 Hz, 1 H) 7.20 (d, J=8.54 Hz, 1 H) 7.19-7.3 (m, 3 H) 7.42-7.65 (m, 4 H) 7.84 (d, *J*=7.81 Hz, 1 H) 7.90 (d, *J*=2.68 Hz, 1 H) 8.49 (d, *J*=2.20 Hz, 1 H) 8.58 (d, *J*=2.20 Hz, 1 H) 10.44 (s, 1 H) 12.08 (s, 1 H).

### Example 14

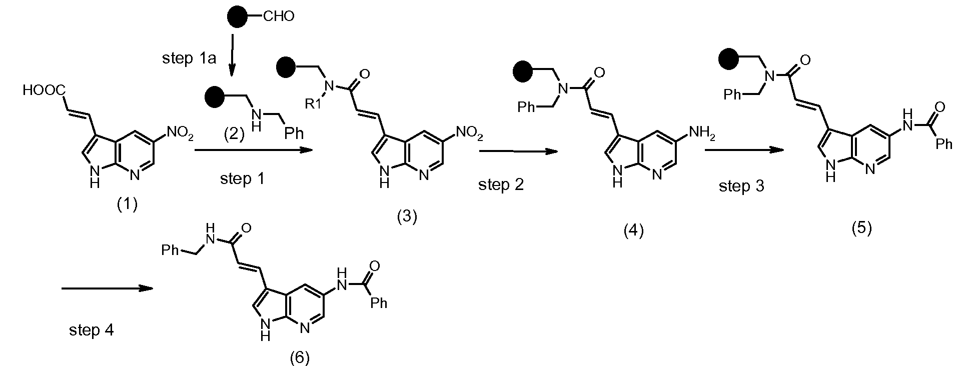

### N-[3-((E)-2-Benzylcarbamoyl-vinyl)-1H-pyrrolo[2,3-b]pyridin-5-yl]-benzamide

### Step 1a

The formyl resin was subjected to reductive amination with benzylamine.

To the resin swollen in DCM, 5 eq. of benzylamine, 3 eq. of NaHB(OAc)₃, and 1 eq. of acetic acid were added consecutively. The mixture was shaken overnight. The resin was washed with MeOH, DMF, MeOH, DCM, then dried and weighed.

A 100 mg sample of resin was treated with 2 eq. of benzensulphonyl chloride and 2.5 eq. of DIPEA in DCM. The obtained sulfonamide was cleaved with TFA/DCM 50% and the cleavage solution analyzed by HPLC/MS: the purity of the final product (2) was >98% at 254nm.

### Step 1

2 mmole. of (E)-3-(5-nitro-1H-pyrrolo[2,3-b]pyridin-3-yl)-acrylic acid (1) and 2.2 mmole of CDI were dissolved in 5 ml of DMF and reacted at 50°C for 2 hrs.Then, after addition of 500 mg of resin (2), the suspension was shaken overnight at r.t.and the day after at 50°C. The resin was then washed with DMF, DCM, MeOH, dried and weighed. Purity and identity of the loaded product was verified by cleavage of 50 mg of resin (3).

### Step 2

10 eq. of SnCl₂.2H₂O were added to the resin (3) in DMF. The suspension was shaken overnight, then washed with DMF, a mixture of DMF and H₂O previously heated at 50°C, DMF, DCM, MeOH, DCM and dried.

30 mg of dried resin (4) were cleaved. The HPLC trace showed that the reaction was gone to completion.

### Step 3

About 200 mg of resin (4), 2 eq. of benzoic acid, 2 eq. of PyBOP, 2 eq. of DIPEA and 3 ml of DMF were added. After an overnight shaking, the resin was washed with DMF, DCM, MeOH, DCM and a few milligrams were cleaved with a TFA/DCM 50% solution in order to verify the purity of the final product (5). As the use of PyBOP brought to the formation of bis-acetylated product, the resin was dissolved in DMF/conc. NH₄OH 4/1 (v/v) and shaken for 4 hours at r.t. to remove the byproduct.

### Step 4

The resin (5) was cleaved with a TFA/DCM 50% solution 1 hour and the title compound (6) was analyzed and subjected to preparative HPLC/MS.

1H NMR (400 MHz, DMSO-D6) δ ppm 4.39 (d, *J*=5.85 Hz, 2 H) 6.60 (d, *J*=15.85 Hz, 1H) 7.23-7.34 (m, 5 H) 7.55-7.60 (m, 3 H) 7.64 (d, *J*=15.85 Hz, 1H) 7.90(d, *J*=2.80 Hz, 1 H) 7.91-8.02 (m, 2 H) 8.46 (d, *J*=2.32 Hz, 1 H) 8.48 (t, *J*=6.10 Hz, 1 H) 8.61(d, *J* =2.19Hz, 1 H) 10.38 (s, 1 H) 12.06 (s, 1 H).

According to this methodology the following compounds may be prepared:

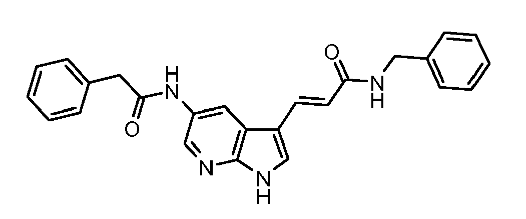

### (E)-N-Benzyl-3-(5-phenylacetylamino-1H-pyrrolo[2,3-b]pyridin-3-yl]-acrylamide

1H NMR (400 MHz, DMSO-D6) δ ppm 3.68 (s, 2H) 4.25-4.36 ( dd, *J* = 5.98 ,7.07 Hz, 2H) 6.48 (d, *J*=15.97 Hz, 1 H) 7.21-7.34 (m, 10 H) 7.60 (d, *J*=15.85 Hz, 1 H) 7.87 (d, *J*=2.80 Hz, 1 H) 8.30 (d, *J*=2.19 Hz, 1 H) 8.54 (m, 2H) 10.44 (s, 1 H) 12.08 (s, 1 H).

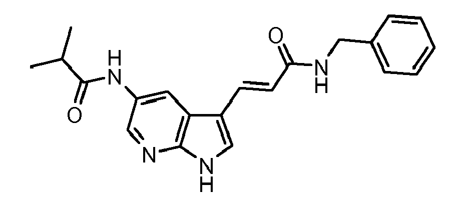

### (E)-N-Benzyl-3-(5-isobutyrylamino-1H-pyrrolo[2,3-b]pyridin-3-yl]-acrylamide

1H NMR (400 MHz, DMSO-D6) δ ppm 1.12-1.14 (2s, 6H) 2.60-2.65 (m, 1H) 4.40 ( d, *J* = 5.97 Hz, 2H) 6.50 (d, J=15.85 Hz, 1 H) 7.24-7.33 (m, 5 H) 7.61(d, J=15.86 Hz, 1 H) 7.87(d, *J*=2.88 Hz, 1 H) 8.29 (d, *J*=2.20 Hz, 1 H) 8.56 (m, 2H) 10.04 (s, 1 H) 11.99 (s, 1 H).

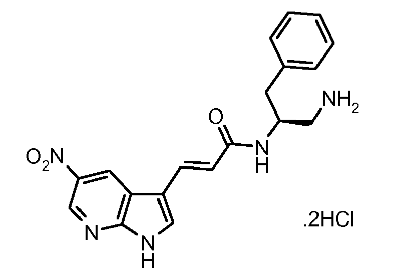

### (2E)-N-[(1S)-2-amino-1-benzylethyll-3-(5-nitro-1H-pyrrolo[2,3-b]pyridin-3-yl)acrylamide dihydrochloride

1H NMR (400 MHz, DMSO-D6) δ ppm 12.95 (bs, NH), 9.21 (d, J= 2.44 Hz, 1H), 9.05 (d, J= 2.44 Hz, 1H), 8.25 (d, J= 8.29 Hz, 1H), 8.23 (d, J= 2.68 hz, 1H), 7.95 (b, 4H), 7.61 (d, J= 15.97 Hz, 1H), 7.30 (m, 5H), 7.64 (d, J= 15.97 Hz, 1H), 4.34 (m, 1H), 2.98 (m, 4H).

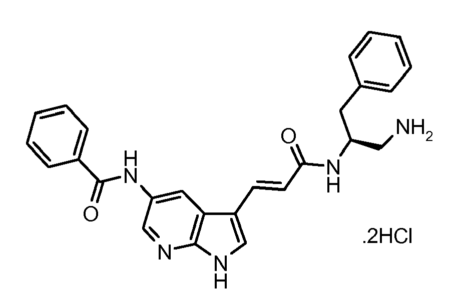

### N-{3-[(1E)-3-{[(1S)-2-amino-1-benzylethyl]amino}-3-oxoprop-1-en-1-yl]-1H-pyrrolo[2,3-b]pyridin-5-yl}benzamide dihydrochloride

1H NMR (400 MHz, DMSO-D6) δ ppm 12.15 (bs, NH), 10.46 (b, NH), 8.69 (bs, 1H), 8.47 (bs, 1 H), 8.14 (d, J= 8.17 Hz, 1 H), 8.05 (m, 2H), 7.94 (bs, 1 H), 7.91 (b, 3H), 7.58 (m, 4H), 7.28 (m, 5H), 6.51 (d, J= 15.90 Hz, 1 H), 4.34 (m, 1 H), 2.86 (m, 4H).

### Example 15

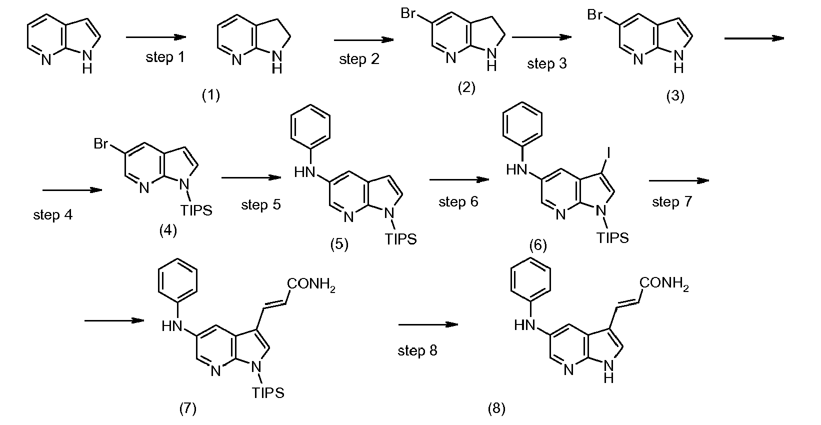

### (E)-3-(5-Phenylamino-1H-pyrrolo[2,3-b]pyridin-3-yl)-acrylamide

### Step 1

To a stirred and cooled solution of 10 g (85 mmole)of 1H-pyrrolo[2,3-b]pyridine in 150 ml of formic acid, were added, cautiously, 5.35 g of Pd/C 10% and 30 ml of TEA dropwise; the mixture was stirred at 80°C for 4 days. Then the mixture was cooled to r.t., the catalyst was filtered off and washed with formic acid and ethanol. After concentration in vacuo, the residual solution was cooled, basified to pH 13 by slow addition of 50% aqueous NaOH (100 ml) and refluxed for 2 hrs. The two-phase system was extracted with ethyl acetate and concentrated in vacuo giving 7 g of 2.3-dihydro- 1H-pyrrolo[2,3-b]pyridine (1) (70% yield).

1 H NMR (400 MHz, DMSO-D6) δ ppm 2.96 ( t, *J* = 8.65 Hz, 2H) 3.45 (t, *J*=8.29 Hz, 2H) 6.26 (bs, 1 H) 6.42 (dd, *J*= 5.24 Hz, *J*= 7.07 Hz,1 H) 7.23 (d, *J*=6.95 Hz, 1 H) 7.67 (d, *J*=5.84 Hz, 1 H).

### Step 2

A solution of Br₂ (2.78 ml, 8.64 g, 54 mmole) in dry dichloromethane (40 ml) was added dropwise over a period of 1 h 45 min to a stirred and cooled (-10°C) solution of 2.3-dihydro-1H-pyrrolo[2,3-b]pyridine (6.5 g,54 mmole) in a mixture of dry dichloromethane (60 ml) and pyridine (6 ml). The suspension was stirred at 0°C for 2 hrs, poured into a mixture of NaHCO₃ (120 ml) and saturated aqueous Na₂S₂O₃ (15 ml) and extracted with a solution of ethyl acetate/methanol (3X200 ml). The organic layers were concentrated to afford 3.7 g of 5-bromo- 2,3-dihydro- 1H-pyrrolo[2,3-b]pyridine (2) after purification by flash chromatography using dichloromethane as eluent (35% yield).

1H NMR (400 MHz, DMSO-D6) δ ppm 2.98 ( t, *J* = 8.54 Hz, 2H ) 3.48 (t, *J*=8.55 Hz, 2H) 6.58 (bs, 1 H) 7.37(s,1 H) 7.71 (s,1 H)

### Step 3

To a stirred solution of 5-bromo- 2,3-dihydro- 1H-pyrrolo[2,3-b]pyridine (3.4 g, 17 mmole) in 30 ml of toluene was added activated MnO₂ ( 9.5 g, 100 mmole) and the mixture was heated at 90°C for 2 hrs. Then the reaction mixture was filtered through a pad of celite and washed with dichloromethane. Purification was performed by flash chromatography using dichloromethane as eluent giving 2.5 g of 5-bromo- 1 H-pyrrolo[2,3-b]pyridine (3) (75% yield).

1H NMR (400 MHz, DMSO-D6) δ ppm 6.46 (dd, *J*=1.83 Hz , *J*=1.70 Hz, 1H) 7.55 (t, J=3.05 Hz , 1H) 8.21(d, *J*=2.32 Hz , 1 H) 8.27(d, *J*=2.90 Hz , 1 H) 11.87 (bs,1 H)

### Step 4

To a stirred solution of 5-bromo- 1 H-pyrrolo[2,3-b]pyridine (1.5 g, 7.5 mmole) in 15 ml of dioxane and 5 ml of DMF, sodium hydride ( 345 mg, 9 mmole) was added portionwise and, after 0.5 hrs., TIPSCI (2.5 ml, 12.3 mmole); the reaction was stirred at r.t. overnight, then diluted with water and extracted with methylene chloride; a fast purification by silica gel chromatography with dichloromethane gave 2.6 g of 5-bromo-1-triisopropylsilanyl- 1H-pyrrolo[2,3-b]pyridine (4) (97% yield). This product was used for the following reaction without any further purification.

### Step 5

To a solution of 5-bromo-1-triisopropylsilanyl- 1H-pyrrolo[2,3-b]pyridine ( 2.3 g, 6 mmole) in 50 ml of dry toluene were added consecutively: aniline ( 0.65 ml, 7.2 mmole), sodium t-butoxide ( 810 mg, 8.4 mmole ), Pd₂(dba)₃ (330 mg, 0.36 mmole) and 1,1'-bis(diphenylphosphino)ferrocene (dppf) (400 mg, 0.72 mmole); the mixture was refluxed for 4 hrs, then diluted with dichloromethane and filtered through a pad of celite giving, after chromatography with cyclohexane/ethyl acetate 8/2, 2 g of phenyl-(1-triisopropylsilanyl- 1H-pyrrolo[2,3-b]pyridin-5-yl)-amine (5) (90% yield).

1H NMR (400 MHz, DMSO-D6) δ ppm 1.10 (d, *J*=7.56 Hz , 18 H) 1.86 (m, 3 H) 6.53 (d, *J*=3.42Hz, 1 H) 6.74 (m, 1 H) 6.97 (m, 2 H) 7.19 (m, 2 H) 7.42 (d, *J*=3.41 Hz , 1 H) 7.73 (s , 1 H) 7.91 (s , 1 H) 8.04 (d, J=2.56 Hz , 1 H)

### Step 6

A solution of 2 g (5.4 mmole) of phenyl-(1-triisopropylsilanyl- 1H-pyrrolo[2,3-b]pyridin-5-yl)-amine in 25 ml of DMF was stirred at r.t. for 1 day after addition of 330 mg (5.9 mmole) of potassium hydroxide and 1.5 g (5.9 mmole) of iodine; the solution was diluted with water and extracted with ethyl acetate giving, after purification by silica gel chromatography with cyclohexane/methylene chloride 9/1, 500 mg of ( 3-iodo-1-triisopropylsilanyl- 1H-pyrrolo[2,3-b]pyridin-5-yl)-phenyl-amine (6) (20% yield).

### Step 7

To a solution of (3-iodo-1-triisopropylsilanyl-1H-pyrrolo[2,3-b]pyridin-5-yl)-phenyl-amine (290 mg, 0.59 mmole) in 10 ml of dry acetonitrile were added consecutively: acrylamide (340 mg, 4.72 mmole), Pd(OAc)₂ (27 mg, 0.120 mmole), P(oTolyl)₃ (71.7 mg, 0.236 mmole) and TEA (0.41 ml, 2.95 mmole); the mixture was refluxed for 4 hrs, then diluted with water, extracted with dichloromethane and filtered giving, after chromatography with cyclohexane/ethyl acetate 8/2, 55 mg of (E)- 3-(5-phenylamino-1-triisopropylsilanyl- 1H-pyrrolo[2,3-b]pyridin-5-yl)-acrylamide (7) (22% yield).

### Step 8

55 mg (0.126 mmole) of (E)- 3-(5-phenylamino-1-triisopropylsilanyl- 1 H-pyrrolo[2,3-b]pyridin-5-yl)-acrylamide were dissolved in 1 ml of methanol and stirred at r.t. for 1 h after addition of HCl 1M in dioxane (0.25 ml, 2 mmole). The solution was concentrated in vacuo and, after precipitation with diethylether, the solid was filtered to give 35 mg of the title compound (80% yield).

1H NMR (400 MHz, DMSO-D6) δ ppm 6.46 (d, *J*=15.85 Hz , 1 H) 6.72 (t, *J*=7.26 Hz , 1 H) 6.80(bs, 1 H) 6.90 (d, *J*=7.68 Hz , 2 H) 7.18 (dd, *J*=8.40,7.40 Hz , 2 H) 7.37 (bs , 1 H) 7.55 (d, *J*=15.85 Hz, 1 H) 7.85 (d, *J*=2.80 Hz, 1 H) 8.02 (d, *J*=2.32 Hz , 1 H) 8.13 (d, *J*=2.20 Hz , 1 H) 11.98 (s , 1 H)

## Claims

1. A compound represented by formula (I) 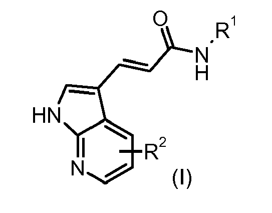 wherein
R¹ is a hydrogen or a straight or branched C₁-C₆ alkyl, C₃-C₆ cycloalkyl, heterocyclyl, aryl, arylalkyl, heteroarylalkyl, heterocyclylalkyl; or R¹ is the group of formula (II) 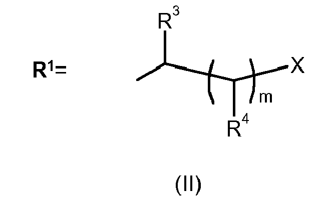 wherein each of said C₁-C₆ alkyl, C₃-C₆ cycloalkyl, heterocyclyl, aryl, arylalkyl, heteroarylalkyl, heterocyclylalkyl moieties can be unsubstituted or substituted by one or more substituents, each substituent being independently selected from the group consisting of alkyl, aryl, -OCF₃, -OC(O)alkyl, -OC(O)aryl, -CF₃, heteroaryl, aralkyl, alkylaryl, heteroaralkyl, alkylheteroaryl, hydroxy, hydroxyalkyl, alkoxy, aryloxy, aralkoxy, acyl, aryl, halo, haloalkyl, haloalkoxy, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, heterocyclyl, amino; - NH(alkyl),
-NH(cycloalkyl), and -N(alkyl)₂;
m = 0-1-2;
R³ and R⁴ are selected from the group consisting of hydrogen, (C₁-C₆)alkyl, (C₂-C₆)alkenyl, (C₂-C₆)alkynyl, (C₁-C₆)cycloalkyl, phenyl, arylalkyl, and heteroarylalkyl; wherein each of said (C₁-C₆)alkyl, (C₂-C₆)alkenyl, (C₂-C₆)alkynyl, (C₁-C₆)cycloalkyl, phenyl, arylalkyl, and heteroarylalkyl moieties can be unsubstituted or substituted by one or more substituents, each substituent being independently selected from the group consisting of alkyl, aryl, -OCF₃, - OC(O)alkyl, -OC(O)aryl, -CF₃, heteroaryl, aralkyl, alkylaryl, heteroaralkyl, alkylheteroaryl, hydroxy, hydroxyalkyl, alkoxy, aryloxy, aralkoxy, acyl, aryl, halo, haloalkyl, haloalkoxy, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, heterocyclyl, amino; -NH(alkyl), -NH(cycloalkyl), and - N(alkyl)_{2;}
X can be hydrogen, hydroxyl, alkyloxy, carbamoyl, cyano, azido or a group of formula (III) 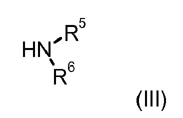
wherein R⁵ and R⁶ are, each independently, hydrogen or an optionally substituted group selected from straight or branched C₁-C₆ alkyl, C₃-C₆ cycloalkyl, and R⁵ and R⁶, taken together with the nitrogen atom to which they are bonded, may form an optionally substituted heterocyclyl group, optionally containing an additional heteroatom selected from N, O and S;
R² is selected from the group consisting of hydrogen, nitro, amino, arylamino, arylalkylamino, heteroarylamino, (C₃-C₈)alkylcarbonylamino, (C₄-C₇)cycloalkylcarbonylamino, arylcarbonylamino, heteroarylcarbonylamino, heterocyclylcarbonylamino, arylalkylcarbonylamino, and halogen; wherein each of said arylamino, heteroarylamino, (C₃-C₈)alkylcarbonylamino, (C₄-C₇)cycloalkylcarbonylamino, arylcarbonylamino, heteroarylcarbonylamino, heterocyclylcarbonylamino, and arylalkylcarbonylamino moieties can be unsubstituted or substituted by one or more substituents, each substituent being independently selected from the group consisting of alkyl, aryl, -OCF₃, -OC(O)alkyl, -OC(O)aryl, -CF₃, heteroaryl, aralkyl, alkylaryl, heteroaralkyl, alkylheteroaryl, hydroxy, hydroxyalkyl, alkoxy, aryloxy, aralkoxy, acyl, aryl, halo, haloalkyl, haloalkoxy, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, heterocyclyl, amino; -NH(alkyl), and -NH(cycloalkyl), and -N(alkyl)2, or a pharmaceutically acceptable salt thereof;
with the proviso that the compound (E)-2-propenamide, N-[4-[4-(diphenylmethyl)-1-piperazinyl]butyl]-3-(1H-pyrrolo[2,3-b]pyridin-3-yl) is excluded.

2. The compound according to claim 1, wherein R⁵ and R⁶ are hydrogen atoms.

3. A compound or a pharmaceutically acceptable salt thereof is selected from the group consisting of:
(E)-3-(1H-Pyrrolo[2,3-b]pyridin-3-yl)-acrylamide;
(E)-N-Methyl-3-(1H-pyrrolo[2,3-b]pyridin-3-yl)-acrylamide;
(E)-N-Benzyl-3-(1H-pyrrolo[2,3-b]pyridin-3-yl)-acrylamide;
(2E)-N-[(1S)-1-phenyl-2-pyrrolidin-1-ylethyl]-3-(1H-pyrrolo[2,3-b]pyridin-3-yl)acrylamide;
(2E)-N-[(1S)-2-(4-methylpiperazin-1-yl)-1-phenylethyl]-3-(1H-pyrrolo[2,3-b]pyridin-3-yl)acrylamide;
(2E)-N-(2-morpholin-4-ylethyl)-3-(1H-pyrrolo[2,3-b]pyridin-3-yl)acrylamide;
(2E)-N-(2-methoxyethyl)-3-(1H-pyrrolo[2,3-b]pyridin-3-yl)acrylamide;
(2E)-N,N-dimethyl-3-(1H-pyrrolo[2,3-b]pyridin-3-yl)acrylamide;
(2E)-N-(2-pyrrolidin-1-ylethyl)-3-(1H-pyrrolo[2,3-b]pyridin-3-yl)acrylamide;
(2E)-N-(1-phenylethyl)-3-(1H-pyrrolo[2,3-b]pyridin-3-yl)acrylamide;
(2E)-N-[3-(4-methylpiperazin-1-yl)propyl]-3-(1H-pyrrolo[2,3-b]pyridin-3-yl)acrylamide;
(2E)-N-[(1S)-2-morpholin-4-yl-1-phenylethyl]-3-(1H-pyrrolo[2,3-b]pyridin-3-yl)acrylamide;
(2E)-N-benzyl-3-(1H-pyrrolo[2,3-b]pyridin-3-yl)acrylamide;
(2E)-N-(4-fluorobenzyl)-3-(1H-pyrrolo[2,3-b]pyridin-3-yl)acrylamide;
(2E)-N-(2-phenylethyl)-3-(1H-pyrrolo[2,3-b]pyridin-3-yl)acrylamide;
(2E)-N-(4-methoxyphenyl)-3-(1H-pyrrolo[2,3-b]pyridin-3-yl)acrylamide;
(2E)-N-[4-(4-methylpiperazin-1-yl)benzyl]-3-(1H-pyrrolo[2,3-b]pyridin-3-yl)acrylamide;
(2E)-N-(2-morpholin-4-ylbenzyl)-3-(1H-pyrrolo[2,3-b]pyridin-3-yl)acrylamide;
(2E)-N-[(2R)-2-hydroxy-2-phenylethyl]-3-(1H-pyrrolo[2,3-b]pyridin-3-yl)acrylamide;
(2E)-N-[(2S)-2-hydroxy-2-phenylethyl]-3-(1H-pyrrolo[2,3-b]pyridin-3-yl)acrylamide;
(2E)-N-(1-benzylpyrrolidin-3-yl)-3-(1H-pyrrolo[2,3-b]pyridin-3-yl)acrylamide;
(2E)-N-(diphenylmethyl)-3-(1H-pyrrolo[2,3-b]pyridin-3-yl)acrylamide;
(2E)-N-(3-fluorobenzyl)-3-(1H-pyrrolo[2,3-b]pyridin-3-yl)acrylamide;
(2E)-N-(3-chlorobenzyl)-3-(1H-pyrrolo[2,3-b]pyridin-3-yl)acrylamide;
(2E)-N-(4-chlorobenzyl)-3-(1H-pyrrolo[2,3-b]pyridin-3-yl)acrylamide;
(2E)-N-(2-chlorobenzyl)-3-(1H-pyrrolo[2,3-b]pyridin-3-yl)acrylamide;
(2E)-N-(3,4-difluorobenzyl)-3-(1H-pyrrolo[2,3-b]pyridin-3-yl)acrylamide;
(2E)-N-[(3S)-2-oxoazepan-3-yl]-3-(1H-pyrrolo[2,3-b]pyridin-3-yl)acrylamide;
(2E)-N-(3-methoxybenzyl)-3-(1H-pyrrolo[2,3-b]pyridin-3-yl)acrylamide;
(2E)-N-[(1R)-1-phenyl-2-pyrrolidin-1-ylethyl]-3-(1H-pyrrolo[2,3-b]pyridin-3-yl)acrylamide;
(2E)-N-(2,5-difluorobenzyl)-3-(1H-pyrrolo[2,3-b]pyridin-3-yl)acrylamide;
(2E)-N-(1-methylpiperidin-4-yl)-3-(1H-pyrrolo[2,3-b]pyridin-3-yl)acrylamide;
(2E)-N-(1-benzylpiperidin-4-yl)-3-(1H-pyrrolo[2,3-b]pyridin-3-yl)acrylamide;
(2E)-N-[3-(dimethylamino)-2,2-dimethylpropyl]-3-(1H-pyrrolo[2,3-b]pyridin-yl)acrylamide;
(2E)-N-(2,6-difluorobenzyl)-3-(1H-pyrrolo[2,3-b]pyridin-3-yl)acrylamide;
(2E)-3-(1H-pyrrolo[2,3-b]pyridin-3-yl)-N-[3-(1H-pyrrol-1-yl)benzyl]acrylamide;
(2E)-N-[2-(diisopropylamino)ethyl]-3-(1H-pyrrolo[2,3-b]pyridin-3-yl)acrylamide;
(2E)-N-[3-(2-oxopyrrolidin-1-yl)propyl]-3-(1H-pyrrolo[2,3-b]pyridin-3 yl)acrylamide;
(2E)-N-[(1S)-1-benzyl-2-hydroxyethyl]-3-(1H-pyrrolo[2,3-b]pyridin-3-yl)acrylamide;
N-α-[(2E)-3-(1H-pyrrolo[2,3-b]pyridin-3-yl)prop-2-enoyl]-L-phenylalaninamide;
(2E)-N-[(1S)-2-cyclohexyl-1-(hydroxymethyl)ethyl]-3-(1H-pyrrolo[2,3-b]pyridin-3-yl)acrylamide;
(2E)-N-[(2E)-3-phenylprop-2-en-1-yl]-3-(1H-pyrrolo[2,3-b]pyridin-3-yl)acrylamide;
(2E)-N-[(1S)-1-benzyl-2-methoxyethyl]-3-(1H-pyrrolo[2,3-b]pyridin-3-yl)acrylamide;
N-α-[(2E)-3-(1H-pyrrolo[2,3-b]pyridin-3-yl)prop-2-enoyl]-L-tryptophanamide;
(2E)-N-[(1S)-2-hydroxy-1-(4-hydroxybenzyl)ethyl]-3-(1H-pyrrolo[2,3-b]pyridin-yl)acrylamide;
(2E)-N-[cyano(phenyl)methyl]-3-(1H-pyrrolo[2,3-b]pyridin-3-yl)acrylamide;
(2E)-N-(1-benzyl-2-pyrrolidin-1-ylethyl)-3-(1H-pyrrolo[2,3-b]pyridin-3-yl)acrylamide;
(2E)-N-[1-benzyl-2-(dimethylamino)ethyl]-3-(1H-pyrrolo[2,3-b]pyridin-3-yl)acrylamide; (2*E*)-*N*-[1-benzyl-2-(4-methylpiperazin-1-yl)ethyl]-3-(1*H*-pyrrolo[2,3-b]pyridin-3-yl)acrylamide;
(2E)-N-(1-phenyl-3-pyrrolidin-1-ylpropyl)-3-(1H-pyrrolo[2,3-b]pyridin-3-yl)acrylamide;
3-{(1E)-3-[(2S)-2-benzyl-4-methylpiperazin-1-yl]-3-oxoprop-1-en-1-yl}-1H-pyrrolo[2,3-b]pyridine;
(2E)-N-[(1S)-3-methyl-1-(pyrrolidin-1-ylmethyl)butyl]-3-(1H-pyrrolo[2,3-b]pyridin-3-yl)acrylamide;
(2E)-N-[(1S)-2,2-dimethyl-1-(pyrrolidin-1-ylmethyl)propyl]-3-(1H-pyrrolo[2,3-b]pyridin-3-yl)acrylamide;
(2E)-N-[(1S)-2-cyclohexyl-1-(pyrrolidin-1-ylmethyl)ethyl]-3-(1H-pyrrolo[2,3-b]pyridin-3-yl)acrylamide;
(2E)-N-[(1S)-2-methyl-1-(pyrrolidin-1-ylmethyl)propyl]-3-(1H-pyrrolo[2,3-b]pyridin-3-yl)acrylamide;
(2E)-N-[(1R)-2-phenoxy-1-(pyrrolidin-1-ylmethyl)ethyl]-3-(1H-pyrrolo[2,3-b]pyridin-3-yl)acrylamide;
(2*E*)-*N*-[(1*S*)-2-amino-1-benzylethyl]-3-(1*H*-pyrrolo[2,3-b]pyridin-3-yl)acrylamide;
(3S)-4-phenyl-3-{[(2E)-3-(1H-pyrrolo[2,3-b]pyridin-3-yl)prop-2-enoyl]amino}butan-1-aminium chloride;
[(1S,2S)-2-{[(2E)-3-(1H-pyrrolo[2,3-b]pyridin-3-yl)prop-2-enoyl]amino} cyclohexyl] methanaminium chloride;
(2R)-3-phenyl-2-{[(2E)-3-(1H-pyrrolo[2,3-b]pyridin-3-yl)prop-2-enoyl]amino}propan-1-aminium chloride;
(2S)-1-phenyl-3-{[(2E)-3-(1H-pyrrolo[2,3-b]pyridin-3-yl)prop-2-enoyl]amino}propan-2-aminium chloride;
(2E)-N-[(1S)-2-amino-1-(3-fluorobenzyl)ethyl]-3-(1H-pyrrolo[2,3-b]pyridin-3-yl)acrylamide dihydrochloride;
(2E)-N-{(1S)-2-amino-1-[3-(aminomethyl)benzyl]ethyl}-3-(1H-pyrrolo[2,3-b]pyridin-3-yl)acrylamide trihydrochloride;
(2E)-N-[(1S)-2-amino-1-(2-fluorobenzyl)ethyl]-3-(1H-pyrrolo[2,3-b]pyridin-3-yl)acrylamide dihydrochloride;
(2E)-N-[(1S)-2-amino-1-(2-chlorobenzyl)ethyl]-3-(1H-pyrrolo[2,3-b]pyridin-3-yl)acrylamide dihydrochloride;
(2E)-N-[(1S)-2-amino-1-(4-chlorobenzyl)ethyl]-3-(1H-pyrrolo[2,3-b]pyridin-3-yl)acrylamide dihydrochloride;
(2E)-N-[(1S)-2-amino-1-(4-fluorobenzyl)ethyl]-3-(1H-pyrrolo[2,3-b]pyridin-3-yl)acrylamide dihydrochloride;
(2E)-N-[(S)-2-amino-1-(1-naphthylmethyl)ethyl]-3-(1H-pyrrolo[2,3-b]pyridin-3-yl)acrylamide dihydrochloride;
(2E)-N-[(1S)-2-amino-1-(1-benzothien-3-ylmethyl)ethyl]-3-(1H-pyrrolo[2,3-b]pyridin-3-yl)acrylamide dihydrochloride;
(2E)-N-[(1S)-2-amino-1-(pyridin-3-ylmethyl)ethyl]-3-(1H-pyrrolo[2,3-b]pyridin-3-yl)acrylamide trihydrochloride;
(2E)-N-[(1S)-2-amino-1-(2-furylmethyl)ethyl]-3-(1H-pyrrolo[2,3-b]pyridin-3-yl)acrylamide dihydrochloride;
(2E)-N-[(2S)-2-amino-2-phenylethyl]-3-(1H-pyrrolo[2,3-b]pyridin-3-yl)acrylamide dihydrochloride;
(2E)-N-[(1S)-1-(aminomethyl)-2,2-diphenylethyl]-3-(1H-pyrrolo[2,3-b]pyridin-3-yl)acrylamide dihydrochloride;
(2E)-N-[(1S)-1-benzyl-2-pyrrolidin-1-ylethyl]-3-(1H-pyrrolo[2,3-b]pyridin-3-yl)acrylamide;
(2E)-N-[(1S)-1-(aminomethyl)-3-phenylpropyl]-3-(1H-pyrrolo[2,3-b]pyridin-3-yl)acrylamide dihydrochloride;
(2E)-N-[(1S)-2-amino-1-(2-naphthylmethyl)ethyl]-3-(1H-pyrrolo[2,3-b]pyridin-3-yl)acrylamide dihydrochloride;
(2E)-N-[(1S)-2-amino-1-(4-methoxybenzyl)ethyl]-3-(1H-pyrrolo[2,3-b]pyridin-3-yl)acrylamide dihydrochloride;
(2E)-N-[(2R)-2-amino-3-(1H-indol-3-yl)propyl]-3-(1H-pyrrolo[2,3-b]pyridin-3-yl)acrylamide;
(2E)-N-{(1S)-2-amino-1-[3-(trifluoromethyl)benzyl]ethyl}-3-(1H-pyrrolo[2,3-b]pyridin-3-yl)acrylamide dihydrochloride;
(2E)-N-[(1S)-2-amino-1-(3-methylbenzyl)ethyl]-3-(1H-pyrrolo[2,3-b]pyridin-3-yl)acrylamide;
(2E)-N-[(1S)-2-amino-1-(biphenyl-4-ylmethyl)ethyl]-3-(1H-pyrrolo[2,3-b]pyridin-3-yl)acrylamide dihydrochloride;
(2E)-N-[(1S)-2-amino-1-(3,4-dichlorobenzyl)ethyl]-3-(1H-pyrrolo[2,3-b]pyridin-3-yl)acrylamide;
(2E)-N-[(1S)-2-amino-1-(3,4-dichlorobenzyl)ethyl]-3-(1H-pyrrolo[2,3-b]pyridin-3-yl)acrylamide dihydrochloride;
(2E)-N-[(1S)-2-amino-1-(pyridin-4-ylmethyl)ethyl]-3-(1H-pyrrolo[2,3-b]pyridin-3-yl)acrylamide dihydrochloride;
(2E)-N-[(2S)-2-amino-3-(1H-indol-3-yl)propyl]-3-(1H-pyrrolo[2,3-b]pyridin-3-yl)acrylamide;
(2E)-N-[(1S)-2-amino-1-(1H-indol-3-ylmethyl)ethyl]-3-(1H-pyrrolo[2,3-b]pyridin-3-yl)acrylamide;
(E)-N-(S)-2-Amino-1-phenyl-ethyl)-3-(1H-pyrrolo[2,3-b]pyridin-3-yl)-acrylamide;
(2E)-N-[(1S)-2-amino-1-cyclohexylethyl]-3-(1H-pyrrolo[2,3-b]pyridin-3-yl)acrylamide;
(2E)-N-[(1R)-2-amino-1-(phenoxymethyl)ethyl]-3-(1H-pyrrolo[2,3-b]pyridin-3-yl)acrylamide;
(2E)-N-[(1S)-1-(aminomethyl)-3-methylbutyl]-3-(1H-pyrrolo[2,3-b]pyridin-3-yl)acrylamide;
(2E)-N-[(1S)-1-(aminomethyl)-2,2-dimethylpropyl]-3-(1H-pyrrolo[2,3-b]pyridin-3-yl)acrylamide;
(2E)-N-[(1S)-2-amino-1-(cyclohexylmethyl)ethyl]-3-(1H-pyrrolo[2,3-b]pyridin-3-yl)acrylamide;
(2E)-N-[(1S)-1-(aminomethyl)-2-methylpropyl]-3-(1H-pyrrolo[2,3-b]pyridin-3-yl)acrylamide;
(E)-N-((R)-2-Amino-1-{3-[5-(3,3-dimethyl-piperidin-1-yl)-2-hydroxy-benzoyl]-phenoxymethyl}-ethyl)-3-(1H-pyrrolo[2,3-b]pyridin-3-yl)-acrylamide;
(E)-3-(5-Phenylacetylamino-1 H-pyrrolo[2,3-b]pyridin-3-yl)-acrylamide;
Cyclopropanecarboxylic acid [3-((E)-2-carbamoyl-vinyl)-1H-pyrrolo[2,3-b]pyridin-5-yl]-amide;
(E)-3-(5-acetylamino-1 H-pyrrolo[2,3-b]pyridin-3-yl)-acrylamide;
N-[3-((E)-2-Carbamoyl-vinyl)-1H-pyrrolo[2,3-b]pyridin-5-yl]-benzamide;
thiophene-2-carboxylic acid [3-((E)-2-carbamoyl-vinyl)-1H-pyrrolo[2,3-b]pyridin-5-yl]-amide;
(E)-3-(5-Isobutyrylamino-1H-pyrrolo[2,3-b]pyridin-3-yl)-acrylamide;
N-[3-((E)-2-Carbamoyl-vinyl)-1H-pyrrolo[2,3-b]pyridin-5-yl]-3-methyl-butyramide;
Cyclohexanecarboxylic acid [3-((E)-2-carbamoyl-vinyl)-1H-pyrrolo[2,3-b]pyridin-5-yl]-amide;
N- [3-((E)-2-carbamoyl-vinyl)-1H-pyrrolo[2,3-b]pyridin-5-yl]-nicotinamide;
1-methyl-piperidine-4-carboxilic acid [3-((E)-2-carbamoyl-vinyl)-1H-pyrrolo[2,3-b]pyridin-5-yl]-amide;
N- [3-((E)-2-carbamoyl-vinyl)-1H-pyrrolo[2,3-b]pyridin-5-yl]-4-(4-methyl-piperazin-1-yl)-benzamide;
N-[3-((E)-2-carbamoyl-vinyl)-1H-pyrrolo[2,3-b]pyridin-5-yl]-4-morpholin-4-ylmethyl-benzamide;
N-[3-((E)-2-carbamoyl-vinyl)-1H-pyrrolo[2,3-b]pyridin-5-yl]-3-phenoxy-benzamide;
N- [3-((E)-2-Benzylcarbamoyl-vinyl)-1H-pyrrolo[2,3-b]pyridin-5-yl]-benzamide;
(E)-N-Benzyl-3-(5-phenylacetylamino-1H-pyrrolo[2,3-b]pyridin-3-yl]-acrylamide;
(E)-N-Benzyl-3-(5-isobutyrylamino-1H-pyrrolo[2,3-b]pyridin-3-yl]-acrylamide;
(2E)-N-[(1S)-2-amino-1-benzylethyl]-3-(5-nitro-1H-pyrrolo[2,3-b]pyridin-3-yl)acrylamide dihydrochloride;
N-{3-[(1E)-3-{[(1S)-2-amino-1-benzylethyl]amino}-3-oxoprop-1-en-1-yl]-1H-pyrrolo[2,3-b]pyridin-5-yl}benzamide dihydrochloride; and
(E)-3-(5-phenylamino-1 H-pyrrolo[2,3-b]pyridin-3-yl)-acrylamide.

4. A pharmaceutical composition comprising an amount of the compound according to claim 1, or a pharmaceutically acceptable salt thereof, and a pharmaceutically acceptable carrier.

5. A compound according to claim 1 for use as medicament.

6. A compound according to claim 1 for use in a method for treating a cell proliferative disorder or condition in a mammal.

7. A compound according to claim 6 wherein the cell proliferative disorder or condition is selected from the group consisting of bladder cancer, breast cancer, colon cancer, kidney cancer, liver cancer, lung cancer, including small cell lung cancer, esophagus cancer, gall-bladder cancer, ovarian cancer, pancreatic cancer, stomach cancer, cervical cancer, thyroid cancer, prostate cancer, and skin cancer, including squamous cell carcinoma, hematopoietic tumors of lymphoid lineage, including leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma, Burkett's lymphoma, hematopoietic tumors of myeloid lineage, including acute and chronic myelogenous leukemias, myelodysplastic syndrome and promyelocytic leukemia; tumors of mesenchymal origin, including fibrosarcoma and rhabdomyosarcoma; tumors of the central and peripheral nervous system, including astrocytoma, neuroblastoma, glioma and schwannomas; other tumors, including melanoma, seminoma, teratocarcinoma, osteosarcoma, xeroderma pigmentosum, keratoxanthoma, thyroid follicular cancer and Kaposi's sarcoma.
